# EUROPEAN PATENT APPLICATION

(11) **EP 4 722 206 A1**
(43) Date of publication of application: **08.04.2026**
(21) Application number: 24814602.9
(22) Date of filing: 31.05.2024
(51) Int. Cl.: C07D 401/14, C07D 401/00, A61K 31/4545, A61K 31/435, A61P 35/00

(54) **BENZO(HETERO)CYCLOALKYL COMPOUNDS FOR ANDROGEN RECEPTOR-DEPENDENT DISEASES**

(30) Priority: 01.06.2023 CN 202310646204
(71) Applicant: Haihe Biopharma Co., Ltd., Shanghai 201203 (CN)
(72) Inventor: TU, Wangyang, Shanghai 201203 (CN); CHEN, Xiaoxu, Shanghai 201203 (CN); YU, Bing, Shanghai 201203 (CN); ZHANG, Yixiang, Shanghai 201203 (CN); LI, Leping, Shanghai 201203 (CN)
(74) Representative: Sagittarius IP
(86) International application number: PCT/CN2024/096721
(87) International publication number: WO 2024/245408

(57) **Abstract**

The present disclosure relates to benzo(hetero)cycloalkyl compounds or pharmaceutically acceptable salts thereof for the treatment or prevention of androgen receptor-dependent diseases. In particular, the present disclosure relates to compounds of formula (I), pharmaceutical compositions comprising the compounds and the use of the compounds for the prevention or treatment of diseases, in particular for androgen receptor-dependent diseases.

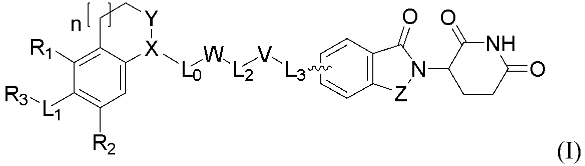

## Description

### CROSS-REFERENCES TO RELATED APPLICATIONS

The present application claims priority for Chinese patent application with application number 202310646204.1, filed on June 1, 2023, the entire disclosures of which are incorporated herein by reference in their entirety.

### TECHNICAL FIELD

The present disclosure relates to benzo(hetero)cycloalkyl compounds or pharmaceutically acceptable salts thereof for the treatment or prevention of androgen receptor (AR)-dependent diseases. The present disclosure also relates to pharmaceutical compositions comprising these compounds and to the use of the compounds for the treatment or prevention of diseases, the diseases being in particular for androgen receptor-dependent diseases.

### BACKGROUND

Prostate cancer is the most common tumor and one of the most lethal tumors in men. Currently, the main treatments for prostate cancer include surgery, radiotherapy, chemotherapy, castration, androgen-targeted therapy, PARP inhibitors and PD-1 immune checkpoint inhibitor antibodies. Because the growths of most prostate cancer cells are androgen-dependent, castration and androgen-targeted therapy are common clinical treatments. Androgen-targeted therapy includes the inhibition of androgen synthesis and the inhibition of androgen receptor, among which Bicalutamide, Abiraterone and Enzalutamide are commonly used clinical drugs, which can effectively prolong the survival of prostate cancer patients. However, most prostate cancer patients eventually develop resistance to castration and androgen-targeted drugs, and the known mechanisms for developing resistance include 1) reactivation of the androgen receptor, 2) activation of the glucocorticoid receptor pathway, and 3) neuroendocrine-type prostate cancer (Nat Rev Cancer. 2015 Dec; 15(12): 701-11); of these, androgen receptor reactivation accounts for more than 60% of drug-resistant patients.

In addition to prostate cancer as the approved indictation, there is substantial evidence that traditional antiandrogens therapies can also be used in a variety of other hormone-dependent and hormone-independent cancer. For example, antiandrogen drugs have been tested in the following cancer: breast cancer (enzalutamide, Breast Cancer Res. (2014) 16(1): R7; darolutamide, ClinicalTrials. gov Identifier: NCT03004534), non-small-cell lung cancer (shRNAi AR), renal cell carcinoma (ASC-J9), partial androgen insensitivity syndrome (PAIS)-associated malignancies (e.g., gonadal tumors and seminoma), advanced pancreatic cancer (World J. Gastroenterology 20(29), 9229), ovarian cancer, fallopian tube cancer or peritoneal cancer, and salivary gland cancer (Head and Neck (2016) 38, 724-731; androgen deprivation therapy (ADT) tested in recurrent/metastatic salivary gland carcinoma expressing AR and confirmed to be beneficial for progression-free survival and overall survival), bladder cancer (Oncotarget 6(30), 29860-29876; IntJ. Endocrinol (2015), ID 384860).

Androgen receptor (AR) is a transcription factor activated by androgen. In prostate cancer, after activation via its ligand-binding domain, AR will enter the cell nucleus from the cell membrane and form a binary complex to activate the transcription and expression of downstream genes. The androgen receptor is divided into three main structural domains: the N-terminal is a loosely structured region (NTD), whose main role is to bind other transcription factors to form a complex; the middle is the DNA-binding region (DBD), which helps the androgen receptor to bind to the DNA of the downstream genes; and the C-terminal is the ligand-binding region (LBD), through which to bind to the androgen. Existing AR-targeted therapeutic drugs mainly act on androgen synthesis and on the ligand-binding domain of the androgen receptor to antagonize the activation of the receptor by androgens. Mutations leading to resistance to AR-targeted therapies mainly occur in the LBD-binding region, including a) point mutations at amino acid sites, such as F877L, T878A, etc., which diminish the antagonistic effect of AR-targeted therapy or even produce an agonistic effect; and b) androgen receptor splice variations, such as AR-Vs, etc., which make the androgen receptor no longer express the complete ligand-binding domain, but only the N-terminal and DNA-binding domains. The absence of the LBD-binding region or mutations in the LBD-binding region renders existing androgen receptor antagonists ineffective in inhibiting the functions of androgen receptor, and thus no longer effective in treating these drug-resistant prostate patients. The above resistance mechanisms arising from AR LBD domain mutations and deletions have necessitated the efforts to develop the next generation of AR-targeted drugs with different mechanisms of action to provide cancer patients with potential new therapies. AR-LBD binding PROTAC molecules can degrade the androgen receptor, thereby completely blocking the androgen receptor pathway. Arvinas' ARV-110 has shown preliminary efficacy in the clinic in prostate cancer patients harboring AR LBD mutations such as T878A, but ARV-110 does not bind to mutations such as AR-Vs which lacks LBD-binding domain. Therefore, it could not benefit prostate cancer patients with AR-Vs mutations. N-terminal targeting AR inhibitor EPI-7386 developed by ESSA is also in early clinical trials and has been shown to be well tolerated in clinical trials, but its efficacy remains to be tested. Therefore, there are still strong unmet medical needs to develop AR-targeted agents with novel mechanisms of action as the potential effective treatment of prostate cancer. Moreover, despite the multiple therapeutic options available to cancer patients, there is still a need for effective and safe therapeutic agents and their preferred use in combination therapies.

### SUMMARY OF THE INVENTION

The compounds of the present disclosure are androgen receptor inhibitors and/or degraders that can be used to treat various diseases and disorders such as those disclosed herein, and in particular for modulating and/or degrading AR full-length (AR-FL), AR splice variant (AR-SV) and/or androgen receptors containing mutations or deletions in the LBD structural domain; the diseases and disorders select from androgen receptor-dependent disorders, which include, but are not limited to prostate cancer, other prostate diseases, breast cancer, non-small cell lung cancer, renal cell carcinoma, gonadal tumors, seminoma, pancreatic cancer, ovarian cancer, fallopian tube cancer, peritoneal cancer, salivary gland cancer, bladder cancer, acne, hirsutism, hidradenitis suppurativa, androgenetic alopecia, cryptorchidism, androgen insensitivity syndrome, Kennedy's disease, etc.

In particular, the present disclosure provides androgen receptor inhibitors and/or degraders as shown in formula (I): 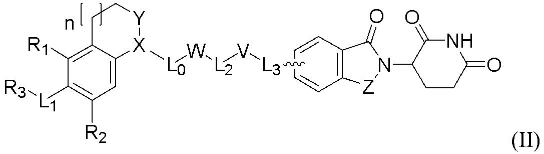 or stereoisomers, solvates, hydrates or pharmaceutically acceptable salts thereof; wherein each of the variables are as defined herein. They can be used for the treatment or prevention of androgen receptor-dependent diseases or disorders, particularly cancer.

The compound of formula (I), or the stereoisomer, the solvate, the hydrate or the pharmaceutically acceptable salt thereof, as well as specific compounds disclosed in the context of the present disclosure and covered by the scope of the said compounds, are collectively referred to as "compounds of the present disclosure".

The present disclosure further provides a method for preparing the compound of the present disclosure, intermediates for preparing the compound of the present disclosure, and a method for preparing the intermediates.

The present disclosure further provides a composition comprising at least one of the compounds of the present disclosure.

The present disclosure further provides a pharmaceutical composition comprising a therapeutically effective amount of the compound of the present disclosure, and one or more pharmaceutically acceptable excipients. The compounds of the present disclosure are androgen receptor inhibitors and/or degraders that can inhibit and/or degrade androgen receptors (including AR-FL, AR-SV and androgen receptors containing mutations or deletions in the LBD structural domain) in vivo, and can thus be used in androgen receptor-dependent diseases or disorders, in particular AR-SV-positive diseases or disorders, AR-V7-positive diseases or disorders, or diseases or disorders containing mutations or deletions in the LBD structural domain, such as cancer. Accordingly, the present disclosure may provide methods or uses for inhibiting transcriptional activation function of the androgen receptor. Examples include uses of the compounds of the present disclosure for inhibiting androgen-mediated responses in an individual, and methods of using the compounds of the present disclosure for modulating immune responses in an individual.

The present disclosure provides the compounds of the present disclosure which are used as drugs.

The present disclosure provides the compounds of the present disclosure for inhibiting and/or degrading androgen receptors, including AR-FL, AR-SV, and androgen receptors containing mutations or deletions in the LBD structural domain.

The present disclosure provides the compounds of the present disclosure for use in the treatment or prevention of androgen receptor-dependent diseases or disorders in individuals, particularly AR-SV-positive diseases or disorders, AR-V7-positive diseases or disorders or diseases, or disorders comprising mutations or deletions in the LBD structural domain, such as cancer.

The present disclosure provides use of the compounds of the present disclosure for the preparation of drugs, the drugs being used for the treatment or prevention of androgen receptor-dependent diseases or disorders, in particular AR-SV-positive diseases or disorders, AR-V7-positive diseases or disorders, or diseases or disorders containing mutations or deletions in the LBD structural domain, such as cancer.

The present disclosure provides a method for inhibiting and/or degrading androgen receptors (including AR-FL, AR-SV, and androgen receptors containing mutations or deletions in the LBD structural domain) in vivo or in vitro, wherein the method comprises contacting an androgen receptor inhibitor and/or degrader, such as the compound of the present disclosure, with an androgen receptor.

The present disclosure provides a method for inhibiting and/or degrading an androgen receptor (including AR-FL, AR-SV, and androgen receptors containing mutations or deletions in the LBD structural domain) in an individual, wherein the method comprises administering an effective amount of an androgen receptor inhibitor and/or degrader, such as the compound of the present disclosure, to the individual in need thereof.

The present disclosure provides a method for treating or preventing diseases or disorders in individuals comprising administering an effective amount of the compound of the present disclosure to an individual in need thereof, the diseases or disorders being androgen receptor-dependent diseases or disorders, in particular AR-SV-positive diseases or disorders, AR-V7-positive diseases or disorders, or diseases or disorders comprising mutations or deletions in the LBD structural domain, e.g., cancer.

The present disclosure provides a method for treating or preventing diseases or disorders, the method comprising administering an effective amount of a first therapeutic agent and optionally a second therapeutic agent to an individual in need thereof, wherein the first therapeutic agent is the compound of the present disclosure, and the second therapeutic agent is one or more other therapeutic agents; the diseases or disorders are androgen receptor-dependent diseases or disorders, in particular an AR-SV positive diseases or disorders, AR-V7 positive diseases or disorders, or diseases or disorders containing mutations or deletions in the LBD structural domain, such as cancer.

Additionally, the present disclosure provides a combination (combination product) or a kit, the combination product or kit comprising the compound of the present disclosure (or the pharmaceutical composition thereof) as defined above with one or more other therapeutic agents (or pharmaceutical compositions thereof), which are intended for use concurrently, separately, or sequentially in the treatment or prevention of androgen receptor-dependent diseases or disorders.

### EMBODIMENTS

**Embodiment** 1. A compound of formula (I), or a stereoisomer, a solvate, a hydrate or a pharmaceutically acceptable salt thereof, 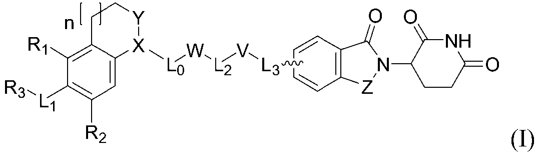
wherein, n is 0 or 1;
X is N, CCH₃ or CH;
Y is NR₄ or CH₂, wherein R₄ is H, acetyl or C₁₋₃ alkyl;
Z is C(O) or CH₂;
R₁ and R₂ are each independently selected from H, halogen, CN, C₁₋₄ alkyl, C₁₋₄ alkoxy, and halo C₁₋₄ alkyl;
L₁ is a single bond, O, S or NH;
R₃ is C₁₋₆ alkyl, C₃₋₆ cycloalkyl or C₃₋₆ cycloalkyl-C₁₋₄ alkyl; optionally being substituted with halogen or CN;
L₀ is a single bond, O, S, NH, -C(O)-NH-, -NH-C(O)-, -NH-(CH₂)- or -NH-(CH₂)₂-;
W is C₆₋₁₂ aryl, 5-12-membered heteroaryl or 4-12-membered heterocyclyl; each of which is optionally substituted with 1, 2 or 3 substituents independently selected from: halogen, CN, oxo, NH₂, NR₇R₈, OH, OR₈, R₈, -C(O)-NHR₇, -OC(O)-NHR₈, -SO₂R₈, -SO₂NHR₇, -NR₇SO₂R₈, -NR₇SO₂NHR₈ and -NHC(O)-R₈;
wherein, R₇ is each independently selected from H, C₁₋₄ alkyl and acetyl; R₈ is C₁₋₄ alkyl optionally substituted with one or more halogens, hydroxyls or NH₂;
L₂ is a single bond, -NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₈ alkyl-, -C₂₋₆ alkenyl-, -C₁₋₄ alkyl-O-C₁₋₄ alkyl-, -C₁₋₄ alkyl-NH-C₁₋₄ alkyl-, -C₃₋₆ cycloalkyl, -O-C₃₋₆ cycloalkyl, -NH-C₃₋₆ cycloalkyl, phenyl, 3-10-membered heterocycloalkyl or 5-10-membered heteroaryl;
L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₈ alkyl-, -C₂₋₆ alkenyl-, -C₁₋₄ alkyl-O-C₁₋₄ alkyl-, -C₁₋₄ alkyl-NH-C₁₋₄ alkyl-, -C₃₋₆ cycloalkyl, -C₃₋₆ cycloalkyl-O-, -C₃₋₆ cycloalkyl-NH-, phenyl, 3-10-membered heterocycloalkyl or 5-10-membered heteroaryl; and
the alkyl, the heteroaryl and the heterocycloalkyl in L₂ and L₃ are each optionally substituted with one or more substituents independently selected from halogen, OH, NH₂, CN, C(O)C₁₋₆ alkyl, C(O)NH₂, C₁₋₆ alkyl, C₃₋₆ cycloalkyl, C₁₋₆ alkoxy, -NHC₁₋₆ alkyl, and -N(C₁₋₆ alkyl)₂;
V is CH₂, O, S or NH;
wherein, the heteroaryl, the heterocyclyl, the heterocycloalkyl are each independently comprising 1, 2, 3 or 4 heteroatoms independently selected from N, O and S.

**Embodiment 2.** The compound, or the stereoisomer, the solvate, the hydrate or the pharmaceutically acceptable salt thereof according to embodiment 1, wherein. the structural fragment 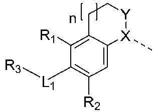 is

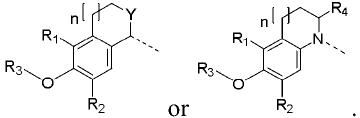

**Embodiment 3.** The compound, or the stereoisomer, the solvate, the hydrate or the pharmaceutically acceptable salt thereof according to any one of the preceding embodiments, wherein the structural fragment 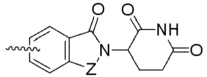 is 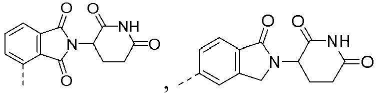 or

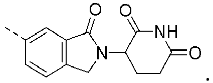

**Embodiment 4.** The compound, or the stereoisomer, the solvate, the hydrate or the pharmaceutically acceptable salt thereof according to any one of the preceding embodiments, wherein
n is 0 or 1;
X is CH;
Y is NR₄ or CH₂, wherein R₄ is H, acetyl or C₁₋₃ alkyl;
Z is C(O) or CH₂;
R₁ and R₂ are each independently selected from halogen, CN, C₁₋₄ alkyl, C₁₋₄ alkoxy, and halo C₁₋₄ alkyl;
L₁ is a single bond, O, S or NH;
R₃ is C₁₋₆ alkyl optionally substituted with halogen or CN;
L₀ is a single bond, O, S, NH, -C(O)-NH-, -NH-C(O)-, -NH-(CH₂)- or -NH-(CH₂)₂-;
W is C₆₋₁₂ aryl, 5-10-membered heteroaryl or 5-10-membered heterocyclyl, said aryl, heteroaryl and heterocyclyl being each optionally substituted with 1, 2 or 3 substituents independently selected from: halogen, CN, OH, NH₂, oxo, C(O)NH₂, C₁₋₄ alkyl and C₁₋₄ alkoxy;
L₂ is a single bond, -NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₈ alkyl-, -C₂₋₆ alkenyl-, -C₁₋₄ alkyl-O-C₁₋₄ alkyl-, -C₁₋₄ alkyl-NH-C₁₋₄ alkyl-, -C₃₋₆ cycloalkyl, -O-C₃₋₆ cycloalkyl, -NH-C₃₋₆ cycloalkyl, phenyl, 3-10-membered heterocycloalkyl or 5-10-membered heteroaryl;
L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₈ alkyl-, -C₂₋₆ alkenyl-, -C₁₋₄ alkyl-O-C₁₋₄ alkyl-, -C₁₋₄ alkyl-NH-C₁₋₄ alkyl-, -C₃₋₆ cycloalkyl, -C₃₋₆ cycloalkyl-O-, -C₃₋₆ cycloalkyl-NH-, phenyl, 3-10-membered heterocycloalkyl or 5-10-membered heteroaryl; and
said heteroaryl and heterocycloalkyl in L₂ and L₃ are each optionally substituted with one or more substituents independently selected from halogen, OH, CN, C(O)C₁₋₆ alkyl, C₁₋₄ alkyl and C₁₋₄ alkoxy;
V is CH₂, O, S or NH;
wherein, said heteroaryl, heterocyclyl, heterocycloalkyl are each independently comprising 1, 2, 3 or 4 heteroatoms independently selected from N, O and S.

**Embodiment 5.** The compound, or the stereoisomer, the solvate, the hydrate or the pharmaceutically acceptable salt thereof according to any one of the preceding embodiments, wherein
n is 0 or 1;
X is CH;
Y is CH₂;
Z is C(O) or CH₂;
R₁ and R₂ are each independently selected from halogen, CN, C₁₋₄ alkyl, C₁₋₄ alkoxy, and halo C₁₋₄ alkyl;
L₁ is a single bond, O, S or NH;
R₃ is C₁₋₆ alkyl optionally substituted with halogen or CN;
L₀ is a single bond, O, S or NH;
W is C₆₋₁₂ aryl, 5-10-membered heteroaryl or 5-10-membered heterocyclyl, said aryl, heteroaryl and heterocyclyl being each optionally substituted with 1, 2 or 3 substituents independently selected from: halogen, OH, NH₂, CN, oxo, C(O)NH₂, C₁₋₄ alkyl and C₁₋₄ alkoxy;
L₂ is a single bond, -NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₈ alkyl-, -C₂₋₆ alkenyl-, -C₁₋₄ alkyl-O-C₁₋₄ alkyl -, -C₁₋₄ alkyl-NH-C₁₋₄ alkyl-, -C₃₋₆ cycloalkyl, -O-C₃₋₆ cycloalkyl, -NH-C₃₋₆ cycloalkyl, phenyl, 3-10-membered heterocycloalkyl or 5-10-membered heteroaryl;
L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₈ alkyl-, -C₂₋₆ alkenyl-, -C₁₋₄ alkyl-O-C₁₋₄ alkyl-, -C₁₋₄ alkyl-NH-C₁₋₄ alkyl-, -C₃₋₆ cycloalkyl, -C₃₋₆ cycloalkyl-O-, -C₃₋₆ cycloalkyl-NH-, phenyl, 3-10-membered heterocycloalkyl or 5-10-membered heteroaryl; and
said heteroaryl and heterocycloalkyl in L₂ and L₃ are each optionally substituted with one or more substituents independently selected from halogen, OH, CN, C(O)C₁₋₆ alkyl, C₁₋₄ alkyl and C₁₋₄ alkoxy;
V is CH₂, O, S or NH;
wherein, said heteroaryl, heterocyclyl, heterocycloalkyl are each independently comprising 1, 2, 3 or 4 heteroatoms independently selected from N, O and S.

**Embodiment 6.** The compound, or the stereoisomer, the solvate, the hydrate or the pharmaceutically acceptable salt thereof according to any one of the preceding embodiments, wherein W is C₆₋₁₂ aryl or 5-10-membered heteroaryl; the heteroaryl comprises 1, 2, 3 or 4 heteroatoms independently selected from N, O, and S, for example, the heteroaryl comprises 1 N heteroatom and optionally further comprises 1, 2 or 3 heteroaryl atoms independently selected from N, O, and S.

**Embodiment 7.** The compound, or the stereoisomer, the solvate, the hydrate or the pharmaceutically acceptable salt thereof according to any one of the preceding embodiments, wherein
n is 0 or 1;
X is CH;
Y is CH₂;
Z is C(O) or CH₂;
R₁ and R₂ are each independently selected from halogen, CN, C₁₋₄ alkyl, C₁₋₄ alkoxy, and halo C₁₋₄ alkyl;
L₁ is a single bond, O, S or NH;
R₃ is C₁₋₆ alkyl optionally substituted with halogen or CN;
L₀ is a single bond, O, S or NH;
W is C₆₋₁₂ aryl or 5-10-membered heteroaryl; wherein the aryl and the heteroaryl are each optionally substituted with 1, 2 or 3 substituents independently selected from halogen, CN, OH, NH₂, oxo, C(O)NH₂, C₁₋₄ alkyl and C₁₋₄ alkoxy;
L₂ is a single bond, -NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₆ alkyl-, -C₁₋₄ alkyl-O-C₁₋₄ alkyl-, -C₁₋₄ alkyl-NH-C₁₋₄ alkyl-, -O-C₃₋₆ cycloalkyl, -NH-C₃₋₆ cycloalkyl, 3-10-membered heterocycloalkyl or 5-10-membered heteroaryl;
L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₆ alkyl-, -C₁₋₄ alkyl-O-C₁₋₄ alkyl-, -C₁₋₄ alkyl-NH-C₁₋₄ alkyl-, -C₃₋₆ cycloalkyl-O-, -C₃₋₆ cycloalkyl-NH-, 3-10-membered heterocycloalkyl or 5-10-membered heteroaryl; and
V is CH₂, O, S or NH;
wherein, the heterocycloalkyl or the heteroaryl each independently comprises 1, 2, 3 or 4 heteroatoms independently selected from N, O and S, for example, the heterocycloalkyl or the heteroaryl each independently comprises 1 N heteroatom and optionally further comprises 1, 2 or 3 heteroatoms independently selected from N, O and S.

**Embodiment 8.** The compound according to any one one of the preceding embodiments having the structure of formula (II):

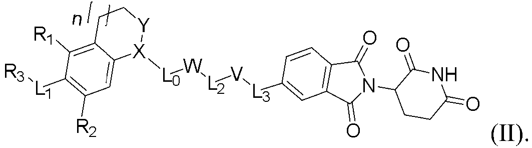

**Embodiment 9.** The compound, or the stereoisomer, the solvate, the hydrate or the pharmaceutically acceptable salt thereof according to any one of the preceding embodiments, wherein W is C₆₋₁₂ aryl or 5-10-membered heteroaryl; the heteroaryl comprises 1 N heteroatom and optionally further comprises 1, 2 or 3 heteroatoms independently selected from N, O and S, and is each optionally substituted with 1, 2 or 3 substituents independently selected from halogen and CN.

**Embodiment 10.** The compound, or the stereoisomer, the solvate, the hydrate or the pharmaceutically acceptable salt thereof according to any one of the preceding embodiments, wherein W is 5-10-membered heteroaryl; the heteroaryl comprises 1 N heteroatom and optionally further comprises 1, 2 or 3 heteroatoms independently selected from N, O and S, and is optionally substituted with 1, 2 or 3 substituents independently selected from halogen and CN.

**Embodiment 11.** The compound, or the stereoisomer, the solvate, the hydrate or the pharmaceutically acceptable salt thereof according to any one of the preceding embodiments 1-9, wherein W is C₆₋₁₀ aryl (e.g., benzene ring), or 5-10-membered heteroaryl comprising 1, 2 or 3 N heteroatoms; each of which optionally substituted with 1, 2 or 3 substituents independently selected from halogen and CN.

**Embodiment 12.** The compound, or the stereoisomer, the solvate, the hydrate or the pharmaceutically acceptable salt thereof according to any one of the preceding embodiments, wherein W is 5-10-membered heteroaryl; the heteroaryl comprises 1 N heteroatom and optionally further comprises 1 or 2 heteroatoms independently selected from N, O and S (e.g., comprising 1, 2 or 3 N heteroatoms), (e.g., pyridinyl, indazolyl, azaindazolyl or pyrimidinyl), and is optionally substituted with 1, 2 or 3 substituents independently selected from halogen and CN.

**Embodiment 13.** The compound, or the stereoisomer, the solvate, the hydrate or the pharmaceutically acceptable salt thereof according to any one of the preceding embodiments, wherein W is 5-6-membered heteroaryl or 8-10-membered heteroaryl, the heteroaryl comprising 1 N heteroatom and optionally further comprising 1 or 2 heteroatoms independently selected from N, O and S; each of which is optionally substituted with 1, 2 or 3 substituents independently selected from N, O and S.

**Embodiment 14.** The compound, or the stereoisomer, the solvate, the hydrate or the pharmaceutically acceptable salt thereof according to any one of the preceding embodiments, wherein W is 5-6-membered heteroaryl (e.g., a 6-membered heteroaryl), the heteroaryl comprising 1 N-heteroatom and optionally further comprising 1 or 2 heteroatoms independently selected from N, O and S (e.g., comprising 1, 2 or 3 N heteroatoms) (e.g., pyridinyl or pyrimidinyl); which is optionally substituted with 1, 2 or 3 substituents independently selected from halogen and CN.

**Embodiment 15.** The compound, or the stereoisomer, the solvate, the hydrate or the pharmaceutically acceptable salt thereof according to any one of the preceding embodiments, wherein W is 6-membered heteroaryl comprising 1, 2 or 3 N heteroatoms (e.g., pyridyl or pyrimidyl), which is optionally substituted with 1 or 2 CN.

**Embodiment 16.** The compound, or the stereoisomer, the solvate, the hydrate or the pharmaceutically acceptable salt thereof according to any one of the preceding embodiments, wherein W is 8-10-membered heteroaryl, the heteroaryl comprising 1 N heteroatom and optionally further comprising 1 or 2 heteroatoms independently selected from N, O and S (e.g., comprising 1, 2 or 3 N-heteroatoms) (e.g., indazolyl or azaindazolyl); which is optionally substituted with 1 or 2 CN.

**Embodiment 17.** The compound, or the stereoisomer, the solvate, the hydrate or the pharmaceutically acceptable salt thereof according to any one of the preceding embodiments 1-5, wherein L₂ is a single bond, -NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₆ alkyl-, -C₁₋₄ alkyl-O-C₁₋₄ alkyl-, -C₁₋₄ alkyl-NH-C₁₋₄ alkyl-, -O-C₃₋₆ cycloalkyl, -NH-C₃₋₆ cycloalkyl or 3-10-membered heterocycloalkyl; wherein the heterocycloalkyl comprises 1 N heteroatom and optionally further comprises 1, 2 or 3 heteroatoms independently selected from N, O and S, and the heterocycloalkyl is optionally substituted with halogen, OH, CN, C(O)C₁₋₄ alkyl, C₁₋₄ alkyl or C₁₋₄ alkoxy.

**Embodiment 18.** The compound, or the stereoisomer, the solvate, the hydrate or the pharmaceutically acceptable salt thereof according to any one of the preceding embodiments, wherein L₂ is a single bond, -NH-C₁₋₆ alkyl-, -NH-C₂₋₆ alkenyl-, -NH-C₁₋₄ alkyl-O-C₁₋₄ alkyl-, - O-C₁₋₆ alkyl-, -O-C₂₋₆ alkenyl-, -O-C₁₋₄ alkyl-O-C₁₋₄ alkyl-, -C₁₋₆ alkyl-, -C₁₋₄ alkyl-O-C₁₋₄ alkyl-, -C₁₋₄ alkyl-NH-C₁₋₄ alkyl-, -O-C₃₋₆ cycloalkyl, -NH-C₃₋₆ cycloalkyl or 3-10-membered heterocycloalkyl; wherein the heterocycloalkyl comprises 1 N heteroatom and optionally further comprises 1, 2 or 3 heteroatoms independently selected from N, O and S.

**Embodiment 19.** The compound, or the stereoisomer, the solvate, the hydrate or the pharmaceutically acceptable salt thereof according to any one of the preceding embodiments, wherein L₂ is a single bond, -NH-C₁₋₆ alkyl-, -O-C₁₋₆ alkyl-, -C₁₋₆ alkyl-, -O-C₃₋₆ cycloalkyl or 4-8-membered heterocycloalkyl; the 4-8-membered heterocycloalkyl comprises 1 N heteroatom and optionally further comprises 1 or 2 heteroatoms independently selected from N, O and S.

**Embodiment 20.** The compound, or the stereoisomer, the solvate, the hydrate or the pharmaceutically acceptable salt thereof according to any one of the preceding embodiments, wherein L₂ is a single bond, -NH-C₁₋₄ alkyl-, -O-C₁₋₄ alkyl-, -C₁₋₄ alkyl-, -O-C₃₋₆ cycloalkyl or 4-6-membered heterocycloalkyl; the 4-6-membered heterocycloalkyl comprises 1 N heteroatom and optionally further comprises 1 or 2 heteroatoms independently selected from N, O and S.

**Embodiment 21.** The compound, or the stereoisomer, the solvate, the hydrate or the pharmaceutically acceptable salt thereof according to any one of the preceding embodiments, wherein L₂ is a single bond, -NH-C₁₋₄ alkyl-, -O-C₁₋₄ alkyl-, -C₁₋₄ alkyl-, -O-C₃₋₆ cycloalkyl or 4-6-membered heterocycloalkyl; the 4-6-membered heterocycloalkyl comprises 1 N heteroatom, and L₂ is connected to W via a heteroatom when L₂ is not a single bond or -C₁₋₄ alkyl-.

**Embodiment 22.** The compound, or the stereoisomer, the solvate, the hydrate or the pharmaceutically acceptable salt thereof according to any one of the preceding embodiments, wherein L₂ is a single bond, -NH-C₁₋₄ alkyl-, -O-C₁₋₄ alkyl-, -O-C₅₋₆ cycloalkyl or 4-6-membered heterocycloalkyl; the 4-6-membered heerocycloalkyl comprises 1 N heteroatom, and L₂ is connected to W via a heteroatom when L₂ is not a single bond.

**Embodiment 23.** The compound, or the stereoisomer, the solvate, the hydrate or the pharmaceutically acceptable salt thereof according to any one of the preceding embodiments 1-17, wherein L₂ is selected from -NH-C₁₋₄ alkyl-, -O-C₁₋₄ alkyl-, -O-C₃₋₆ cycloalkyl, and from 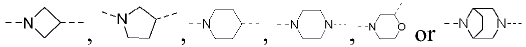 optionally substituted with halogen, OH, - C(O)C₁₋₄ alkyl, C₁₋₄ alkyl or C₁₋₄ alkoxy; wherein L₂ is connected to W via a heteroatom.

**Embodiment 24.** The compound, or the stereoisomer, the solvate, the hydrate or the pharmaceutically acceptable salt thereof according to any one of the preceding embodiments, wherein L₂ is selected from a single bond, -NH-C₁₋₃ alkyl-, -O-C₁₋₃ alkyl-, -O-C₅₋₆ cycloalkyl-, 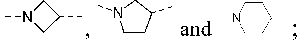 wherein L₂ is connected to W via a heteroatom when L₂ is not a single bond.

**Embodiment 25.** The compound, or the stereoisomer, the solvate, the hydrate or the pharmaceutically acceptable salt thereof according to any one of the preceding embodiments, wherein L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₆ alkyl-, -C₁₋₄ alkyl-O-C₁₋₄ alkyl-, -C₁₋₄ alkyl-NH-C₁₋₄ alkyl-, -C₃₋₆ cycloalkyl-O-, -C₃₋₆ cycloalkyl-NH- or 3-10-membered heterocycloalkyl; wherein the heterocycloalkyl comprises 1 N heteroatom and optionally further comprises 1, 2 or 3 heteroatoms independently selected from N, O and S, and the heterocycloalkyl is optionally substituted with halogen, OH, CN, C(O)C₁₋₄ alkyl, C₁₋₄ alkyl or C₁₋₄ alkoxy.

**Embodiment 26.** The compound, or the stereoisomer, the solvate, the hydrate or the pharmaceutically acceptable salt thereof according to any one of the preceding embodiments, wherein L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₆ alkyl-, -C₁₋₄ alkyl-O-C₁₋₄ alkyl-, -C₁₋₄ alkyl-NH-C₁₋₄ alkyl-, -C₃₋₆ cycloalkyl-O-, -C₃₋₆ cycloalkyl-NH- or 3-10-membered heterocycloalkyl; wherein the heterocycloalkyl comprises 1 N heteroatom and optionally further comprises 1, 2 or 3 heteroatoms independently selected from N, O and S.

**Embodiment 27.** The compound, or the stereoisomer, the solvate, the hydrate or the pharmaceutically acceptable salt thereof according to any one of the preceding embodiments, wherein L₃ is -C₁₋₄ alkyl-O-, -C₁₋₄ alkyl-NH-, -C₃₋₆ cycloalkyl-O-, -C₃₋₆ cycloalkyl-NH- or 4-8-membered heterocycloalkyl; the 4-8-membered heterocycloalkyl comprises 1 N heteroatom and optionally further comprises 1 or 2 heteroatoms independently selected from N, O and S.

**Embodiment 28.** The compound, or the stereoisomer, the solvate, the hydrate or the pharmaceutically acceptable salt thereof according to any one of the preceding embodiments, wherein L₃ is -C₁₋₄ alkyl-O-, -C₁₋₄ alkyl-NH-, -C₃₋₆ cycloalkyl-O- or 4-6-membered heterocycloalkyl; the 4-6-membered heterocycloalkyl comprises 1 N heteroatom, and L₃ is connected to the benzene ring of the general formula via a heteroatom.

**Embodiment 29.** The compound, or the stereoisomer, the solvate, the hydrate or the pharmaceutically acceptable salt thereof according to any one of the preceding embodiments, wherein L₃ is -C₁₋₃ alkyl-O-, -C₁₋₃ alkyl-NH-, -C₅₋₆ cycloalkyl-O- or 4-6-membered heterocycloalkyl; the 4-6-membered heterocycloalkyl comprises 1 N heteroatom, L₃ is connected to the benzene ring of the general formula via a heteroatom.

**Embodiment 30.** The compound, or the stereoisomer, the solvate, the hydrate or the pharmaceutically acceptable salt thereof according to embodiment 25, wherein L₃ is selected from -C₁₋₆ alkyl-O-, -C₁₋₆ alkyl-NH-, and from 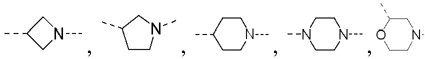 or 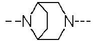 optionally substituted with halogen, OH, -C(O)C₁₋₄ alkyl, C₁₋₄ alkyl or C₁₋₄ alkoxy; and L₃ is connected to the benzene ring of the general formula via a heteroatom.

**Embodiment 31.** The compound, or the stereoisomer, the solvate, the hydrate or the pharmaceutically acceptable salt thereof according to any one of the preceding embodiments, wherein L₃ is selected from -C₁₋₃ alkyl-O-, -C₁₋₃ alkyl-NH-, 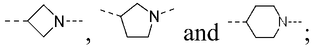 and L₃ is connected to the benzene ring of the general formula via a heteroatom.

Embodiment 32. The compound, or the stereoisomer, the solvate, the hydrate or the pharmaceutically acceptable salt thereof according to any one of the preceding embodiments, wherein the structure 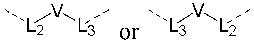 is selected from:

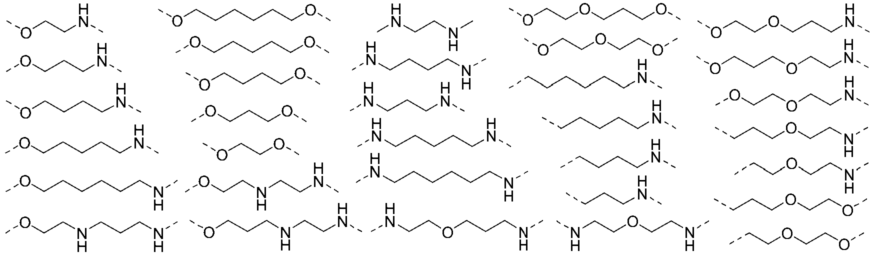

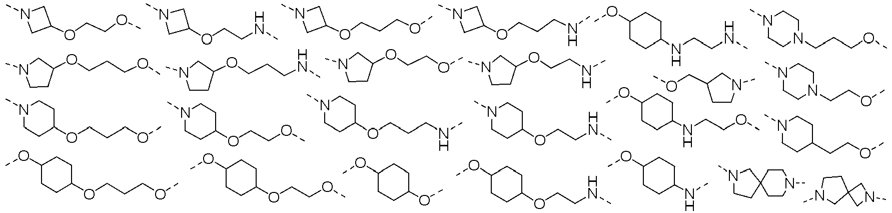

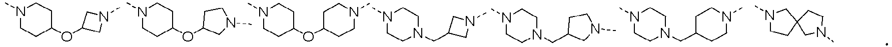

**Embodiment 33.** The compound, or the stereoisomer, the solvate, the hydrate or the pharmaceutically acceptable salt thereof according to any one of the preceding embodiments, wherein V is CH₂, O or NH.

**Embodiment 34.** The compound, or the stereoisomer, the solvate, the hydrate or the pharmaceutically acceptable salt thereof according to any one of the preceding embodiments, wherein V is CH₂ or O.

**Embodiment 35.** The compound, or the stereoisomer, the solvate, the hydrate or the pharmaceutically acceptable salt thereof according to any one of the preceding embodiments, wherein n is 1.

**Embodiment 36.** The compound, or the stereoisomer, the solvate, the hydrate or the pharmaceutically acceptable salt thereof according to any one of the preceding embodiments, wherein R₁ and R₂ are each independently halogen, CN or halo C₁₋₄ alkyl.

**Embodiment 37.** The compound, or the stereoisomer, the solvate, the hydrate or the pharmaceutically acceptable salt thereof according to any one of the preceding embodiments, wherein R₁ and R₂ are each independently halogen, CN, or CF₃, e.g., Cl or CN.

**Embodiment 38.** The compound, or the stereoisomer, the solvate, the hydrate or the pharmaceutically acceptable salt thereof according to any one of the preceding embodiments, wherein R₁ and R₂ are each independently halogen or CN, e.g., Cl or CN.

**Embodiment 39.** The compound, or the stereoisomer, the solvate, the hydrate or the pharmaceutically acceptable salt thereof according to any one of the preceding embodiments, wherein L₁ is O or S, e.g., O.

**Embodiment 40.** The compound, or the stereoisomer, the solvate, the hydrate or the pharmaceutically acceptable salt thereof according to any one of the preceding embodiments, wherein L₁ is O.

**Embodiment 41.** The compound, or the stereoisomer, the solvate, the hydrate or the pharmaceutically acceptable salt thereof according to any one of the preceding embodiments, wherein R₃ is C₁₋₄ alkyl optionally substituted with halogen, e.g., C₁-C₄ alkyl optionally substituted with Cl, such as ethyl or chloroethyl.

**Embodiment 42.** The compound, or the stereoisomer, the solvate, the hydrate or the pharmaceutically acceptable salt thereof according to any one of the preceding embodiments, wherein L₀ is a single bond or NH.

**Embodiment 43.** The compound, or the stereoisomer, the solvate, the hydrate or the pharmaceutically acceptable salt thereof according to any one of the preceding embodiments, wherein
n is 0 or 1;
X is CH;
Y is CH₂;
Z is C(O);
R₁ and R₂ are each independently selected from halogen and CN;
L₁ is O;
R₃ is ethyl optionally substituted with halogen;
L₀ is a single bond or NH;
W is C₆₋₁₂ aryl or 5-10-membered heteroaryl containing 1, 2 or 3 N heteroatoms; each of which is optionally substituted with 1, 2 or 3 substituents independently selected from halogen and CN;
L₂ is selected from a single bond, -C₁₋₄ alkyl-, -NH-C₁₋₄ alkyl-, -O-C₁₋₄ alkyl-, -O-C₃₋₆ cycloalkyl, -NH-C₃₋₆ cycloalkyl, and from 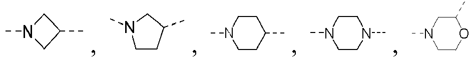 or 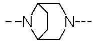 optionally substituted with F, Cl, OH, C(O)C₁₋₆ alkyl or C₁₋₄ alkyl; and L₂ is connected to W via a heteroatom when L₂ is not a single bond or -C₁₋₄ alkyl;
L₃ is selected from -C₁₋₄ alkyl-O-, -C₁₋₄ alkyl-NH-, -C₃₋₆ cycloalkyl-O-, -C₃₋₆ cycloalkyl-NH-, and from 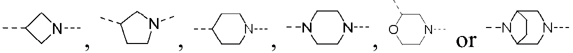 optionally substituted with F, Cl, OH, C(O)C₁₋₄ alkyl or C₁₋₄ alkyl; and L₃ is connected to the benzene ring in the general formula via a heteroatom;
V is CH₂ or O.

**Embodiment 44.** The compound, or the stereoisomer, the solvate, the hydrate or the pharmaceutically acceptable salt thereof according to any one of the preceding embodiments, wherein W is 5-10-membered heteroaryl containing 1, 2 or 3 N heteroatoms, which is optionally substituted with 1, 2 or 3 substituents independently selected from halogen and CN.

**Embodiment 45.** The compound, or the stereoisomer, the solvate, the hydrate or the pharmaceutically acceptable salt thereof according to any one of the preceding embodiments, wherein W is pyrazolyl, phenyl, pyridyl, pyrimidyl, indolyl, indazolyl, or azaindazolyl (e.g., 7-azaindazolyl, 6-azaindazolyl, 5-azaindazolyl, 4-azaindazolyl), for example 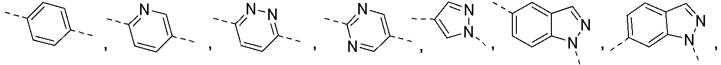 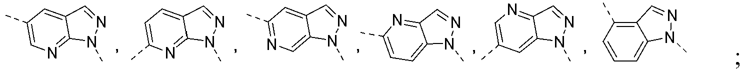 each of which is optionally substituted with 1, 2 or 3 substituents independently selected from halogen and CN,

**Embodiment 46.** The compound, or the stereoisomer, the solvate, the hydrate or the pharmaceutically acceptable salt thereof according to any one of the preceding embodiments, wherein
n is 1;
X is CH;
Y is CH₂;
Z is C(O);
R₁ is halogen;
R₂ is CN;
L₁ is O;
R₃ is ethyl optionally substituted with halogen;
L₀ is a single bond or NH;
W is 5-10-membered heteroaryl (e.g., pyridinyl, pyrimidinyl, azaindazolyl or indazolyl) containing 1, 2 or 3 N heteroatoms, which is optionally substituted with 1 or 2 substituents independently selected from halogen and CN;
L₂ is a single bond, -NH-CH₂-, -NH-CH₂-CH₂-, -O-CH₂-, -O-CH₂-CH₂-, -C₁₋₃ alkyl-, -O-C₃₋₆ cycloalkyl- or 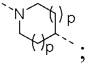 wherein p is each independently selected from 0 and 1, and L₂ is connected to W via a heteroatom when L₂ is not a single bond or -C₁₋₃ alkyl-;
L₃ is -CH₂-CH₂-O-, -CH₂-CH₂-CH₂-O-, -CH₂-NH-, -C₃₋₆ cycloalkyl-O- or 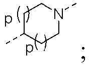 wherein p is each independently selected from 0 and 1, e.g., p is each 0, and L₃ is connected to the benzene ring of the general formula via a heteroatom; and
V is CH₂ or O.

**Embodiment 47.** The compound, or the stereoisomer, the solvate, the hydrate or the pharmaceutically acceptable salt thereof according to any one of the preceding embodiments, wherein L₀ is a single bond.

**Embodiment 48.** The compound, or the stereoisomer, the solvate, the hydrate or the pharmaceutically acceptable salt thereof according to any one of the preceding embodiments, wherein L₀ is NH.

**Embodiment 49.** The compound, or the stereoisomer, the solvate, the hydrate or the pharmaceutically acceptable salt thereof according to any one of the preceding embodiments, wherein V is O.

**Embodiment 50.** The compound, or the stereoisomer, the solvate, the hydrate or the pharmaceutically acceptable salt thereof according to any one of the preceding embodiments, wherein V is CH₂.

**Embodiment 51.** The compound, or the stereoisomer, the solvate, the hydrate or the pharmaceutically acceptable salt thereof according to any one of the preceding embodiments, wherein R₁ is halogen, e.g., Cl; and R₂ is CN.

**Embodiment 52.** The compound, or the stereoisomer, the solvate, the hydrate or the pharmaceutically acceptable salt thereof according to any one of the preceding embodiments, wherein
n is 1;
X is CH;
Y is CH₂;
Z is -C(O)-;
R₁ is halogen;
R₂ is halogen or CN;
L₁ is O;
R₃ is C₁₋₃ alkyl optionally substituted with halogen;
L₀ is a single bond;
W is 5-10-membered heteroaryl containing 1, 2 or 3 N heteroatoms, and being optionally substituted with 1 or 2 CN;
L₂ is selected from a single bond, -NH-C₁₋₄ alkyl-, -O-C₁₋₄ alkyl, -O-C₅₋₆ cycloalkyl, 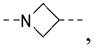 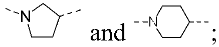 and L₂ is connected to W via a heteroatom when L₂ is not a single bond;
L₃- is selected from -C₁₋₄ alkyl-O-, -C₁₋₄ alkyl-NH-, 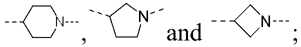 and L₃ is connected to the benzene ring of the general formula via a heteroatom; and
V is O.

**Embodiment 53.** The compound, or the stereoisomer, the solvate, the hydrate or the pharmaceutically acceptable salt thereof according to any one of the preceding embodiments, wherein R₁ is Cl.

**Embodiment 54.** The compound, or the stereoisomer, the solvate, the hydrate or the pharmaceutically acceptable salt thereof according to any one of the preceding embodiments, wherein R₂ is CN.

**Embodiment 55.** The compound, or the stereoisomer, the solvate, the hydrate or the pharmaceutically acceptable salt thereof according to any one of the preceding embodiments, wherein R₃ is ethyl or chloroethyl.

**Embodiment 56.** The compound, or the stereoisomer, the solvate, the hydrate or the pharmaceutically acceptable salt thereof according to any one of the preceding embodiments, wherein W is pyridinyl or pyrimidinyl, azaindazolyl or indazolyl (e.g. 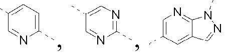 or 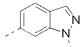 ); which is optionally substituted with 1 or 2 CN.

**Embodiment 57.** The compound, or the stereoisomer, the solvate, the hydrate or the pharmaceutically acceptable salt thereof according to any one of the preceding embodiments, wherein L₂ is connected to W via its heteroatom.

**Embodiment 58.** The compound, or the stereoisomer, the solvate, the hydrate or the pharmaceutically acceptable salt thereof according to any one of the preceding embodiments, wherein L₃ is connected to the benzene ring of the general formula via its heteroatom.

**Embodiment 59.** The compound, or the stereoisomer, the solvate, the hydrate or the pharmaceutically acceptable salt thereof according to any one of the preceding embodiments, wherein the compound is racemic.

**Embodiment 60.** The compound, or the stereoisomer, the solvate, the hydrate or the pharmaceutically acceptable salt thereof according to any one of the preceding embodiments, wherein the compound is selected from:

| | | | |
|---|---|---|---|
| **HANT-312** | 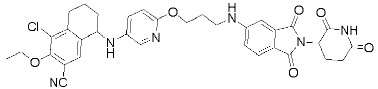 | **HAN T-393** | 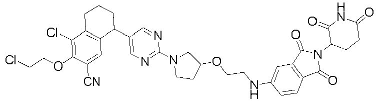 |
| **HANT-314** | 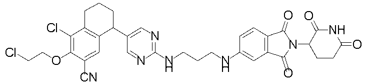 | **HAN T-431** | 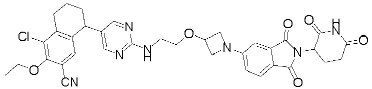 |
| **HANT-315** | 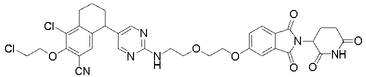 | **HAN T-501** | 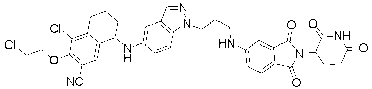 |
| **HANT-315-P1** | 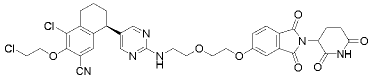 | **HAN T-502** | 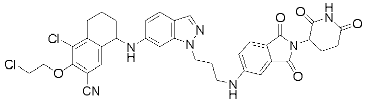 |
| **HANT-315-P2** | 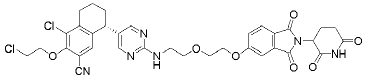 | **HAN T-503** | 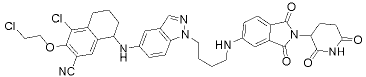 |
| **HANT-331** | 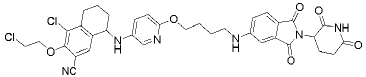 | **HAN T-504** | 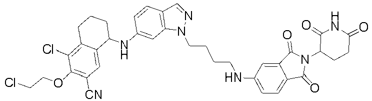 |
| **HANT-328** | 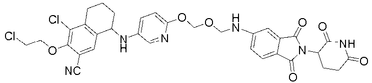 | **HAN T-329** | 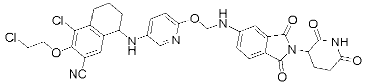 |
| **HANT-362** | 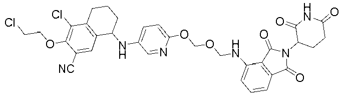 | **HAN T-369** | 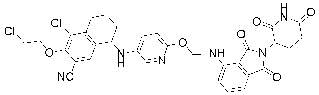 |
| **HANT-336** | 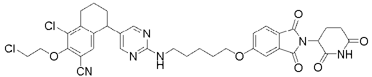 | **HAN T-505** | 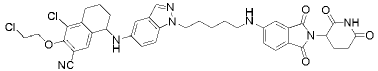 |
| **HANT-338** | 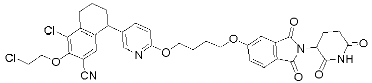 | **HAN T-506** | 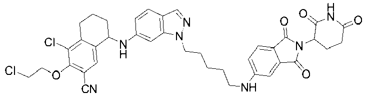 |
| **HANT-339** | 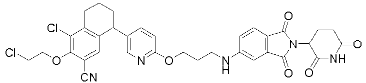 | **HAN T-507** | 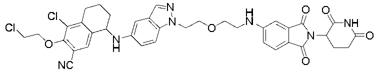 |
| **HANT-342** | 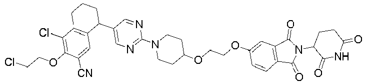 | **HAN T-508** | 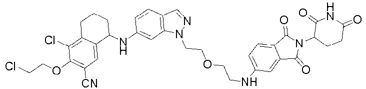 |
| **HANT-346** | 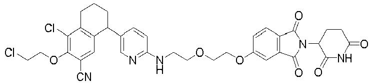 | **HAN T-509** | 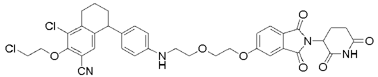 |
| **HANT-354** | 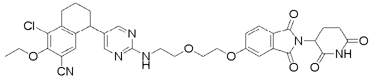 | **HAN T-510** | 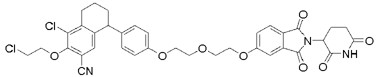 |
| **HANT-371** | 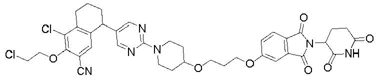 | **HAN T-511** | 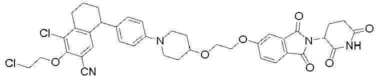 |
| **HANT-380** | 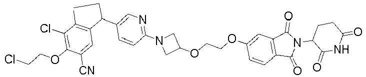 | **HAN T-512** | 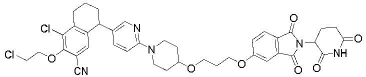 |
| **HANT-390** | 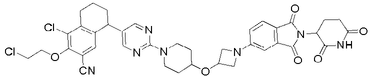 | **HAN T-513** | 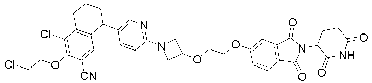 |
| **HANT-514** | 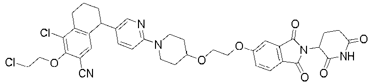 | **HAN T-515** | 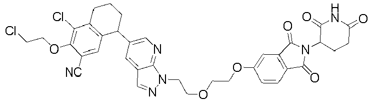 |
| **HANT-516** | 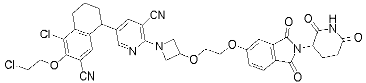 | **HAN T-517** | 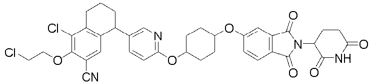 |
| **HANT-518** | 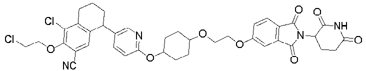 | **HAN T-519** | 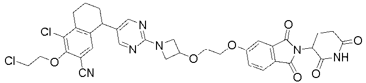 |

**Embodiment 61.** A pharmaceutical composition comprising the compound, or the stereoisomer, the solvate, the hydrate, or the pharmaceutically acceptable salt thereof according to any one of embodiments 1-60, and pharmaceutically acceptable excipients.

**Embodiment 62.** The compound, or the stereoisomer, the solvate, the hydrate, or the pharmaceutically acceptable salt thereof according to any one of embodiments 1-60, which is used as drugs.

**Embodiment 63.** The compound, the stereoisomer, the solvate, the hydrate or the pharmaceutically acceptable salt thereof according to any one of embodiments 1-60, which is used for treating or preventing androgen receptor-dependent diseases or disorders in individuals, in particular AR-SV-positive diseases or disorders or AR-V7-positive diseases or disorders; wherein the disorder or disease are selected, for example, from cancer, e.g., prostate cancer, denervation-resistant prostate cancer (CRPC), primary/localized prostate cancer, locally progressive prostate cancer, recurrent prostate cancer, non-metastatic castration-resistant prostate cancer (nmCRPC), metastatic castration-resistant prostate cancer, metastatic prostate cancer (mCRPC), hormone-sensitive prostate cance, other prostate diseases (e.g., prostate hyperplasia, prostatitis), breast cancer, non-small cell lung cancer, renal cell carcinoma, gonadal tumors, seminoma, pancreatic cancer, ovarian cancer, fallopian tube cancer, peritoneal cancer; salivary gland cancer; bladder cancer; acne; hirsutism; hidradenitis suppurativa; androgenetic alopecia, cryptorchidism, androgen insensitivity syndrome and Kennedy's disease; wherein the breast cancer includes, but is not limited to, Luminal A (ER+/PR+, HER-2-), Luminal B (ER+/PR+, HER-2+), HER-2+ (ER-/PR-/HER-2+) and Basal-like (ER-/PR-/HER-2-) breast cancer.

**Embodiment 64.** The use of the compound, the stereoisomer, the solvate, the hydrate or the pharmaceutically acceptable salt thereof according to embodiment 63, wherein the androgen receptor-dependent diseases or disorders have androgen receptor abnormal activity e.g. the androgen receptor abnormal activity is caused by an AR splice variation or an androgen receptor containing mutations or deletions in the LBD structural domain.

**Embodiment 65.** Use of the compound, or the stereoisomer, the solvate, the hydrate or the pharmaceutically acceptable salt thereof according to any one of embodiments 1-60 in the preparation of drugs for use in the treatment or prevention of diseases or disorders in individuals, wherein the disorders or diseases are androgen receptor-dependent diseases or disorders, in particular AR-SV-positive diseases or disorders or AR-V7-positive diseases or disorders; the disorders or diseases are selected, for example, from prostate cancer, e.g., castration-resistant prostate cancer, primary/localized prostate cancer, locally progressive prostate cancer, recurrent prostate cancer, non-metastatic castration-resistant prostate cancer, metastatic castration-resistant prostate cancer, metastatic prostate cancer, hormone-sensitive prostate cancer; other prostate diseases, e.g., prostate hyperplasia, prostatitis; breast cancer; non-small cell lung cancer; renal cell carcinoma; gonadal tumors; seminoma; pancreatic cancer; ovarian cancer; fallopian tube cancer; peritoneal cancer; salivary gland cancer; bladder cancer; acne; hirsutism; hidradenitis suppurativa; androgenetic alopecia; cryptorchidism; androgen insensitivity syndrome and Kennedy's disease; wherein the breast cancer includes, but is not limited to, Luminal A (ER+/PR+, HER-2-), Luminal B (ER+/PR+, HER-2+), HER-2+ (ER-/PR-/HER-2+) and Basal-like (ER-/PR-/HER-2-) breast cancer.

**Embodiment 66.** The use according to embodiment 65, wherein the androgen receptor-dependent diseases or disorders have androgenic abnormal activity, such as the androgenic abnormal activity is caused by an AR splice variation or an androgen receptor containing mutations or deletions in the LBD structural domain.

**Embodiment 67.** A method for treating or preventing diseases or disorders in individuals, the method comprising administering to the individual in need thereof an effective amount of the compound, the stereoisomer, the solvate, the hydrate or the pharmaceutically acceptable salt thereof according to any one of embodiments 1-60, wherein the disorders or diseases are androgen receptor-dependent diseases or disorders, and in particular AR-SV-positive diseases or disorders or AR-V7-positive diseases or disorders; wherein the disorders or diseases are, for example, selected from: prostate cancer, e.g., castration-resistant prostate cancer, primary/localized prostate cancer, locally progressive prostate cancer, recurrent prostate cancer, non-metastatic castration-resistant prostate cancer, metastatic castration-resistant prostate cancer, metastatic prostate cancer, hormone-sensitive prostate cancer; other prostate diseases, e.g., prostatehyperplasia, prostatitis; breast cancer; non-small cell lung cancer; renal cell carcinoma; gonadal tumors; seminoma; pancreatic cancer; ovarian cancer; fallopian tube cancer; peritoneal cancer; salivary gland cancer; bladder cancer; acne; hirsutism; hidradenitis suppurativa; androgenetic alopecia; cryptorchidism; androgen insensitivity syndrome and kennedy's disease; wherein the breast cancer includes, but is not limited to, Luminal A (ER+/PR+, HER-2-), Luminal B (ER+/PR+, HER-2+), HER-2+ (ER-/PR-/HER-2+) and Basal-like (ER-/PR-/HER-2-) breast cancer.

**Embodiment 68.** The method according to embodiment 67, wherein the androgen receptor-dependent diseases or disorders have androgenic abnormal activity, e.g. the androgenic abnormal activity is caused by an AR splice variation or an androgen receptor containing mutations or deletions in the LBD structural domain.

**Embodiment 69.** A method for inhibiting and/or degrading an androgen receptor in vivo or in vitro, in particular for modulating and/or degrading an AR full-length (AR-FL), an AR splice variation (AR-SV) and/or an androgen receptor containing mutations or deletions in the LBD structural domain, comprising administering an effective amount of the compound, the stereoisomer, the solvate, the hydrate or the pharmaceutically acceptable salt thereof according to any one of embodiments 1-60, in contact with the androgen receptor.

**Embodiment 70.** A pharmaceutical composition comprising the compound, the stereoisomer, the solvate, the hydrate or the pharmaceutically acceptable salt thereof according to any one of embodiments 1-60, and one or more other therapeutic agents.

It should be understood that within the scope of the present disclosure, the various embodiments described in the present disclosure (including the examples below) and the features in the various embodiments can be combined with each other at will, and the various embodiments resulting from these combinations are included within the scope of the present disclosure as if the embodiments resulting from these combinations were specifically and individually set forth herein, unless the context clearly indicates otherwise.

### Beneficial Effects

The compounds of the present disclosure are inhibitors of the N-terminus of the androgen receptor, are highly selective for the AR receptor, and show good inhibition of proliferation in cells expressing an AR splice variant (AR-SV) of the androgen receptor that lacks the ligand-binding domain (LBD), in particular cells expressing the androgen receptor shear mutation of an AR-SV that is resistant to a second generation AR antagonist.

In some embodiments, the compounds of the present disclosure are bifunctional compounds that inhibit and degrade androgen receptors, in particular inhibit and degrade AR full-length (AR-FL), AR splice variant (AR-SV), and androgen receptors containing mutations or deletions in the LBD structural domain, such as AR-V7.

In specific embodiments, the compounds of the present disclosure have an IC₅₀ value of ≤ 4 µM, preferably ≤ 1.5 µM, more preferably ≤ 1 µM, even more preferably ≤ 0.5 µM, and most preferably ≤ 0.2 µM, in AR inhibition and degradation assays.

### Terminology

In the present disclosure, unless otherwise explicitly stated, the terms used in the present disclosure have the meanings defined below. Terms not explicitly defined in the present disclosure have general meanings that are generally understood by those skilled in the art.

As used herein, the singular form and the words "the" and "said" are intended to include the plural form as well, unless the context clearly indicates otherwise.

A dash ("-") that is not between two letters or symbols indicates a connecting site for a substituent connecting to the rest of the molecule. In the case of a divalent group with a "-", it is connected to each of the other two variables in a manner consistent with the direction of connection of the corresponding variable shown in the general formula. For example, "L₂" is a divalent group which is connected to W and V in the general formula respectively in a W-L₂-V manner, e.g., when L₂ is -NH-C₁₋₆ alkyl-, it is connected to W via the N atom on the left side and to V on the other side.

As used herein, a "---" on a group indicates a linkage bond whereby the group is connected to the rest of the molecule in an arbitrary direction if not otherwise specified, e.g., a divalent group with two "---" is connected to the rest of the molecule in an arbitrary direction, the direction of its connection is arbitrary. For example, when W is connected to groups L₀ and L₂, respectively, and when W is 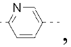 L₀ is NH, and L₂ is -O-CH₂-, then W can be formed either in left-to-right order with L₀ and L₂ to be 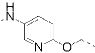 or in right-to-left order with L₀ and L₂ to be 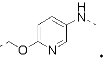 Groups containing two "---" are preferably connected in left-to-right order. Said combinations of connecting groups, substituents and/or variants thereof are permissible only if such combinations result in stable compounds.

As used herein, "heteroatom" refers to nitrogen (N), oxygen (O) or sulfur (S) atoms, particularly nitrogen or oxygen atoms, each of which can be substituted or unsubstituted, including their oxidized forms. Examples of heteroatoms include but are not limited to -O-, -N=, -NR-, -S-, -S(O)- and -S(O)₂-, wherein R is hydrogen, C₁-C₄ alkyl or nitrogen protective groups.

As used herein, "halogen" or "halo" refers to fluorine, chlorine, bromine and iodine. The preferred halogen as a substituent is fluorine and chlorine, preferrely is chlorine.

As used herein, "alkyl" refers to a monovalent hydrocarbon group of completely saturated straight or branched chains. Alkyl preferably contains 1-20 carbon atoms (C₁₋₂₀), more preferably 1-10 carbon atoms (C₁₋₁₀), 1-8 carbon atoms (C₁₋₈), 1-6 carbon atoms (C₁₋₆), 1-4 carbon atoms (C₁₋₄), 1-3 carbon atoms (C₁₋₃) or 2-3 carbon atoms (C₂₋₃). "C₁-C₆ alkyl" refers to an alkyl with 1-6 carbon atoms, and "C₁-C₄ alkyl" refers to an alkyl with 1, 2, 3 or 4 carbon atoms. Exemplary examples of alkyl include but are not limited to methyl, ethyl, n-propyl, isopropyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, etc.

As used herein, "alkenyl" refers to a monovalent hydrocarbon group of straight or branched chains containing one or more, e.g., 1, 2 or 3 carbon-carbon double bonds (C=C). Alkenyl preferably contains 2-20 carbon atoms (C₂₋₂₀), more preferably 2-10 carbon atoms (C₂₋₁₀), 2-6 carbon atoms(C₂₋₆), 2-4 carbon atoms (C₂₋₄), 2-3 carbon atoms (C₂₋₃) or 2-3 carbon atoms (C₂₋₃). For example, "C₂₋₆ alkenyl" refers to an alkenyl group having 2-6 carbon atoms.

As used herein, "alkoxy" refers to an alkyl-O group, wherein alkyl as defined above. "C₁-C₆ alkoxy" refers to an alkoxy with 1-6 carbon atom.

As used herein, "halo C₁₋₆ alkyl" (e.g., halo C₁₋₄ alkyl) refers to C₁-₆ alkyl (e.g., C₁₋₄ alkyl) as defined above where one or more (e.g., 1, 2, 3 or 4) hydrogen atoms are substituted with one or more halogens, for example, fluoromethyl, difluoromethyl, trifluoromethyl, difluoroethyl, trifluoroethyl, chloromethyl, dichloromethyl, trichloromethyl, chloroethyl, dichloroethyl, trichloroethyl etc.

As used herein, "cycloalkyl" refers to a saturated or unsaturated monocyclic, bicyclic or tricyclic hydrocarbon group with 3 to 12 ring carbon atoms, preferably 3-8 ring carbon atoms, for example, 3-8, 3-7, 3-6, 4-7 or 5-6 ring carbon atoms. Exemplary monocyclic cycloalkyls include but are not limited to cyclopropyl, cyclobutyl, cyclopentyl, cyclopentenyl, cyclohexyl and cyclohexenyl. Exemplary bicyclic cycloalkyls include bornyl, decahydronaphthyl, bicyclo[2.1.1]hexyl, bicyclo[2.2.1]heptyl, bicyclo[2.2.1]heptenyl, 6,6-dimethyldicyclo[3.1.1]heptyl, 2,6,6-trimethyldicyclo[3.1.1]heptyl, bicyclo[2.2.2]octyl, etc.

As used herein, "aryl" refers to a carbocyclic hydrocarbon ring group consisting of one ring or more fused rings (such as two fused rings) containing 6-12 carbon atoms, at least one of the rings is an aromatic ring. Aryl includes but are not limited to phenyl, naphthyl, 1,2,3,4-tetrahydronaphthyl, preferably phenyl.

As used herein, "heteroaryl" refers to a mono- or bicyclic ring system having 5-14 (5-14 membered), 5-10 (5-10 membered), e.g. 5-9 (5-9 membered), e.g. 5-6 (5-6 membered) or 6 (6 membered) ring atoms, which comprises 1, 2, 3 or 4 heteroatoms independently selected from N, O and S, the remaining ring atoms being carbon atoms, wherein at least one of the rings is an aromatic ring containing a heteroatom, for example, comprises 1 N heteroatom and optionally ffurther comprises 1, 2 or 3 heteroatoms independently selected from N, O and S. When the total number of S and O atoms in heteroaryl exceeds 1, these S and O heteroatoms are not adjacent to each other. For example, heteroaryl includes, but is not limited to:
5-6-membered monocyclic heteroaryl, i.e. a monocyclic aromatic hydrocarbon group having 5 or 6 ring atoms comprising 1, 2 or 3 heteroatoms independently selected from N, O and S with the remaining ring atoms being carbon atoms; for example, comprising 1 N heteroatom and optionally further comprising 1 or 2 heteroatoms independently selected from N, O and S, e.g. comprising 1, 2 and 3 N heteroatoms, and the remaining ring atoms being carbon atoms. Heteroaryl is, for example, pyrrolyl, pyrazolyl, pyrazinyl, pyridazinyl, pyridinyl or pyrimidinyl; and
8-10-membered bicyclic heteroaryl, i.e., a bicyclic aromatic hydrocarbon group having 8, 9 or 10 ring atoms comprising 1, 2, 3 or 4 heteroatoms independently selected from N, O and S, and the remaining ring atoms being carbon atoms, wherein at least one of the rings is an aromatic ring containing a heteroatom; for example, comprises 1 N heteroatom and optionally further comprises 1, 2, 3 or 4, e.g., 1, 2 or 3, independently selected from N, O and S heteroatoms, e.g. comprising 1, 2 or 3 N heteroatoms and the remaining ring atoms are carbon atoms. Heteroaryl is, for example, an 8-9 membered bicyclic heteroaryl, such asbenzimidazolyl, indazolyl, azaindazolyl, benzotriazolyl or indolyl.

For example, examples of the above heteroaryl include, but are not limited to: pyrrolyl, furanyl, thienyl, pyrazolyl, imidazolyl, thiazolyl, isothiazolyl, indolyl, benzotriazolyl, benzoimidazolyl, benzofuranyl, quinolinyl, isoquinolinyl, cinnolinyl, quinazolinyl, quinoxalinyl, tetrahydroquinolinyl, oxazolopyridinyl, 7-azaindolyl, 6-azazaindolyl, 5-azaindolyl, 4-azaindolyl, indazolyl (e.g. 1*H*-indazolyl), 7-azaindazolyl, 6-azaindazolyl, 5-azaindazolyl, 4-azaindazolyl, pyridyl, pyrimidinyl, pyridazinyl, pyrazinyl or imidazo[1,2-a]pyridine, preferably pyridinyl, pyrimidinyl, indazolyl (e.g. 1*H-*indazolyl).

Exemplary examples of heteroaryl include

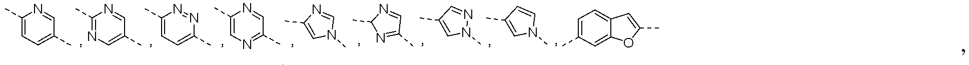

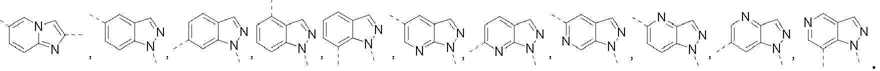

As used herein, "heterocyclic" and "heterocyclyl" are used interchangeably to refer to a fully saturated or partially saturated, non-aromatic, monocyclic, bicyclic or tricyclic cyclic group having 3-15 ring atoms (e.g., 4-12 ring atoms, 3-10 ring atoms, 5-10 ring atoms, 4-7 ring atoms, 5-6 ring atoms, 7-12 ring atoms, 10-15 ring atoms), e.g., which is a ring system of a 4- to 7-membered monocyclic ring, a 7- to 12-membered bicyclic ring or a 10- to 15-membered tricyclic ring, and which comprises 1, 2, 3 or 4 heteroatoms independently selected from N, O, and S, e.g., comprising 1 N heteroatom and optionally further comprising 1, 2 or 3 heteroatoms independently selected from N, O and S, the remaining ring atoms being carbon atoms. The nitrogen and sulfur heteroatoms may optionally be oxidized, e.g., the sulfur heteroatoms may form a -S(O)- or -S(O)₂- structure. Heterocyclyl may be a monocyclic group, a bicyclic group, a fused cyclic group, a spirocyclic group or a bridging cyclic group. "5-6-membered heterocyclyl" indicates heterocyclyl having 5 or 6 ring atoms comprising 1, 2, 3 or 4 heteroatoms independently selected from N, O and S, e.g. containing 1, 2 or 3 N heteroatoms, which is monocyclic.

As used herein, "heterocycloalkyl" is a fully saturated heterocyclyl group as defined herein. For example, "3-10-membered heterocycloalkyl" comprises a saturated heterocyclic ring having 3-10 ring atoms comprising 1, 2, 3 or 4 heteroatoms independently selected from N, O, and S. "4-6-membered heterocycloalkyl" comprises a saturated heterocyclic ring having 4-6 ring atoms comprising 1, 2, 3 or 4 heteroatoms independently selected from N, O, and S, for example, comprising 1 N heteroatom.

Exemplary examples of monocyclic heterocycle include fully saturated or partially saturated pyrrolidane, oxetane, oxolane, oxane, pyrazoline, imidazoline, imidazolidine, thiazolidine, isothiazolidine, tetrahydrofuran, piperidine, piperazine, 2-oxopiperazine, 2-oxopiperidine, 2-oxopyrrolidine, 4-piperidone, imidazolidinone, tetrahydropyran, morpholine, thiomorpholine, thiomorpholine sulfoxide, thiomorpholine sulfone, 1,3-dioxolane and tetrahydro-1,1-dioxothiophene, 1,1,4-trioxo-1,2,5-thiadiazolidin-2-yl, and the like.

Exemplary examples of bicyclic heterocycle include fully saturated or partially saturated indoline, isoindoline, dihydroindoline, hexahydroindoline, octahydropyrrole, dihydrobenzofuran, tetrahydroquinoline, tetrahydroisoquinoline, and the like.

Exemplary examples of heterocyclyl include epoxyethyl, aziridinyl, oxetanyl, azetidinyl, pyrrolidinyl, tetrahydrofuranyl, tetrahydropyranyl, pyrazolidinyl, imidazolidinyl, piperidinyl, piperazinyl, morpholinyl, thiomorpholinyl, 2-oxopiperazinyl, hexahydropyrimidinyl, hexahydroindolyll, imidazolidinonyl, octahydropyrrolinyl,

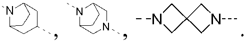

Exemplary examples of heterocycloalkyl include epoxyethyl, oxetanyl, tetrahydrofuranyl, pyrazolidinyl, imidazolidinyl,

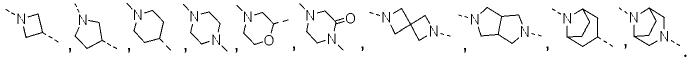

As used herein, "C(O)" refers to the group -C(=O)-, i.e., carbonyl. "Oxo" refers to the group -(=O)-.

As used herein, "optional" or "optionally" refers to the events described subsequently that may or may not occur, and this description includes both the situations in which the event occurred and the situations in which it did not occur. For example, "optionally substituted alkyl" includes "unsubstituted alkyl" and "substituted alkyl" as defined herein. Those skilled in the art should understand that for any functional group containing one or more substituents, the group does not include any spatially impractical, chemically incorrect, synthetic infeasible, and/or inherently unstable substitution patterns.

As used herein, "substituted" or being substituted" refers to one or more hydrogen atoms (i.e. 1, 2, 3 or 4) on a given atom or group being replaced by one or more substituents (i.e. 1, 2, 3 or 4) selected from a given substituent group, provided that they do not exceed the normal valence of the given atom, the substituents can be the same or different from each other. Only when the combination of substituents and/or variables leads to chemically correct and stable compounds, such combinations are allowed. A chemically correct and stable compound means that it is sufficiently stable to be separated from the reaction mixture and its chemical structure can be determine. Alkyl, alkenyl, alkoxy, cycloalkyl, heteroaryl, heterocyclyl, heterocycloalkyl, carbonyl, sulfonyl, sulfinyl, and other functional groups as used herein can be substituted with substituents, and the substituents include but not limited to OH, Boc, halogen, cyanide, substituted or unsubstituted alkyl, substituted or unsubstituted alkoxy, substituted or unsubstituted cycloalkyl, substituted or unsubstituted heterocycloalkyl; NRR', C(O)R, C(O)NRR' or C(O)OR, and R and R' are each independently selected from H and substituted or unsubstituted alkyl.

As used herein, "pharmaceutically acceptable salt" includes both acid addition salts and base addition salts. Pharmaceutically acceptable salt includes those obtained by reacting an active compound acting as a base with an inorganic or organic acid to form a salt, such as hydrochloride, hydrobromide, sulphate, nitrate, phosphate, methanesulfonate, oxalate, maleate, succinate, citrate, formate, benzoate, fumarate, tartrate, salicylate, mandelate, carbonate, etc. It is known to those skilled in the art that acid addition salts are prepared by reaction of the described compounds with suitable inorganic or organic acids by any of numbers of known methods.

As used herein, "pharmaceutically acceptable excipient" includes solvents, dispersants, coatings, surfactants, antioxidants, preservatives (such as antibacterial agents, antifungal agents), isotopes, absorption retardants, salts, preservatives, drugs, drug stabilizers, adhesives, excipients, disintegrants, lubricants, sweeteners, correctors, dyes, similar substances and their combinations, which is well-known to those skilled in the art.

Compounds of the present disclosure or pharmaceutically usable salts thereof contain one or more asymmetric centers, thus producing enantiomers; diastereomers; and other stereoisomers that can be defined as (R)- or (S)- in absolute stereochemistry. It is intended herein to include all such possible isomers as well as their racemic and optically pure forms, whether or not they are specifically described herein. Optically active (+)- and (-)-, (R)- and (S)- isomers may be prepared using chiral synthesis or chiral reagents or split by conventional techniques such as chromatography and hierarchical crystallization. Conventional techniques for the preparation/separation of individual enantiomers include chiral synthesis from suitable optically pure precursors or splitting of racemates (or racemates of salts or derivatives) using, for example, chiral high-pressure liquid chromatography (HPLC).

"Stereoisomer" refers to compounds consisting of the same atoms bonded by the same bonds but having different three-dimensional structures that are not interchangeable. The present disclosure contemplates a variety of stereoisomers and mixtures thereof, and includes "enantiomer", which refers to two such stereoisomers whose molecules are non-overlapping mirror images of each other.

As used herein, "effective amount" refers to the amount of the compound of the present disclosure that can cause individual biological or medical reactions, improve symptoms, slow or delay disease progression, or prevent disease.

As used herein, "individual" or "patient" refers to animals. Preferably, animals are mammals, such as primates (for example, humans), cows, sheep, goats, horses, dogs, cats, rabbits, rats, mice, fish, birds, etc. In a preferred embodiment, the individual is a human.

As used herein, "treating" diseases or disorders refer to the administration of one or more pharmaceutical substances, in particular the compounds of the present disclosure or pharmaceutically acceptable salts thereof, to individuals suffering from said diseases or disorders, or having symptoms of said diseases or disorders, for the purpose of curing, healing, relieving, alleviating, altering, healing, improving, ameliorating, or affecting said diseases or disorders, or symptoms of said diseases or disorders. In some embodiments, said diseases or disorders are androgen receptor-dependent diseases or disorders, in particular AR-SV-positive diseases or disorders or AR-V7-positive diseases or disorders; wherein said disorders or diseases are, for example, selected from: prostate cancer, such as desmoplasia-resistant prostate cancer (CRPC), primary/localized prostate cancer, locally-progressive prostate cancer, recurrent prostate cancer, non metastatic desmoplasia-resistant prostate cancer (nmCRPC), metastatic desmoplasia-resistant prostate cancer, metastatic prostate cancer (mCRPC), hormone-sensitive prostate cancer; other prostate diseases such as prostatic hyperplasia, prostatitis; breast cancer; non-small cell lung cancer; renal cell carcinoma; gonadal tumors; seminoma; pancreatic cancer; ovarian cancer; fallopian tube cancer; peritoneal cancer; salivary gland cancer; bladder cancer; acne; hirsutism; hidradenitis suppurativa; androgenetic alopecia; cryptorchidism; androgen insensitivity syndrome and kennedy's disease; wherein breast cancer including, but not limited to, Luminal type A (ER+/PR+, HER-2-), Luminal type B (ER+/PR+, HER-2+), HER-2+ type (ER-/PR-/HER-2+), and Basal-like type (ER-/PR-/HER-2-) breast cancer.

As used herein, "preventing" diseases refers to the administration of one or more pharmaceutical substances, particularly the compounds of the present disclosure, to individuals with a predisposition to, or is at risk for, said disease or disorder, for the purpose of preventing or slowing the development of said disease or disorder in said individual.

As used herein, "androgen receptor-dependent diseases or disorders" refers to a medical condition that is partially or wholly dependent on, or sensitive to, androgen activity in the body or activation of the AR axis. Said androgen receptor dependent diseases or disorders include androgen receptor positive diseases. Androgen receptors include AR full-length (AR-FL), AR splice variant (AR-SV) and/or androgen receptors containing mutations or deletions in the LBD structural domain. AR-V7 is a splice variant of AR that lacks the LBD.

All numerical ranges herein should be understood as disclosing each and all values in the range and each and all subsets of values in the range, regardless of whether they are otherwise specifically disclosed. For example, when referring to any range of values, it should be considered to be every value in a range of exponential values, e.g., every integer in a range of values. The present disclosure includes all values that fall within these ranges, all lesser ranges, and upper or lower limits of the range.

### Pharmaceutical Composition and Administration

The compound of the present disclosure (e.g., any of the compounds in the examples herein) alone or in combination with one or more additional therapeutic agents can be formulated into a pharmaceutical composition. The pharmaceutical composition comprises: (a) the compound of the present disclosure; (b) a pharmaceutically acceptable excipient; and optionally (c) at least one additional therapeutic agent.

A pharmaceutically acceptable excipient refers to a carrier or excipient that is compatible with the active ingredient in the composition (in some embodiments, stabilizes the active ingredient) and is not harmful to the individual being treated. Suitable pharmaceutically acceptable carriers are disclosed in standard references in the field (e.g., Remington's Pharmaceutical Sciences, Remington: The Science and Practice of Pharmacy).

The compound of the present disclosure can be administered in various known ways, such as orally, parenterally, by inhalation or by implantation. The term "parenterally " as used herein includes subcutaneous, intradermal, intravenous, intramuscular, intra-articular, intra-arterial, synovial, intrasternal, intraspinal, intra-affected, and intracranial injections or infusions.

The compound of the present disclosure may be administered in any convenient formulation, such as tablets, powders, capsules, solutions, dispersions, suspensions, syrups, sprays, suppositories, gels, emulsions, patches, and the like.

In one embodiment, the effective amount of the compound of the present disclosure administered parenterally per dose will be in the range of about 0.01 to about 100 mg/kg of patient body weight/day, or about 0.1 to about 20 mg/kg of patient body weight/day, wherein a typical initial range of the compound used is from 0.3 to 15 mg/kg/day. In another embodiment, oral unit dosage forms, such as tablets and capsules, contain from about 0.1 to about 1000 mg of the compound of the present disclosure.

### Indications and treatments

The compounds of the present disclosure are AR inhibitors with high selectivity for AR receptors, particularly for AR full-length (AR-FL), AR splice variant (AR-SV) and androgen receptors containing mutations or deletions in the LBD structural domain. The compounds of the present disclosure are particularly bifunctional compounds that inhibit and degrade androgen receptors, especially inhibit and degrade AR full-length (AR-FL), AR splice variant (AR-SV), and androgen receptors containing mutations or deletions in the LBD structural domain, such as AR-V7.

The present disclosure relates to a method of treating or preventing androgen receptor-dependent diseases or disorders, in particular AR-SV-positive diseases or disorders, AR-V7-positive diseases or disorders, or diseases or disorders comprising mutations or deletions in the LBD structural domain, the method comprising administering an effective amount of the compound of the present disclosure to an individual in need thereof.

The present disclosure relates to a method of treating or preventing diseases or disorders, the method comprising administering an effective amount of the compounds of the present disclosure to an individual in need thereof, the diseases or disorders being selected from: prostate cancer, ssuch as castration-resistant prostate cancer (CRPC), primary/localized prostate cancer, locally-progressive prostate cancer, recurrent prostate cancer, non-metastatic castration-resistant prostate cancer (nmCRPC), metastatic castration-resistant prostate cancer, metastatic prostate cancer (mCRPC), hormone-sensitive prostate cancer; other prostate diseases such as prostate hyperplasia, prostatitis; breast cancer; non-small cell lung cancer; renal cell carcinoma; gonadal tumors; seminoma; pancreatic cancer; ovarian cancer; fallopian tube cancer; peritoneal cancer; salivary gland cancer; bladder cancer; acne; hirsutism; hidradenitis suppurativa; androgenic alopecia; cryptorchidism; androgen insensitivity syndrome and Kennedy's disease; wherein the breast cancer includes, but is not limited to, Luminal A type (ER+/PR+, HER-2-), Luminal B type (ER+/PR+, HER-2+), HER-2+ type (ER-/PR-/HER-2+) and Basal-like type (ER-/PR-/HER-2-) breast cancer.

In one embodiment, the compounds of the present disclosure are used to treat or prevent androgen receptor-dependent diseases or disorders, in particular AR-SV-positive diseases or disorders, AR-V7-positive diseases or disorders, or diseases or disorders comprising mutations or deletions in the LBD structural domain.

In one embodiment, the compounds of the present disclosure are used for the treatment or prevention of prostate cancer, such as castration resistant prostate cancer (CRPC), primary/localized prostate cancer, locally progressive prostate cancer, recurrent prostate cancer, non-metastatic castration resistant prostate cancer (nmCRPC), metastatic castration resistant prostate cancer, metastatic prostate cancer (mCRPC), hormonesensitive prostate cancer; other prostate diseases such as prostate hyperplasia, prostatitis; breast cancer; non-small cell lung cancer; renal cell carcinoma; gonadal tumors; seminoma; pancreatic cancer; ovarian cancer; fallopian tube cancer; peritoneal cancer; salivary gland cancer; bladder cancer; acne; hirsutism; hidradenitis suppurativa; androgenetic alopecia; cryptorchidism; androgen insensitivity syndrome; and Kennedy's disease; wherein breast cancer includes but is not limited to Luminal A type (ER+/PR+, HER-2-), Luminal B type (ER+/PR+, HER-2+), HER-2+ type (ER-/PR-/HER-2+) and Basal-like type (ER-/PR-/HER-2-) breast cancer.

### Pharmaceutical Combinations

The compound of the present disclosure can be used in combination with an additional therapeutic agent for the treatment of androgen receptor-dependent diseases or disorders, in particular AR-SV-positive diseases or disorders, AR-V7-positive diseases or disorders, or diseases or disorders comprising mutations or deletions in the LBD structural domain. The additional therapeutic agent can be administered separately from the compound of the present disclosure or can be included with the compound of the present disclosure in the pharmaceutical composition according to the present disclosure, such as a fixed combination product. In some embodiments, the additional therapeutic agents are those known or found to be effective in the treatment of androgen receptor-dependent diseases or disorders, or compounds that antagonize additional targets associated with the specific diseases. The combination may be used to increase the efficacy of the compound of the present disclosure, reduce one or more side effects, or reduce the dose required.

In some embodiments, the compound of the present disclosure is administered in combination with an antitumor agent. The antitumor agent includes, but is not limited to: radiotherapeutic agent, chemotherapeutic agent, immunotherapeutic agent, targeted therapeutic agent.

### General synthetic procedures

In an embodiment, the compounds of the present disclosure can be synthesized through the following general synthesis scheme, where each variable is defined herein, and the specific reaction conditions are the same as in the examples.

Starting from 6-hydroxy-1-tetrahydronaphthalenone, the 5,7-disubstituted alkoxynaphthalenone was obtained by halogenation, coupling and alkylation reactions, which reacted with Tf₂O to afford an important intermediate INT-2/4; this intermediate can react with different boric acids or boronate esters via Suzuki reaction, followed by reductive hydrogenation and Boc deprotection, and then reacted with fluoro-substituted thalidomide to afford the first type of target product TM1 (Scheme 1). Alternatively, starting from alkoxynaphthones, the carbonyl was reduced to an alcohol and reacted with PBr₃ to obtain an important intermediate INT-1/3, which then reacted with ammonia to convert Br to amine, and the afforded benzyl amine can be subjected to a Buckwald coupling reaction with bromoaryl/heteroaryl, followed by Boc deprotection, and then reacted with fluoro-substituted thalidomide to afford the second type target product TM2; or starting from INT-1/3, INT-1/3 directly reacted with aminoaryl/heteroaryl by substitution, then followed by similar deprotection and substitution reaction to afford product TM2 (Scheme 2).

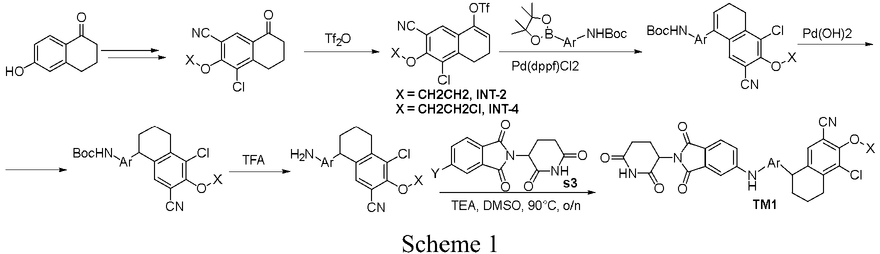

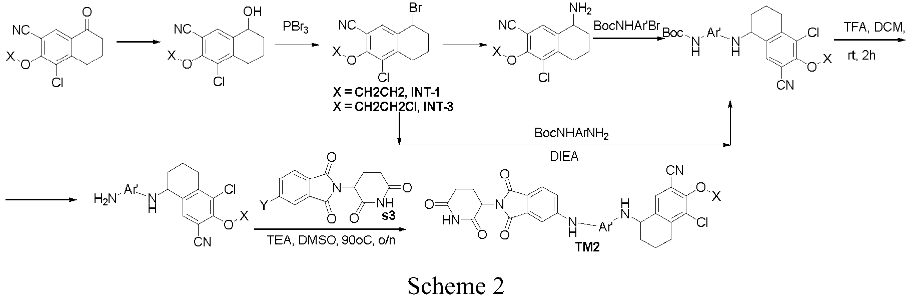

### DETAILED DESCRIPTION OF THE INVENTION

In the present application, when the chemical name and structural formula are inconsistent, the one shown in the structural formula shall prevail, unless it can be inferred from the context that the chemical name rather than the structural formula is correct.

The present disclosure will be further described in combination with specific examples. It should be understood that these examples are only used to illustrate the present disclosure and not to limit the scope of the present disclosure. The experimental methods without specific conditions in the following embodiments are usually under conventional conditions, or the conditions recommended by the manufacturer. Unless otherwise stated, percentages and parts are weight percentages, weight parts.

The experimental materials and reagents used in the following examples can be obtained from commercial channels unless otherwise stated.

In each example, experimental instrument descriptions (e.g., ¹H NMR was recorded by a Varian Mercury-300 or Varian Mercury-400 NMR instrument, ¹³C NMR was recorded by a Varian Mercury-400 or Varian Mercury-500 or Varian Mercury-600 NMR instrument, chemical shifts were expressed as δ (ppm); mass spectra were recorded by Finnigan/MAT-95 (EI) with Finnigan LCQ/DECA and Micromass Ultra Q-TOF (ESI) type mass spectrometers; silica gel for reversed-phase HPLC separations was 200-300 mesh. The method of SFC purification is (Column: Chiralpak IG 250mm*4.6mm 5um, mobile phase: Hex-EtOH, 30°C).

Wherein, the Chinese names of the reagents represented by the chemical formula or alphabetical abbreviation are listed below:
DCM: Dichloromethane; DCE: 1,2-dichloroethane; THF: Tetrahydrofuran; DMF: N,N-dimethylformamide; Tol: Toluene; TFA: Trifluoroacetic acid; PE: Petroleum ether; EA: Ethyl acetate; TEA: triethylamine; DIEA: N,N-diisopropylethylamine; Tf₂O: Trifluoromethanesulfonic anhydride; DBAD: Dibenzyl azodicarboxylate; KOAc: Potassium acetate; NMP: 1-methyl-2-pyrrolidinone; NIS: N-Iodosuccinimide; Pd(dppf)Cl₂: 1,1'-bis(diphenylphosphino)ferrocene palladium dichloride; 9-BBN: 9-Borabicyclo[3.3.1]nonane; RT: Retention time; LCMS: Liquid chromatography-mass spectrometry.

### Synthesis of Key Intermediates

### Intermediate INT-1

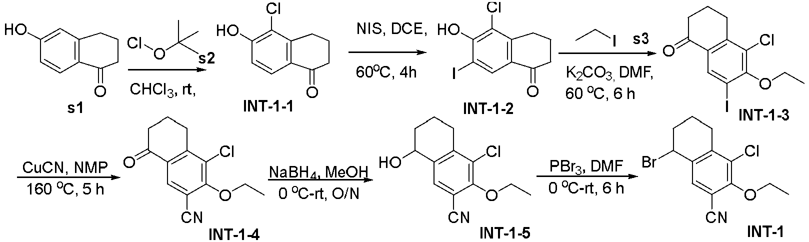

### Step 1: Synthesis of 5-chloro-6-hydroxy-3,4-dihydronaphthalen-1(2H)-one

Compound **S1** (20 g, 125 mmol) was dissolved in trichloromethane (300 mL), then S2 (12 g, 110 mmol) was added at 0 °C and stirred overnight at room temperature. The reaction was concentrated to obtain **INT-1-1** as a solid (18 g, yield: 75%). LCMS (ESI): *m*/*z* =197.0 [M+H]⁺. **Step 2:** Synthesis of 5-chloro-6-hydroxy-7-iodo-3,4-dihydronaphthalen-1(2H)-one

Compounds **INT-1-1** (18 g, 91.8 mmol), NIS (26 g, 115 mmol) were dissolved in DCE (300 mL) and reacted at 60 °C for 4 h. The reaction was cooled to room temperature, filtered, and concentrated under reduced pressure to afford a brown solid **INT-1-2** (20 g, yield: 77%). LCMS (ESI): *m*/*z* = 322.9 [M+H]⁺.

### Step 3: Synthesis of 5-chloro-6-ethoxy-7-iodo-3,4-dihydronaphthalen-1(2H)-one

To a dry flask was sequentially added **INT-1-2** (1 g, 3.1 mmol), **S3** (960 mg, 6.2 mmol) DMF (15 mL), and potassium carbonate (1.3 g, 9.3 mmol), and the reaction was stirred at 60 °C for 6 hours. The reaction was cooled to room temperature, poured into EA and washed with saturated brine. The organic phase was collected and concentrated under reduced pressure, and the residue was purified by flash chromatography to obtain yellow solid INT-1-3 (370 mg, yield: 34%). LCMS (ESI): *m*/*z =351.0* [M+H]⁺.

### Step 4: Synthesis of 4-chloro-3-ethoxy-8-oxo-5,6,7,8-tetrahydronaphthalene-2-carbonitrile

**INT-1-3** (320 mg, 0.91 mmol) and CuCN (410 mg, 4.6 mmol) were dissolved in NMP (15 mL) in a dry sealed tube and stirred at 160 °C for 5 hours. The reaction was cooled to room temperature, quenched with ammonia then poured into EA and washed with aqueous NH₄Cl. The organic phase was collected and concentrated under reduced pressure, and the residue was purified by flash chromatography to obtain **INT-1-4** (140 mg, yield: 54%) as a yellow solid. LCMS (ESI): *m*/*z* =250.1 [M+H]⁺.

### Step 5: Synthesis of 4-chloro-3-ethoxy-8-hydroxy-5,6,7,8-tetrahydronaphthalene-2-carbonitrile

**INT-1-4** (140 mg, 0.56 mmol) was dissolved in MeOH (10 mL) in a dry flask and NaBH₄ (85 mg, 2.2 mmol) was added under an ice bath, then the reaction was stirred overnight at room temperature. The reaction was quenched with water, concentrated under reduced pressure, and the residue was purified by flash chromatography to obtain **INT-1-5** (110 mg, yield: 78%) as a white solid. LCMS (ESI): *m*/*z* =251.1 [M+H]⁺.

### Step 6: Synthesis of 8-bromo-4-chloro-3-ethoxy-5,6,7,8-tetrahydronaphthalene-2-carbonitrile

**INT-1-5** (110 mg, 0.44 mmol) was dissolved in DMF (10 mL) in a dry flask and PBr₃ (590 mg, 2.2 mmol) was added at 0 °C and stirred for 6 hours at room temperature. The reaction was quenched with aqueous NH₄Cl, extracted by EA, and the organic phase was washed with saturated brine, dried and concentrated under reduced pressure. The residue was purified by flash chromatographyto obtain **INT-1** (80 mg, yield: 51%) as a white solid. LCMS (ESI): m/z =314.0 [M+H]⁺; ¹H NMR (400 MHz, CDCl₃) δ 7.53 (s, 1H), 5.46 (brs, 1H), 4.25 (q, *J* = 6.9 Hz, 2H), 3.16-3.08 (m, 1H), 2.79-2.70 (m, 1H), 2.40-2.35 (m, 1H), 2.26-2.21 (m, 1H), 2.10-1.99 (m, 2H), 1.49 (t, *J =* 6.9 Hz, 2H).

### Intermediate INT-2

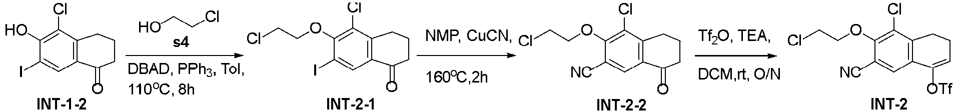

### Step 1: Synthesis of 5-chloro-6-(2-chloroethoxy)-7-iodo-3,4-dihydronaphthalen-1(2H)-one

**INT-1-2** (20 g, 62 mmol), **S4** (15 g, 186 mmol), **DBAD** (14 g, 61 mmol) and PPh₃ (16 g, 61 mmol) were dissolved in Tol (300 mL) and reacted under N₂ protection at 110 °C for 8 h. The reaction was cooled to room temperature and concentrated under reduced pressure. The residue was purified by flash chromatography to obtain a white solid **INT-2-1** (15 g, yield: 85%). LCMS (ESI): *m*/*z* =384.9 [M+H]⁺.

### Step 2: Synthesis of 4-chloro-3-(2-chloroethoxy)-8-oxo-5,6,7,8-tetrahydronaphthalene-2-carbonitrile

**INT-2-1** (5 g, 13 mmol) and CuCN (2.4 g, 26 mmol) were dissolved in a sealed tube with NMP (80 mL) and reacted under N₂ protection at 120 °C for 8 h. The reaction solution was to room temperature, extracted with EA, and the organic phase was concentrated under reduced pressure. The residue was purified by flash chromatography to obtain **INT-2-2** as a white solid (3.2 g, yield: 86%). LCMS(ESI): m/z =284.0[M+H]⁺.

### Step 3: Synthesis of 5-chloro-6-(2-chloroethoxy)-7-cyano-3,4-dihydronaphthalen-1-yl trifluoromethanesulfonate

A three-neck flask was charged with **INT-2-2** (3.2 g, 11.3 mmol), TEA (3.4 g, 33.9 mmol), DCM (100 mL), and Tf₂O (16 g, 56.5 mmol) was added at 0 °C under N₂ protection and the reaction was stirred at room temperature overnight. The reaction was extracted with DCM, and the organic phase was concentrated under reduced pressure. The residue was purified by flash chromatography to obtain **INT-2** (3.5 g, yield: 66%) as a yellow solid. LCMS (ESI): m/z = 416.0 [M+H]⁺; ¹H NMR (400 MHz, CDCl₃) δ 7.46 (s, 1H), 6.15 (t, *J =* 4.8 Hz, 1H), 4.48 (t, *J =* 6.0 Hz, 2H), 3.90 (t, *J =* 6.0 Hz, 2H), 3.08 (t, *J =* 8.3 Hz, 2H), 2.66-2.56 (m, 2H).

### Intermediate INT-3

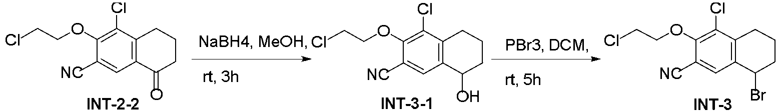

### Step 1: Synthesis of 4-chloro-3-(2-chloroethoxy)-8-hydroxy-5,6,7,8-tetrahydronaphthalene-2-carbonitrile

**INT-2-2** (800 mg, 2.8 mmol) was dissolved in methanol (10 mL), then NaBH₄ (430 mg, 11.3 mmol) was added at 0 °C, and the mixture was stirred at room temperature for 3 h. The reaction solution was concentrated and extracted with EA. The organic phase was dried and concentrated to obtain **INT-3-1** as a solid (700 mg, yield: 87%). LCMS (ESI): *m*/*z* = 286.0 [M+H]⁺.

### Step 2: Synthesis of 8-bromo-4-chloro-3-(2-chloroethoxy)-5,6,7,8-tetrahydronaphthalene-2-carbonitrile

**INT-3-1** (700 mg, 2.4 mmol) was dissolved in DCM (10 mL), then PBr₃ (1.3 g, 4.8 mmol) was added at 0 °C, and the mixture was stirred at room temperature for 5 h. The reaction was extracted with DCM, and the organic phase was dried and concentrated. The residue was purified by flash chromatography to obtain **INT-3** as a solid (500 mg, yield: 59%). LCMS (ESI): *m*/*z* =347.9[M+H]⁺; ¹H NMR (400 MHz, CDCl₃) δ 7.55 (s, 1H), 5.45 (t, *J =* 3.7 Hz, 1H), 4.42 (td, *J* = 6.1, 1.7 Hz, 2H), 3.88 (t, *J =* 6.1 Hz, 2H), 3.18-3.05 (m, 1H), 2.82-2.65 (m, 1H), 2.43-2.32 (m, 1H), 2.29-2.18 (m, 1H), 2.12-1.96 (m, 2H).

### Intermediate INT-4

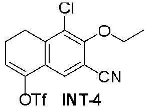

Synthesis was performed using methods described in **INT-2.** ¹H NMR (400 MHz, CD₃OD) δ 7.47 (s, 1H), 6.27 (t, J = 4.8 Hz, 1H), 4.30 (q, J = 7.2 Hz, 2H), 3.09 (t, J = 8.4 Hz, 2H), 2.61 (td, J = 8.4 Hz, 4.8 Hz, 2H), 1.48 (t, J = 7.2 Hz, 3H).

### Intermediate INT-5

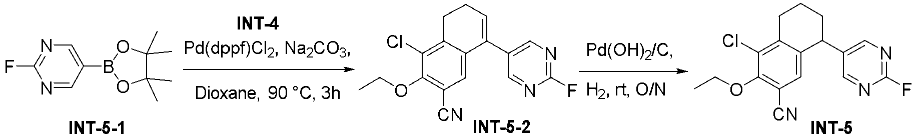

### Step 1: Synthesis of 4-chloro-3-ethoxy-8-(2-fluoropyrimidin-5-yl)-5,6-dihydronaphthalene-2-carbonitrile

**INT-5-1** (500 mg) was dissolved in 16 mL of 1,4-dioxane and 1.6 mL of water, then **INT-4** (500 mg, 1.20 mmol), Pd(dppf)Cl₂ (88.2 mg, 0.12 mmol) and Na₂CO₃ (383 mg, 3.61 mmol) were added and reacted for 3 h at 90 °C under N₂ protection. The reaction was cooled to room temperature, then the organic phase was filtered, poured into saturated aqueous NH₄Cl and extracted with EA. The organic phase was washed with saturated brine, dried with Na₂SO₄, and concentrated under reduced pressure. The residue was purified by flash chromatography (PE: EA=2:1) to obtain compound **INT-5-2** (337 mg, white solid), yield: 85%. LCMS (ESI): *m*/*z* =330.1 [M+H]⁺.

### Step 2: Synthesis of 4-chloro-3-ethoxy-8-(2-fluoropyrimidin-5-yl)-5,6,7,8-tetrahydronaphthalene-2-carbonitrile

**INT-5-2** (330 mg, 1.0 mmol) was dissolved in 33 mL of methanol and 15 mL of EA, then Pd(OH)₂/C (33 mg) was added. The reaction was stirred under H₂ overnight at room temperature, then filtered and concentrated to obtain the crude **INT-5** (white oil), yield: 100%. LCMS (ESI): *m*/*z* =330.0 [M-H]⁻.

### Intermediate INT-6

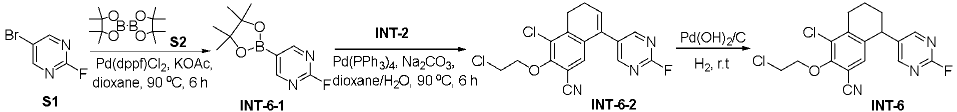

### Step 1: Synthesis of 2-fluoro-5-(4,4,5,5-tetramethyl-1,3,2-dioxaborolan-2-yl)pyrimidine

To a dry flask was added **S1** (1 g, 5.7 mmol), **S2** (2 g, 7.8 mmol), Pd(dppf)Cl₂ (300 mg, 0.4 mmol) and KOAc (1.5 g, 15.3 mmol) and dioxane (30 mL) sequentially, and the reaction was stirred at 90 °C under N₂ protection for 6 hours. The reaction was cooled to room temperature, concentrated under reduced pressure to obtain the crude product **INT-6-1,** which was directly used into next step. LCMS (ESI): *m*/*z* =225.2 [M+H]⁺.

### Step 2: Synthesis of 4-chloro-3-(2-chloroethoxy)-8-(2-fluoropyrimidin-5-yl)-5,6-dihydronaphthalene-2-carbonitrile

**INT-6-1** (800 mg, 3.6 mmol), **INT-2** (1 g, 2.4 mmol), Pd(PPh₃)₄ (138 mg, 0.12 mmol), and Na₂CO₃ (760 mg, 7.2 mmol) were dissolved in dioxane/water (30/6 mL) in a dry flask and stirred for 6 h at 90 °C under N₂ protection. The reaction was cooled to room temperature, then poured into saturated brine and extracted with EA. The organic phase was collected and concentrated under reduced pressure, and the residue was purified by flash chromatography to obtain **INT-6-2** (400 mg, yield: 46%) as a yellow oil. LCMS (ESI): *m*/*z* =364.1 [M+H]⁺.

### Step 3: Synthesis of 4-chloro-3-(2-chloroethoxy)-8-(2-fluoropyrimidin-5-yl)-5,6,7,8-tetrahydronaphthalene-2-carbonitrile

**INT-6-2** (mg, 1.0 mmol) was dissolved in 33 mL of methanol and 15 mL of EA, then Pd(OH)₂/C (33 mg) was added and the reaction was stired under H₂ overnight at room temperature. The reaction was filtered and concentrated to obtain the crude **INT-6** (yellow oil), yield: 100%. LCMS (ESI): *m*/*z* =364.0 [M-H]⁻; ¹H NMR (400 MHz, DMSO-*d*₆) δ 8.56 (s, 2H), 7.36 (s, 1H), 4.39 (t, *J =* 6.4 Hz, 2H), 3.97 (t, *J=* 6.4 Hz, 2H), 2.87 (brs, 1H), 2.07-1.99 (m, 1H), 1.86-1.79 (m, 3H).

### Example 1

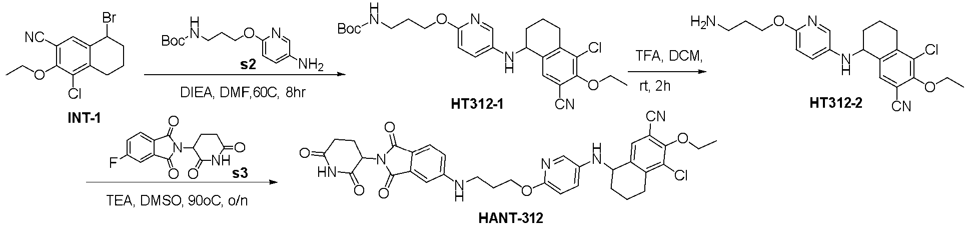

### Step 1: Synthesis of tert-butyl (3-((5-(5-chloro-7-cyano-6-ethoxy-1,2,3,4-tetrahydronaphthalen-1-yl)amino)pyridin-2-yl)oxy)propyl)carbamate

**INT-1** (100 mg, 0.32 mmol), **S2** (128 mg, 0.47 mmol), DIEA (205 mg, 1.60 mmol) were dissolved in DMF (10 mL) and the reaction was stirred under N₂ protection at 60 °C for 8 h. The reaction was cooled to room temperature, quenched with NH₄Cl and extracted with EA. The organic phases were combined and dried over anhydrous Na₂SO₄, filtered, and concentrated under reduced pressure. The residue was purified by flash chromatography to obtain compound **HT312-1** (150 mg, yellow oil, yield: 94%). LCMS (ESI): *m*/*z* =501.2 [M+H]⁺.

### Step 2: Synthesis of 8-(6-(3-aminopropoxy)pyridin-3-yl)amino)-4-chloro-3-ethoxy-5,6,7,8-tetrahydronaphthalene-2-carbonitrile

**HT312-1** (150 mg, 0.30 mmol) was dissolved in DCM (5 mL), then TFA (0.5 mL) was added, and the reaction was stirred for 2 h at room temperature. The reaction was quenched with NH₄Cl, extracted by EA, and the organic phase was dried with anhydrous Na₂SO₄, filtered and concentrated under reduced pressure. The residue was purified by flash chromatography to obtain compound **HT312-2** (100 mg, yellow oily, 83% yield). LCMS (ESI): *m*/*z* =401.2 [M+H]⁺.

### Step 3: Synthesis of 4-chloro-8-(6-(3-((2-(2,6-dioxopiperidin-3-yl)-1,3-dioxoisoindolin-5-yl)amino)propoxy)pyridin-3-ylamino)-3-ethoxy-5,6,7,8-tetrahydronaphthalene-2-carbonitrile

**HT312-2** (100 mg, 0.25 mmol), **S3** (173 mg, 0.63 mmol) were dissolved in DMSO (10 mL), then TEA (125 mg, 1.25 mmol) was added and the reaction was stirred at 90 °C overnight. The reaction was poured into saturated aq. NH₄Cl and extracted with EA. The organic phase was dried with anhydrous Na₂SO₄, concentrated under reduced pressure, and the residue was purified by flash chromatography to obtain compound **HANT-312** (16.3 mg, white solid, yield: 4%). LCMS (ESI): *m*/*z* =657.2 [M+H]⁺; ¹H NMR (400 MHz, DMSO-*d₆*) δ 11.04 (s, 1H), 7.71 (s, 1H), 7.61 (d, *J* = 3.0 Hz, 1H), 7.56 (d, *J =* 8.4 Hz, 1H), 7.15-7.20 (m, 2H), 6.98-6.99 (m, 1H), 6.87 (dd, *J =* 8.5, 2.1 Hz, 1H), 6.66 (d, *J =* 8.8 Hz, 1H), 5.62 (d, *J =* 9.0 Hz, 1H), 5.03 (dd, *J =* 12.9, 5.4 Hz, 1H), 4.61-4.50 (m, 1H), 4.25-4.20 (m, 2H), 4.17 (t, *J =* 7.0 Hz, 2H), 3.30-3.31 (m, 1H), 2.94-2.79 (m, 2H), 2.76-2.66 (m, 1H), 2.63-2.52 (m, 2H), 2.11-1.89 (m, 4H), 1.88-1.71 (m, 4H), 1.39 (t, *J* = 7.0 Hz, 3H).

### Example 2

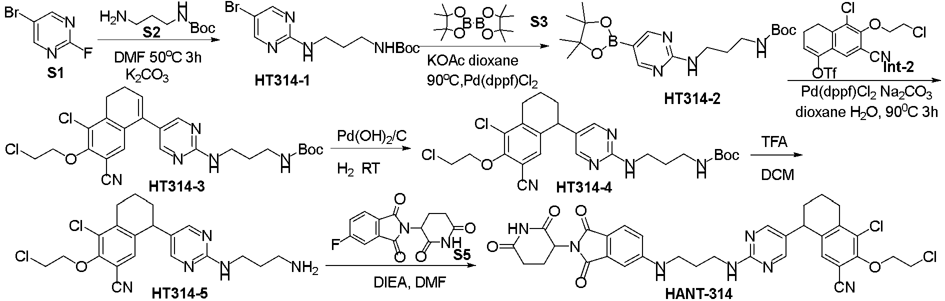

### Step 1: Synthesis of tert-butyl (3-((5-bromopyrimidin-2-yl)amino)propyl)carbamate

To a dry flask was added **S1** (1 g, 5.65 mmol), S2 (1.08 g, 6.22 mmol), K₂CO₃ (2.34 g, 16.9 mmol) and DMF (20 mL) sequentially and the reaction was stirred at 50 °C for 3 hours. The reaction was cooled to room temperature, poured into EA and washed with saturated brine. The organic phase was collected and concentrated under reduced pressure, and the residue was purified by flash chromatography to obtain **HT314-1** (1.72 g, yield: 93%) as a white solid. LCMS: *m*/*z* =331.2 [M+H]⁺.

### Step 2: Synthesis of tert-butyl (3-(5-(4,4,5,5-tetramethyl-1,3,2-dioxobenzaldehyde-2-yl)pyrimidin-2-yl)amino)propyl)carbamate

To a dry flask was added **HT314-1** (1.7 g, 5.15 mmol), **S3** (1.96 g, 7.72 mmol) and Pd(dppf)Cl₂ (1.13 g, 1.54 mmol) KOAc (1.52 g, 15.5 mmol), and dioxane (15 mL) sequentially, and the reaction was stirred overnight at 90 °C under N₂ protection. The reaction was cooled to room temperature, filtered and concentrated. The residue was poured into EA, and the organic phase was washed with brine and concentrated under reduced pressure. The residue was purified by flash chromatography to obtain a white solid **HT314-2** (1.56 g, yield: 80%). LCMS: m/z =379.2 [M+H]⁺.

### Step 3: Synthesis of tert-butyl (3-(5-(5-chloro-6-(2-chloroethoxy)-7-cyano-3,4-dihydronaphthalen-1-yl)pyrimidin-2-yl)amino)propyl)carbamate

To a dry flask was added **HT314-2** (272 mg, 0.72 mmol), **INT-2** (200 mg, 0.48 mmol), Pd(dppf)Cl₂ (71 mg, 0.097 mmol), and Na₂CO₃ (153 mg, 1.4 mmol) and dioxane/H₂O (10/2 mL) sequentially, and the reaction was stirred for 3 h at 90 °C under N₂ protection. The reaction was cooled to room temperature, filtered, then poured into saturated brine and extracted with EA. The organic phase was collected and concentrated under reduced pressure, and the residue was purified by flash chromatography to obtain the yellow oil **HT314-3** (220 mg, yield: 80%). LCMS: *m*/*z* =520.0 [M+H]⁺.

### Step 4: Synthesis of tert-butyl (3-(5-(5-chloro-6-(2-chloroethoxy)-7-cyano-1,2,3,4-tetrahydronaphthalen-1-yl)pyrimidin-2-yl)amino)propyl)carbamate

To a dry flask was added **HT314-3** (220 mg, 0.42 mmol) dissolved in methanol (10 mL) followed by Pd(OH)₂/C (22 mg, 10% wt). The reaction was stirred under H₂ at room temperature for 24 h. The reaction was filtered and concentrated under reduced pressure, and the residue was purified by flash chromatography to obtain **HT314-4** (160 mg, yield: 72%) as a colorless oil. LCMS: *m*/*z* =522.0 [M+H]⁺.

### Step 5: Synthesis of 8-(2-(3-aminopropyl)amino)pyrimidin-5-yl)-4-chloro-3-(2-chloroethoxy)-5,6,7,8-tetrahydronaphthalene-2-carbonitrile

To a dry flask was added **HT314-4** (160 mg, 0.308 mmol), DCM (5 mL) and TFA (1 mL), and stirred at room temperature for 1 hour. The reaction was filtered and concentrate under reduced pressure. To the residue was added EA and water, and pH was adjusted to 8-10 with ammonia. The mixture was extracted with EA and concentrated under reduced pressure to obtain **HT314-5** as a yellow oil (85 mg, yield: 65.8%). LCMS: *m*/*z* =420.3 [M+H]⁺.

### Step 6: Synthesis of 4-chloro-3-(2-chloroethoxy)-8-(2-(3-(2-(2-(2,6-dioxopiperidin-3-yl)-1,3-dioxoisoindolin-5-yl)amino)propyl)amino)pyrimidin-5-yl)-5,6,7,8-tetrahydronaphthalene-2-carbonitrile

To a dry flask was added **HT314-5** (85 mg, 0.202 mmol), DMF (1 mL), S5 (140 mg, 0.507 mmol), and DIEA (130 mg, 1.01 mmol) sequentially, and the reaction was stirred at 100 °C overnight. The reaction was cooled to room temperature, poured into EA, and washed with saturated brine. The organic phase was collected and concentrated under reduced pressure, and then purified by reversed-phase prep-HPLC to obtain **HANT-314** (15.4 mg, yield: 11.3%) as a yellow solid. LCMS: *m*/*z* =676.1 [M+H]⁺; ¹H NMR (400 MHz, DMSO-*d₆*) δ 11.04 (s, 1H), 8.00 (s, 2H), 7.55 (d, *J* = 8.4 Hz, 1H), 7.29 (s, 1H), 7.19 (t, *J =* 5.6 Hz, 1H), 7.11 (t, *J* = 5.3 Hz, 1H), 6.95 (s, 1H), 6.84 (dd, *J* = 8.4, 1.9 Hz, 1H), 5.02 (dd, *J =* 12.9, 5.4 Hz, 1H), 4.47-4.31 (m, 2H), 3.99 (dt, *J =* 19.4, 5.0 Hz, 3H), 3.41-3.32 (m, 2H), 3.23 (dd, *J =* 12.5, 6.5 Hz, 2H), 2.95-2.75 (m, 3H), 2.62-2.51 (m, 2H), 2.02-1.91 (m, 2H), 1.87-1.70 (m, 5H).

### Example 3

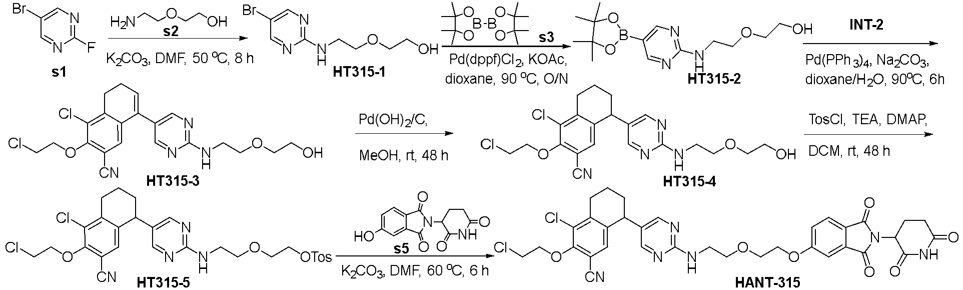

### Step 1: Synthesis of 2-(2-((5-bromopyrimidin-2-yl)amino)ethoxy)ethan-1-ol

**S1** (500 mg, 2.8 mmol), **S2** (330 mg, 3.1 mmol), DMF (15 mL), K₂CO₃ (1.2 g, 8.5 mmol) were added sequentially into a dry flask and reacted at 50 °C for 8 hours. The reaction was cooled to room temperature, then poured into EA, and washed with saturated brine. The organic phase was collected and concentrated under reduced pressure, and the residue was purified by flash chromatography to obtain the colorless oily **HT315-1** (650 mg, yield: 89%). LCMS (ESI): *m*/*z* =262.0 [M+H]⁺.

### Step 2: Synthesis of 2-(2-((5-(4,4,5,5-tetramethyl-1,3,2-dioxaborolan-2-yl)pyrimidin-2-yl)amino)ethoxy)ethan-1-ol

To a dry flask was added **HT315-1** (1.4 g, 5.4 mmol), **S3** (2 g, 7.8 mmol), Pd(dppf)Cl₂ (300 mg, 0.4 mmol), KOAc (1.5 g, 15.3 mmol), and dioxane (50 mL) sequentially, and stirred at 90 °C under N₂ protection overnight. The reaction was cooled to room temperature, concentrated under reduced pressure to obtain the crude **HT315-2,** which was directly used in next step. LCMS (ESI): *m*/*z* =310.2 [M+H]⁺.

### Step 3: Synthesis of 4-chloro-3-(2-chloroethoxy)-8-(2-((2-(2-hydroxyethoxyethyl)amino)pyrimidin-5-yl)-5,6-dihydronaphthalene-2-carbonitrile

To a dry flask was added **HT315-2** (220 mg, 0.72 mmol), **INT-2** (200 mg, 0.48 mmol), Pd(PPh₃)₄ (110 mg, 0.09 mmol), Na₂CO₃ (150 mg, 1.4 mmol) and dioxane/water (15/3 mL) sequentially, and stirred at 90 °C for 6 h under N₂ protection. The reaction was cooled to room temperature, poured into saturated brine and extracted with EA. The organic phase was collected and concentrated under reduced pressure, and the residue was purified by flash chromatography to obtain **HT315-3** (200 mg, yield: 95%) as a yellow oil. LCMS (ESI): *m*/*z* =449.1 [M+H]⁺.

### Step 4: Synthesis of 4-chloro-3-(2-chloroethoxy)-8-(2-((2-(2-hydroxyethoxyethyl)amino)pyrimidin-5-yl)-5,6,7,8-tetrahydronaphthalene-2-carbonitrile

To a dry flask was added **HT315-3** (200 mg, 0.45 mmol), methanol (10 mL) and Pd(OH)₂/C (20 mg, 10% wt). The reaction was stirred under H₂ at room temperature for 48 h. The reaction was filtered and concentrated under reduced pressure to obtain a white solid **HT315-4** (200 mg, yield: 100%), which was used directly in next step. LCMS (ESI): *m*/*z* =451.1 [M+H]⁺.

### Step 5: Synthesis of 2-(2-((5-(5-chloro-6-(2-chloroethoxy)-7-cyano-1,2,3,4-tetrahydronaphthalen-1-yl)pyrimidin-2-yl)amino)ethoxyethyl ester of 4-methylbenzenesulfonate

To a dry flask were sequentially added **HT315-4** (110 mg, 0.24 mmol), p-toluenesulfonyl chloride (93 mg, 0.49 mmol), DCM (8 mL), TEA (73 mg, 0.72 mmol) and DMAP (6 mg, 0.05 mmol). The reaction was stirred at room temperature for 48 hours then poured into saturated brine and extracted with EA. The organic phase was collected, dried and concentrated under reduced pressure. The residue was purified by preparative TLC to obtain the colorless oil **HT315-5** (60 mg, yield: 43%). LCMS (ESI): *m*/*z* =605.1 [M+H]⁺.

### Step 6: Synthesis of 4-chloro-3-(2-chloroethoxy)-8-(2-(2-(2-(2,6-dioxopiperidin-3-yl)-1,3-dioxoisoindolin-5-yl)oxy)ethoxy-amino)pyrimidin-5-yl-5,6,7,8-tetrahydronaphthalene-2-carbonitrile

To a dry flask was added **HT315-5** (60 mg, 0.1 mmol), S5 (40 mg, 0.15 mmol), DMF (5 mL), and K₂CO₃ (28 mg, 0.2 mmol). The reaction was stirred at 60 °C for 6 h then cooled to room temperature. The reaction solution was poured into EA, washed with saturated brine, and the organic phase was collected, dried and concentrated under reduced pressure. The residue was purified by neutral prep-HPLC to obtain **HANT-315** (9.5 mg, yield: 13.5%) as a white solid. LCMS (ESI): *m*/*z* =707.0 [M+H]⁺; ¹H NMR (400 MHz, DMSO-*d₆*) δ 11.10 (s, 1H), 8.00 (s, 2H), 7.82 (d, *J =* 8.3 Hz, 1H), 7.45 (d, *J =* 2.3 Hz, 1H), 7.36 (dd, *J =* 8.3, 2.3 Hz, 1H), 7.29 (s, 1H), 7.04 (t, *J =* 5.8 Hz, 1H), 5.11 (dd, *J =* 12.9, 5.4 Hz, 1H), 4.43-4.35 (m, 2H), 4.34-4.28 (m, 2H), 4.02 (t, *J* =5.6 Hz, 1H), 3.99-3.93 (m, 2H), 3.79 (dd, *J* = 5.6, 3.4 Hz, 2H), 3.59 (t, *J =* 6.1 Hz, 2H), 3.44 (q, *J =* 6.0 Hz, 2H), 2.86 (dd, *J =* 11.1, 7.0 Hz, 3H), 2.64-2.53 (m, 2H), 2.09-1.90 (m, 2H), 1.83-1.62 (m, 3H).

### Example 4, 5

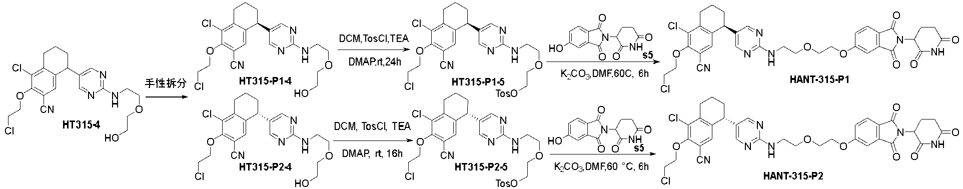

### Step 1: Preparation of HT315-P1-4 and HT315-P2-4

The brown oil HT315-4 (1.3 g, crude) was subjected to chiral separation (SFC purification method (Column: Chiralpak IJ 250mm*4.6mm 5um, Hex-EtOH-50-50-15MIN), 30 °C) to obtain a pair of enantiomers **HT315-P1-4** (RT₁=6.105 min, 600 mg) and **HT315-P2-4** (RT₂=8.309 min, 550 mg). LCMS: m/z =451.1 [M+H]⁺.

### Step 2: Preparation of HT315-P1-5 and HT315-P2-5

**HT315-P1-4** (600 mg, 1.33 mmol), DCM (50 mL), TosCl (512 mg, 2.69 mmol), TEA (408 mg, 4.04 mmol), and DMAP (33 mg, 0.27 mmol) were added to a dry flask and stirred at room temperature for 24 hours.The reaction solution was poured into saturated brine, extracted with DCM. The organic phase was collected and concentrated under reduced pressure, and the residue was purified using reversed-phase prep-HPLC to obtain **HT315-P1-5** (530 mg, yield: 62.3%) as a light brown oil. LCMS: *m*/*z* = 605.1 [M+H]⁺.

The same synthetic protocol was applied to **HT315-P2-4** to obtain **HT315-P2-5** (270 mg, yield: 36%) as a colorless oil. LCMS: *m*/*z* =605.1[M+H]⁺.

### Step 3: Synthesis of HANT315-P1 and HANT315-P2

In a dry flask, **HT315-P1-5** (530 mg, 0.877 mmol), DMF (30 mL), S5 (340 mg, 1.24 mmol), K₂CO₃ (230 mg, 1.67 mmol) were added sequentially and the reaction was stirred at 60 °C for 6 h. The reaction was cooled to room temperature, poured into EA and washed with saturated brine. The organic phase was collected and concentrated under reduced pressure, and the residuee was purified by prep-HPLC to afford a white solid **HANT-315-P1** (163.8 mg, yield: 28.0%). LCMS: *m*/*z* = 707.0 [M+H]⁺; ¹H NMR (400 MHz, DMSO-*d₆*) δ 11.10 (s, 1H), 8.00 (s, 2H), 7.82 (d, *J =* 8.3 Hz, 1H), 7.45 (d, *J =* 1.9 Hz, 1H), 7.36 (dd, *J =* 8.3, 2.1 Hz, 1H), 7.29 (s, 1H), 7.04 (t, *J =* 5.6 Hz, 1H), 5.11 (dd, *J =* 12.9, 5.3 Hz, 1H), 4.44-4.35 (m, 2H), 4.35-4.25 (m, 2H), 4.02 (t, *J* = 5.6 Hz, 1H), 3.99-3.91 (m, 2H), 3.86-3.71 (m, 2H), 3.60 (t, *J =* 6.0 Hz, 2H), 3.44 (dd, *J =* 11.6, 5.8 Hz, 2H), 2.96-2.74 (m, 3H), 2.64-2.52 (m, 2H), 2.10-2.00 (m, 1H), 1.99-1.89 (m, 1H), 1.85-1.67 (m, 3H).

To a dry flask was sequentially added **HT315-P2-5** (250 mg, 0.40 mmol), DMF (10 mL), **S1** (170 mg, 0.60 mmol) and K₂CO₃ (110 mg, 0.80 mmol), and stirred at 60 °C for 6 h. The reaction was cooled to room temperature, diluted with water, and extracted by EA. The organic phase was washed with saturated brine, dried with anhydrous Na₂SO₄, filtered and concentrated under reduced pressure. The residue was purified by prep-HPLC (formic acid) to afford **HANT-315-P2** (57.5 mg, yield: 19%) as a white solid. LCMS: *m*/*z =* 707.0 [M+H]⁺; ¹H NMR (400 MHz, DMSO-*d₆*) δ 11.10 (s, 1H), 8.01 (s, 2H), 7.82 (d, *J =* 8.3 Hz, 1H), 7.45 (d, *J* = 2.1 Hz, 1H), 7.36 (dd, *J =* 8.3, 2.2 Hz, 1H), 7.29 (s, 1H), 7.04 (t, *J =* 5.7 Hz, 1H), 5.11 (dd, *J =* 12.9, 5.4 Hz, 1H), 4.43-4.24 (m, 4H), 4.02 (t, *J =* 5.6 Hz, 1H), 3.99-3.93 (m, 2H), 3.84-3.74 (m, 2H), 3.60 (t, *J =* 6.1 Hz, 2H), 3.44 (dd, *J =* 11.9, 6.0 Hz, 2H), 2.97-2.75 (m, 3H), 2.64-2.52 (m, 2H), 2.11-2.00 (m, 1H), 1.99-1.89 (m, 1H), 1.85-1.67 (m, 3H).

### Example 6

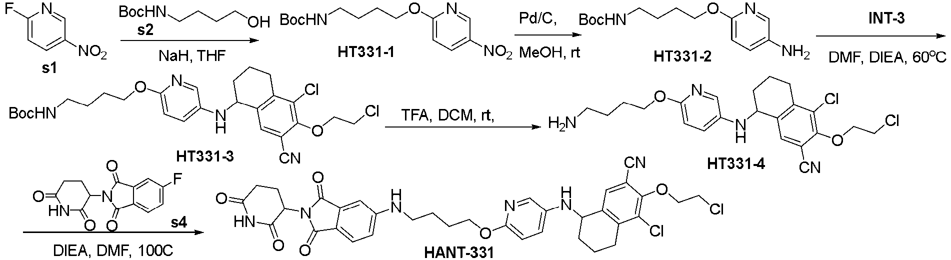

### Step 1: Synthesis of tert-butyl (4-((5-nitropyridin-2-yl)oxy)butyl)carbamate

In a dry flask was added **S1** (500 mg, 3.52 mmol) in THF (5 mL), then NaH (215 mg, 5.4 mmol, 60%) was added at 0 °C and stirred for 0.5 h. S2 (732 mg, 3.87 mmol) was added in and stirred at room temperature overnight. The reaction was quenched by adding water at 0 °C, then poured into the EA and washed with saturated brine. The organic phase was collected and concentrated under reduced pressure. The residue was purified by flash chromatography to obtain **HT331-1** (980 mg, yield: 89.5%) as a white solid. LCMS: *m*/*z* = 312.3 [M+H]⁺.

### Step 2: Synthesis of tert-butyl (4-((5-aminopyridin-2-yl)oxy)amino)formate

To a dry flask was added **HT331-1** (170 mg, 0.55 mmol), methanol (10 mL) and Pd/C (17 mg, 10% wt). The reaction was carried out under H₂ overnight at room temperature. The reaction was filtered, collected and concentrated under reduced pressure, and the residue was purified by flash chromatography to obtain **HT331-2** (138 mg, yield: 90%) as a yellow oil. LCMS : *m*/*z* = 282.4 [M+H]⁺.

### Step 3: Synthesis of tert-butyl (4-((5-((5-chloro-6-(2-chloroethoxy)-7-cyano-1,2,3,4-tetrahydronaphthalen-1- yl)amino)pyridin-2- yl)oxy)butyl)carbamate

To a dry flask was sequentially added **HT331-2** (136 mg, 0.483 mmol), **INT-3** (112 mg, 0.323 mmol), DIEA (208 mg, 1.61 mmol) and DMF (5 mL) and stirred at 60 °C under N₂ protection overnight. The reaction was cooled to room temperature then poured into saturated brine, and extracted with EA. The organic phase was collected and concentrated under reduced pressure, and the residue was purified by flash chromatography to obtain **HT331-3** (160 mg, yield: 90%) as a yellow oil. LCMS: m/z =549.4[M+H]⁺.

### Step 4: Synthesis of 8-((6-(4-aminobutoxy)pyridin-3-yl)amino)-4-chloro-3-(2-chloroethoxy)-5,6,7,8-tetrahydronaphthalene-2-carbonitrile

To a dry flask was added **HT331-3** (160 mg, 0.29 mmol), DCM (5 mL) and TFA (1 mL), and stirred at room temperature for 2 h. The reaction was filtered and concentrated under reduced pressure, then EA was added and pH was adjusted to 8-10 with ammonia. The crude mixture was extracted, and the organic phase was collected and concentrated under reduced pressure to obtain the yellow oily substance **HT331-4** (130 mg, crude product, yield: 99%) which was used directly in next step. LCMS : *m*/*z* =449.2 [M+H]⁺.

### Step 5: Synthesis of 4-chloro-3-(2-chloroethoxy)-8-((6-(4-(2-(2,6-dioxopiperidin-3-yl)-1,3-dioxoisoindolin-5-yl)amino)butoxy)pyridin-3-amino)-5,6,7,8-tetrahydronaphthalene-2-carbonitrile

To a dry flask was added **HT331-4** (76 mg, 0.17 mmol), DMF (1 mL), S4 (117 mg, 0.424 mmol) and DIEA (109 mg, 0.845 mmol), and the reaction was stirred at 100 °C for 8 h. The reaction was cooled to room temperature, then the EA was poured in, and the mixture was washed with saturated brine. The organic phase was collected and concentrated under reduced pressure, and the residue was purified by flash chromatography to obtain a yellow solid **HANT-331** (9.0 mg, yield: 7.5%). LCMS: *m*/*z* = 705.0 [M+H]⁺; ¹H NMR (300 MHz, DMSO-*d₆*) δ 11.05 (s, 1H), 7.73 (s, 1H), 7.60 (d, *J =* 2.8 Hz, 1H), 7.56 (d, *J =* 8.4 Hz, 1H), 7.18-7.12 (m, 2H), 6.96 (d, *J =* 1.7 Hz, 1H), 6.85 (dd, *J =* 8.4, 1.9 Hz, 1H), 6.63 (d, *J =* 8.8 Hz, 1H), 5.62 (d, *J =* 9.1 Hz, 1H), 5.03 (dd, *J =* 12.7, 5.4 Hz, 1H), 4.60-4.53 (m, 1H), 4.39 (t, *J =* 12 Hz, 2H) 4.17 (t, *J =* 6.3 Hz, 2H), 3.98, (t, *J* = 9 Hz, 2H), 3.26-3.19 (m, 2H), 2.94-2.79 (m, 2H), 2.78-2.66 (m, 1H), 2.60-2.53 (m, 1H), 2.02-1.96 (m, 1H), 1.95-1.85 (m, 1H), 1.85-1.72 (m, 6H), 1.72-1.65 (m, 2H).

### Example 7

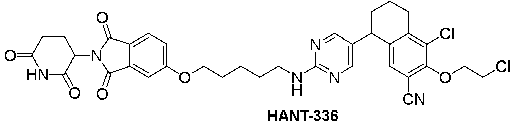

**HANT-336** was synthesized using procedures reported for **HANT-315** in Example 3, where starting material S2 in Example 3 was replaced with with 5-aminopentan-1-ol. The final product was purified by reversed-phase prep-HPLC to afford **HANT-336** (11.5 mg, yield: 12.3%) as a white solid. LCMS: *m*/*z* = 706.0 [M+H]⁺; ¹H NMR (400 MHz, DMSO-*d₆*) δ 11.10 (s, 1H), 7.98 (s, 2H), 7.82 (d, *J* = 8.3 Hz, 1H), 7.42 (d, *J* = 2.1 Hz, 1H), 7.34 (dd, *J =* 8.3, 2.2 Hz, 1H), 7.29 (s, 1H), 7.12 (t, *J =* 5.7 Hz, 1H), 5.11 (dd, *J =* 12.9, 5.3 Hz, 1H), 4.41-4.36 (m, 2H), 4.17 (t, *J =* 6.4 Hz, 2H), 4.01 (t, *J =* 5.7 Hz, 1H), 3.98-3.94 (m, 2H), 3.26 (d, *J =* 6.2 Hz, 2H), 2.93-2.79 (m, 3H), 2.62-2.53 (m, 1H), 2.09-1.88 (m, 3H), 1.81-1.73 (m, 5H), 1.61-1.54 (m, 2H), 1.50-1.42 (m, 2H).

### Example 8

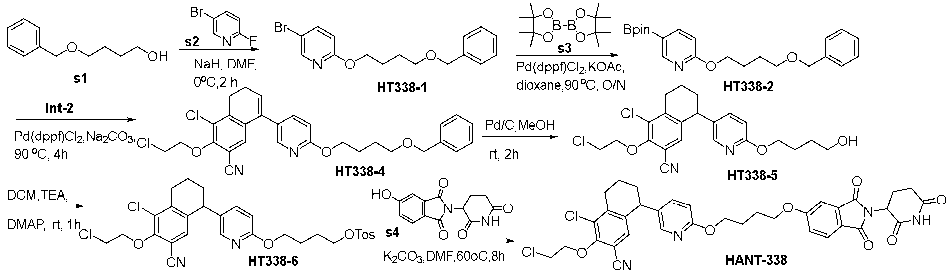

### Step 1: Synthesis of 2-(4-benzyloxy)butoxy)-5-bromopyridine

**S1** (6.0 g, 36.1 mmol) was dissolved in DMF (100 mL), then NaH (2.77 g, 72 mmol) was added at 0 °C and stirred for 0.5 h. S2 (7.5 mg, 43.3 mmol) was added and the reaction was stirred at room temperature for 2 h. The reaction solution was extracted with EA, and the organic phase was collected, dried and concentrated under reduced pressure. The residue was purified by a flash chromatography to obtain the white solid **HT338-1** (8.0 g, yield: 66.7 %). LCMS (ESI): *m*/*z* =336.1[M+H]⁺.

### Step 2: Synthesis of 2-(4-(benzyloxy)butoxy)-5-(4,4,5,5-tetramethyl-1,3,2-dioxaborolan-2-yl)pyridine

**HT338-1** (500 mg, 1.5 mmol), S3 (455 mg, 1.8 mmol), Pd(dppf)Cl₂ (328 mg, 0.45 mmol), KOAc (439 mg, 4.5 mmol) was dissolved into 1,4-dioxane (10 mL) and stirred overnight at 90 °C under N₂ protection. The reaction was cooled to room temperature and extracted with EA. The organic phase was dried and concentrated, and the residue was purified by flash chromatography to obtain the crude **HT338-2** (600 mg); LCMS (ESI): *m*/*z* =383.2[M+H]⁺.

### Step 3: Synthesis of 8-(6-(4-(benzyloxy)butoxy)pyridin-3-yl)-4-chloro-3-(2-chloroethoxy)- 5,6-dihydronaphthalene-2-carbonitrile

**HT338-3** (600 mg, 1.57 mmol), **INT-2** (782 mg, 1.88 mmol), Pd(dppf)Cl₂ (229 mg, 0.31 mmol), and Na₂CO₃ (332 mg, 3.1 mmol) were dissolved in 1,4-dioxane/water (10 mL/2 mL) and stirred at 90 °C for 4 h, then cooled to room temperature. The reaction solution was extracted with EA, and the organic phase was dried and concentrated. The residue was purified by flash chromatography to obtain **HT338-4** (550 mg, yield: 67%) as a solid; LCMS (ESI): *m*/*z* =523.1[M+H]⁺.

### Step 4: Synthesis of 4-chloro-3-(2-chloroethoxy)-8-(6-(4-hydroxybutoxy)pyridin-3yl)-5,6,7,8-tetrahydronaphthalene-2-carbonitrile

**HT338-4** (550 mg, 1.05 mmol), Pd/C (300 mg) was dissolved in MeOH (5 mL) and stirred at room temperature for 2 h. The reaction solution was filtered and the organic phase was concentrated to obtain **HT338-5** (200 mg, yield: 44%) as a solid; LCMS (ESI): *m*/*z =435.1* [M+H]⁺.

### Step 5: Synthesis of 4-methylbenzenesulfonic acid 4-((5-(5-chloro-6-(2-chloroethoxy)-7-cyano-1,2,3,4-tetrahydronaphthalen-1-yl)pyridin-2-yl)oxy)butyl ester

**HT338-5** (200 mg, 0.46 mmol), TosCl (130 mg, 0.69 mmol), TEA (70 mg, 0.69 mmol), DMAP (11 mg, 0.09 mmol) was dissolved in DCM (5 mL) and stirred at room temperature for 1 h. The reaction solution was extracted with DCM, the organic phase was dried and concentrated, and the residue was purified by silica gel column. The solid HT338-6 (120 mg, yield: 44%) was obtained; LCMS (ESI): *m*/*z* =589.1[M+H]⁺.

### Step 6: Synthesis of 4-chloro-3-(2-chloroethoxy)-8-(6-(4-(4-((2-(2,6-dioxopiperidin-3-yl)-1,3-dioxoisoindolin-5-yl)oxy)butoxy)pyridin-3-yl)-5,6,7,8-tetrahydronaphthalene-2-carbonitrile

**HT338-6** (120 mg, 0.2 mmol), S4 (84 mg, 0.31 mmol), K₂CO₃ (84 mg, 0.6 mmol) were dissolved in DMF (5 mL) and stirred at 60 °C for 8 h, then cooled to room temperature. The reaction solution was extracted with EA, and the organic phase was dried and concentrated. The residue was purified by prep-HPLC to obtain solid **HANT-338** (120 mg, yield: 44%); LCMS (ESI): *m*/*z* =691.2 [M+H]⁺; ¹H NMR (400 MHz, DMSO-*d₆*) δ 11.10 (s, 1H), 7.89 (d, *J =* 2.5 Hz, 1H), 7.83 (d, *J =* 8.3 Hz, 1H), 7.43 (d, *J* = 2.3 Hz, 1H), 7.37 (ddd, *J =* 15.5, 8.5, 2.4 Hz, 2H), 7.19 (s, 1H), 6.75 (d, *J =* 8.5 Hz, 1H), 5.11 (dd, *J =* 12.9, 5.4 Hz, 1H), 4.43-4.37 (m, 2H), 4.34-4.13 (m, 5H), 3.97 (dd, *J =* 6.0, 4.3 Hz, 2H), 2.94-2.83 (m, 3H), 2.63-2.52 (m, 2H), 2.08-1.97 (m, 2H), 1.93-1.86 (m, 4H), 1.85-1.74 (m, 3H).

### Example 9

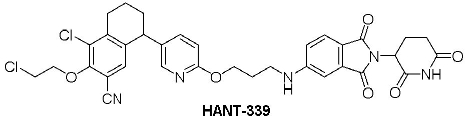

**HANT-339** was synthesized using procedures reported for **HANT-314** in Example 2, where **HT314-1** in Example 2 was replaced with (3-((5-bromopyridin-2-yl)oxy)propyl)carbamic acid tert-butyl ester (synthesis referenced to WO2021155006). The final product was purified by reversed-phase prep-HPLC to afford **HANT-339** as a yellow solid (34.9 mg, yield: 21.7 %). LCMS: *m*/*z* = 676.1 [M+H]⁺; ¹H NMR (400 MHz, DMSO-*d₆*) δ 11.04 (s, 1H), 7.90 (d, *J* = 2.3 Hz, 1H), 7.56 (d, *J =* 8.3 Hz, 1H), 7.40 (dd, *J* = 8.5, 2.3 Hz, 1H), 7.27-7.10 (m, 2H), 6.97 (s, 1H), 6.87 (dd, *J =* 8.4, 2.0 Hz, 1H), 6.79 (d, *J =* 8.6 Hz, 1H), 5.02 (dd, *J =* 12.8, 5.4 Hz, 1H), 4.43-4.37 (m, 2H), 4.34 (t, *J =* 6.2 Hz, 2H), 4.22-4.12 (m, 1H), 3.99-3.92 (m, 2H), 3.34 (d, *J =* 6.3 Hz, 1H), 2.93-2.80 (m, 3H), 2.62-2.52 (m, 2H), 2.07-1.90 (m, 5H), 1.83-1.72 (m, 3H).

### Example 10

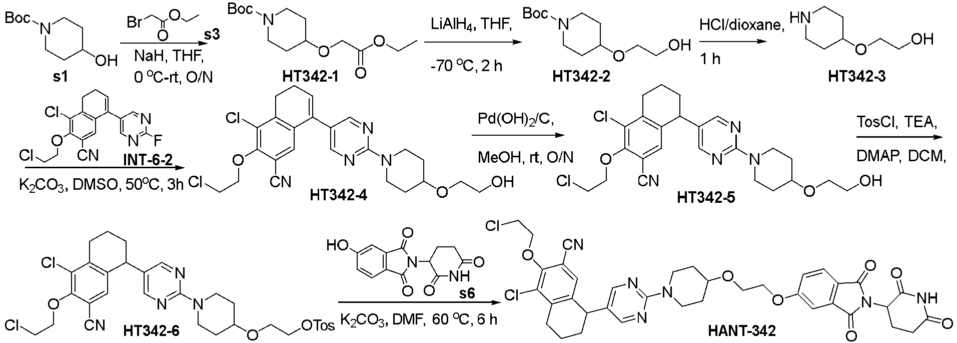

### Step 1: Synthesis of tert-butyl 4-(2-ethoxy-2-oxoethoxy)piperidine-1-carboxylate

**S1** (10 g, 49.75 mmol) was dissolved in THF (100 mL) in a dry flask, then NaH (3 g, 74.63 mmol) was slowly added under ice bath and stirred for 30 min, then S3 (10 g, 59.70 mmol) was added and the reaction was stirred at room temperature overnight. The reaction was poured into iced brine and extracted with EA. The organic phase was collected and concentrated under reduced pressure, and the residue was purified by flash chromatography to obtain **HT342-1** (7.8 g, yield: 55%) as a colorless oil. LCMS (ESI): *m*/*z* =288.2 [M+H]⁺.

### Step 2: Synthesis of tert-butyl 4-(2-hydroxyethoxy)piperidine-1-carboxylate

In a dry flask, **HT342-1** (5 g, 27.3 mmol) was dissolved in THF (100 mL), then LiAlH₄ solution (54.6 mL, 54.6 mmol, 1 M) was added slowly dropwise at -70 °C and stirred for 2 h. The reaction was quenched with methanol, then EA (200 mL) and saturated aqueous sodium potassium tartrate (20 mL) were added and stirred for 2 h. The reaction mixture was dried over anhydrous Na₂SO₄, filtered, and the filtrate was concentrated under reduced pressure to obtain **HT342-2** (4.5 g, yield: 100%) as a yellow oil. LCMS (ESI): *m*/*z* =246.2 [M+H]⁺.

### Step 3: Synthesis of 2-(piperidin-4-yloxy)ethan-1-ol

In a dry flask **HT342-2** (2 g, 18.3 mmol), dioxane (100 mL) and then HCl/dioxane (20 mL, 80 mmol, 4M) were added sequentially and the reaction was stirred for 1 h at room temperature. The reaction solution was concentrated under reduced pressure to obtain a yellow oil as the crude **HT342-3** hydrochloride, which was directly used in next step. LCMS (ESI): *m*/*z* =146.2 [M+H]⁺. **Step 4:** Synthesis of 4-chloro-3-(2-chloroethoxy)-8-(2-(4-(2-hydroxymethoxy)piperidin-1-yl)pyrimidin-5-yl)-5,6-dihydronaphthalene-2-carbonitrile

**INT-6-2** (358 mg, 0.98 mmol), **HT342-3** (120 mg, 0.82 mmol), DMSO (10 mL), K₂CO₃ (400 mg, 2.9 mmol) were added sequentially into a dry flask and reacted at 50 °C for 3 hours. The reaction was cooled to room temperature, poured into EA then washed with saturated brine. The organic phase was collected and concentrated under reduced pressure, and the residue was purified by flash chromatography to obtain **HT342-4** (230 mg, yield: 58%) as a yellow oil. LCMS (ESI): *m*/*z* =489.2 [M+H]⁺.

### Step 5: Synthesis of 4-chloro-3-(2-chloroethoxy)-8-(2-(4-(2-hydroxymethoxy)piperidin-1-yl)pyrimidin-5-yl)-5,6,7,8-tetrahydronaphthalene-2-carbonitrile

To a dry flask was added **HT342-4** (230 mg, 0.47 mmol), methanol (10 mL) and Pd(OH)₂/C (23 mg, 10% wt), and the reaction was stirred overnight under H₂ at room temperature. The reaction was filtered, and filtrate was concentrated under reduced pressure to obtain a white solid **HT342-5** (230 mg, yield: 100%), which was directly used in next step. LCMS (ESI): m/z =491.2 [M+H]⁺.

### Step 6: Synthesis of 2-((1-(5-(5-chloro-6-(2-chloroethoxy)-7-cyano-1,2,3,4-tetrahydronaphthalen-1-yl)pyrimidin-2-yl)piperidin-4-yl)oxy)ethyl-4-methylbenzenesulfonate

**HT342-5** (230 mg, 0.51 mmol) and p-toluenesulfonyl chloride (200 mg, 1.02 mmol) were dissolved in DCM (10 mL) in a dry flask, followed by addition of TEA (154 mg, 1.53 mmol) and DMAP (20 mg, 0.15 mmol). The reaction was stirred for 1 h at room temperature, then poured into saturated brine and extracted with EA. The organic phase was collected, dried and concentrated under reduced pressure. The residue was purified by preparative TLC to afford the colorless oily **HT342-6** (140 mg, yield: 43%). LCMS (ESI): *m*/*z* =645.2 [M+H]⁺.

### Step 7: Synthesis of 4-chloro-3-(2-chloroethoxy)-8-(2-(4-(2-(2-((2-(2,6-dioxopiperidin-3-yl)-1,3-dioxoisoindol-5-yl)oxy)ethoxy)piperidin-1-yl)pyrimidin-5-yl)-5,6,7,8-tetrahydronaphthalene-2-carbonitrile

**HT342-6** (130 mg, 0.2 mmol) and **S6** (82 mg, 0.3 mmol) were dissolved in DMF (6 mL) in a dry flask, followed by addition of K₂CO₃ (55 mg, 0.4 mmol). The reaction was stirred at 60 °C for 6 hours, then poured into EA and washed with saturated brine. The organic phase was collected, dried and concentrated under reduced pressure, and the residue was purified by neutral prep-HPLC to obtain **HANT-342** (36.3 mg, yield: 25%) as a white solid.LCMS (ESI): *m*/*z =* 747.2 [M+H]⁺; ¹H NMR (400 MHz, DMSO-*d₆*) δ 11.10 (s, 1H), 8.08 (s, 2H), 7.83 (d, *J =* 8.3 Hz, 1H), 7.47 (d, *J =* 2.3 Hz, 1H), 7.38 (dd, *J =* 8.3, 2.3 Hz, 1H), 7.30 (s, 1H), 5.11 (dd, *J* = 12.9, 5.4 Hz, 1H), 4.44-4.36 (m, 2H), 4.33 (t, *J* = 4.6 Hz, 2H), 4.17 (dt, *J =* 13.4, 4.9 Hz, 2H), 4.04 (d, *J =* 6.5 Hz, 1H), 4.00-3.92 (m, 2H), 3.84 (t, *J =* 4.6 Hz, 2H), 3.66 (dt, *J =* 8.6, 4.6 Hz, 1H), 3.35-3.32 (m, 2H), 2.93-2.80 (m, 3H), 2.62-2.54 (m, 1H), 2.12-1.69 (m, 8H), 1.49-1.34 (m, 2H).

### Example 11

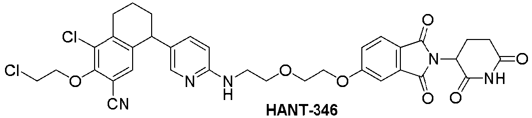

**HANT-346** was synthesized using procedures reported for **HANT-315** in Example 3, where starting material of **S1** in Example 3 was replaced with 5-bromo-2-fluoropyridine. The final product was purified by flash chromatography to afford **HANT-346** (8.3 mg, white solid, yield: 8%). LCMS (ESI): *m*/*z*=706.0 [M+H]⁺; ¹H NMR (400 MHz, DMSO-*d₆*) δ 11.08 (s, 1H), 7.83 (d, *J* = 8.3 Hz, 1H), 7.70 (d, *J =* 2.4 Hz, 1H), 7.46 (d, *J* = 2.4 Hz, 1H), 7.37 (dd, *J* = 8.3, 2.3 Hz, 1H), 7.18 (s, 1H), 7.06 (dd, *J =* 8.6, 2.5 Hz, 1H), 6.50-6.43 (m, 2H), 5.14-5.11 (m, 1H), 4.42-4.37 (m, 2H), 4.34-4.32 (m, 2H), 4.00-3.96 (m, 3H), 3.85-3.77 (m, 2H), 3.62-3.59 (m, 2H), 3.44-3.34 (m, 2H), 2.90-2. 84 (m, 3H), 2.65-2.54 (m, 2H), 2.08-2.03 (m, 1H), 1.98-1.90 (m, 1H), 1.77-1.71 (m, 3H).

### Example 12

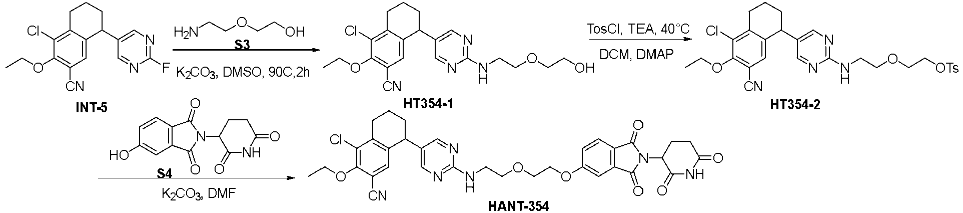

### Step 1: Synthesis of 4-chloro-3-ethoxy-8-(2-(2-hydroxyethoxy)ethyl)amino)pyrimidin-5-yl)-5,6,7,8-tetrahydronaphthalene-2-carbonitrile

**INT-5** (100 mg, 0.303 mmol) was dissolved in 5 mL of DMSO, then **S3** (64 mg, 0.606 mmol) and K₂CO₃ (84 mg, 0.606 mmol) were added, and the reaction was stirred at 90 °C for 2 h under N₂. The reaction was cooled to room temperature. The organic phase was filtered and poured into water and extracted with EA. The organic phase was washed with saturated brine, dried with Na₂SO₄, and purified by flash chromatography (DCM:MeOH=10:1) to obtain **HT354-1** (90 mg, white oily), yield: 74%. LCMS (ESI): *m*/*z* =417.3 [M+H]⁺.

### Step 2: Synthesis of 2-(2-(5-(5-chloro-7-cyano-6-ethoxy-1,2,3,4-tetrahydronaphthalen-1-yl)pyrimidin-2-yl)amino)ethoxy)ethyl-4-methylbenzensulfonate

**HT354-1** (90 mg, 0.216 mmol) was dissolved in 10 mL DCM, then TEA (66 mg, 0.647 mmol) and DMAP (8 mg, 0.065 mmol) were added and stirred at room temperature for 5 min. p-Toluenesulfonyl chloride (82 mg, 0.432 mmol) was added and the reaction was stirred at 40 °C under N₂ overnight. The organic phase was filtered and poured into water then extracted with DCM. The organic phase was washed with saturated brine, dried with Na₂SO₄, and purified by flash chromatography (DCM:MeOH=10:1) to obtain **HT354-2** (80 mg, yellow oily), yield: 61%. LCMS (ESI): *m*/*z* =571.1 [M+H]⁺.

### Step 3: Synthesis of 4-chloro-8-(2-(2-(2-(2,6-dioxopiperidin-3-yl)-1,3-dioxoisoindolin-5-yl)oxy)ethoxy)ethyl)amino)pyrimidin-5-yl-3-ethoxy-5,6,7,8-tetrahydronaphthalene-2-carbonitrile

**HT354-2** (90 mg, 0.158 mmol) was dissolved in 10 mL of DMF, S3 (65 mg, 0.236 mmol) and K₂CO₃ (40 mg, 0.315 mmol) were added, and the reaction was stirred for 3 h at 60 °C under N₂. The reaction was cooled to room temperature, filtered and poured into water, extracted with EA. The organic phase was washed with saturated brine, dried over Na₂SO₄, and purified by prep-HPLC to obtain compound **HANT-354** (8.9 mg, white solid), yield: 13%. LCMS (ESI): m/z =673.1 [M+H]⁺; ¹H NMR (400 MHz, DMSO-*d₆*) δ 11.10 (s, 1H), 7.82 (d, *J =* 8.3 Hz, 1H), 7.45 (d, *J* = 2.3 Hz, 1H), 7.36 (dd, *J =* 8.3, 2.3 Hz, 1H), 7.26 (s, 1H), 7.04 (t, *J =* 5.9 Hz, 1H), 5.11 (dd, *J =* 12.9, 5.4 Hz, 1H), 4.31 (dd, *J =* 5.7, 3.3 Hz, 2H), 4.17 (q, *J =* 7.0 Hz, 2H), 4.01 (d, *J =* 6.3 Hz, 1H), 3.82-3.76 (m, 2H), 3.60 (t, *J =* 6.1 Hz, 2H), 3.44 (q, *J =* 6.0 Hz, 2H), 2.99-2.76 (m, 3H), 2.69-2.53 (m, 2H), 2.12-1.89 (m, 2H), 1.76 (d, *J=* 8.4 Hz, 3H), 1.38 (t, *J =* 7.0 Hz, 3H).

### Example 13

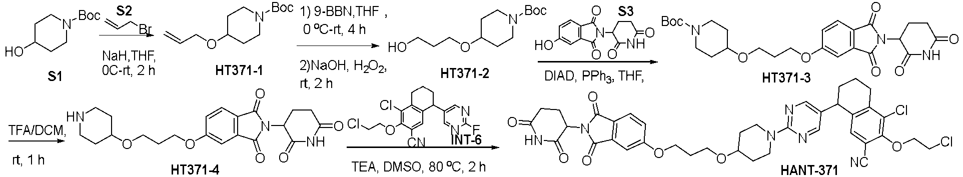

### Step 1: Synthesis of tert-butyl 4-(allyloxy)piperidine-1-carboxylate

**S1** (2 g, 10.0 mmol) was dissolved in THF (15 mL) in a dry flask, then NaH (0.8 g, 20.0 mmol) was added in under ice bath and stirred at 0 °C for 0.5 hr. S2 (1 mL, 12.0 mmol) was added and stirred at room temperature for 2 hours. The reaction was quenched with saturated aqueous NH₄Cl under ice bath, then the reaction solution was poured into EA and washed with saturated brine. The organic phase was collected and concentrated under reduced pressure, and the residue was purified by flash chromatography to obtain the colorless oily **HT371-1** (2.5 g, yield: 100%). LCMS (ESI): *m*/*z* =242.1 [M+H]⁺.

### Step 2: Synthesis of tert-butyl 4-(3-hydroxypropoxy)piperidine-1-carboxylate

**HT371-1** (1 g, 4.15 mmol) was dissolved in THF (20 mL) in a dry flask, then 9-BBN (12.5 mL, 25 mmol, 0.5 M in THF) was added dropwise under ice bath and stirred for 4 h at room temperature. 3 M NaOH(aq.) (3 mL) and 30% H₂O₂ aqueous solution were added into the reaction and it was stirred at room temperature for 2 h, then poured into saturated aq. NaHCO₃. The mixture was extracted with EA, and the organic phase was dried and concentrated under reduced pressure to obtain the crude **HT371-2,** which was used directly in next step. LCMS (ESI): m/z =260.2 [M+H]⁺.

### Step 3: Synthesis of tert-butyl 4-(3-((2-(2,6-dioxopiperidin-3-yl)-1,3-dioxoisoindolin-5-yl)oxy)propoxy)piperidine-1-carboxylate

To a dry flask was sequentially added **HT371-2** (100 mg, 0.39 mmol), S3 (100 mg, 0.36 mmol) and PPh₃ (150 mg, 0.57 mmol) in THF (5 mL), then DIAD (110 mg, 0.55 mmol) was added dropwise under an ice bath and stirred for 3 h at 60 °C under N₂ protection. The reaction was cooled to room temperature, concentrated under reduced pressure, and the resulting crude product was purified by prep-HPLC to obtain **HT371-3** (50 mg, yield: 28%) as a colorless oil. LCMS (ESI): *m*/*z* =516.2 [M+H]⁺.

### Step 4: Synthesis of 2-(2,6-dioxoopidin-3-yl)-5-(3-(piperidin-4-yloxy)propoxy)isoindole-1,3-dione

To a dry flask was added **HT371-3** (200 mg, 0.39 mmol) in DCM (10 mL) followed by TFA (2 mL). The reaction was stirred for 1 hour at room temperature, then concentrated by reduced pressue to obtain the crude **HT371-4** as a colorless oil, which was used directly in next step. LCMS (ESI): *m*/*z* =416.2 [M+H]⁺.

### Step 5: Synthesis of 4-chloro-3-(2-chloroethoxy)-8-(2-(4-(3-((2-(2,6-dioxopiperidin-3-yl)-1,3-dioxoisoindol-5-yl)oxy)propoxy)piperidin-1-yl)pyrimidin-5-yl)-5,6,7,8-tetrahydronaphthalene-2-carbonitrile

To a dry flask was added **HT371-4** (80 mg, 0.2 mmol) and **INT-6** (73 mg, 0.2 mmol) in DMSO (5 mL), followed by addition of TEA (60 mg, 0.6 mmol). The reaction was stirred at 80 °C for 2 hours, then cooled to room temperature, poured into EA and washed with saturated brine. The organic phase was collected, dried and concentrated under reduced pressure. The residue was purified by prep-HPLC to obtain **HANT-371** (57.9 mg, yield: 38%) as a white solid. LCMS (ESI): *m*/*z* =761.2 [M+H]⁺; ¹H NMR (400 MHz, DMSO-*d₆*) δ 11.00 (s, 1H), 8.07 (s, 2H), 7.83 (d, *J =* 8.3 Hz, 1H), 7.44 (d, *J =* 2.3 Hz, 1H), 7.36 (dd, *J =* 8.3, 2.3 Hz, 1H), 7.29 (s, 1H), 5.11 (dd, *J =* 12.9, 5.4 Hz, 1H), 4.39 (dd, *J =* 5.8, 4.6 Hz, 2H), 4.26 (t, *J =* 6.3 Hz, 2H), 4.18-4.09 (m, 2H), 4.08-4.01 (m, 1H), 3.99-3.90 (m, 2H), 3.62 (t, *J =* 6.1 Hz, 2H), 3.56 (p, *J =* 4.3 Hz, 1H), 3.39-3.32 (m, 2H), 2.95-2.75 (m, 3H), 2.64-2.52 (m, 2H), 2.08-1.91 (m, 4H), 1.90-1.68 (m, 5H), 1.45-1.37 (m, 2H).

### Example 14

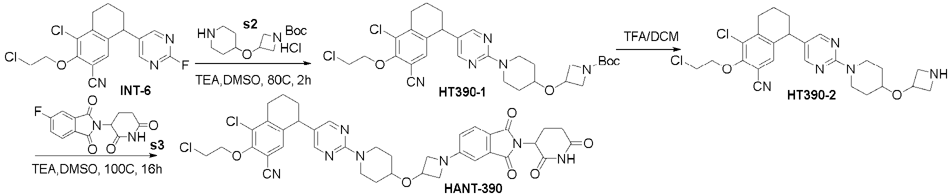

### Step 1: Synthesis of tert-butyl 3-((1-(5-(5-chloro-6-(2-chloroethoxy)-7-cyano-1,2,3,4-tetrahydronaphthalen-1-yl)pyrimidin-2-yl)piperidin-4-yl)oxy)azetidine-1-carboxylate

To a dry flask was added **INT-6** (100 mg, 0.274 mmol), **S2** (80 mg, 0.313 mmol), TEA (140 mg, 1.39 mmol) and DMSO (5 mL) sequentially and stirred at 80 °C for 2 hrs. EA was poured into cooled reaction, and the mixture was washed with saturated brine. The organic phase was collected and concentrated under reduced pressure and purified by flash chromatography to obtain **HT390-1** (140 mg, yield: 84.8%) as a brown oil. LCMS: *m*/*z* = 602.2 [M+H]⁺.

### Step 2: Synthesis of 8-(2-(4-(azetidin-3-yloxy)piperidin-1-yl)pyrimidin-5-yl)-4-chloro-3-(2-chloroethoxy)-5,6,7,8-tetrahydronaphthalene-2-carbonitrile

To a dry flask was added **HT390-1** (140 mg, 0.232 mmol) in DCM (2 mL), then TFA (1 mL) was added and stirred at room temperature for 2 hours. The reaction was filtered, and the filtrate was concentrated under reduced pressure. The residue was extracted with EA and saturated aq. NaHCO₃. The organic phase was collected and concentrated under reduced pressure to obtain the crude **HT390-2** (100 mg). LCMS: *m*/*z* = 502.2 [M+H]⁺.

### Step 3: Synthesis of 4-chloro-3-(2-chloroethoxy)-8-(2-(4-((1-(2-(2-(2,6-dioxopiperidin-3-yl)-1,3-dioxoisoindol-5- yl)azetidin-3- yl)oxy)piperidin-1-yl)pyrimidin-5-yl)-5,6,7,8-tetrahydronaphthalene-2-carbonitrile

To a dry flask was added **HT390-2** (100 mg, 0.199 mmol) in DMSO (2 mL), then S3 (85 mg, 0.308 mmol) and TEA (100 mg, 0.990 mmol) were added in, and the reaction was stirred at 100 °C for 16 h. The reaction was cooled down to room temperature, then EA was poured in, and washed with saturated brine. The organic phase was collected and concentrated under reduced pressure, then purified by prep-HPLC to obtain a yellow solid **HANT-390** (25.9 mg, yield: 17.2%). LCMS: *m*/*z* =758.3 [M+H]⁺; ¹H NMR (400 MHz, DMSO-*d₆*) δ 11.07 (s, 1H), 8.09 (s, 2H), 7.65 (d, *J =* 8.3 Hz, 1H), 7.30 (s, 1H), 6.81 (d, *J =* 1.8 Hz, 1H), 6.66 (dd, *J =* 8.4, 2.0 Hz, 1H), 5.06 (dd, *J =* 12.9, 5.4 Hz, 1H), 4.71-4.59 (m, 1H), 4.42-4.36 (m, 2H), 4.34-4.22 (m, 4H), 4.05 (t, J = 5.6 Hz, 1H), 4.00-3.94 (m, 2H), 3.85 (dd, *J =* 9.0, 4.2 Hz, 2H), 3.72-3.63 (m, 1H), 3.28-3.16 (m, 2H), 2.94-2.78 (m, 3H), 2.62-2.52 (m, 2H), 2.05-1.94 (m, 2H), 1.93-1.84 (m, 2H), 1.83-1.69 (m, 3H), 1.49-1.36 (m, 2H).

### Example 15

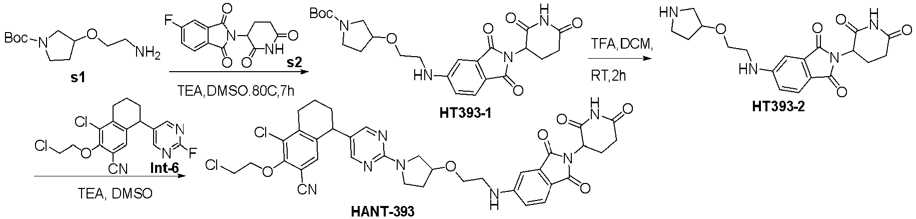

### Step 1: Synthesis of tert-butyl 3-(2-((2-(2,6-dioxopiperidin-3-yl)-1,3-dioxoisoindolin-5-yl)amino)ethoxy)pyrrolidine-1-carboxylate

**S1** (200 mg, 0.87 mmol) was dissolved in DMSO (2 mL) in a dry flask, followed by addition of S2 (360 mg, 1.30 mmol) and TEA (440 mg, 4.36 mmol). The reaction was stirred at 90 °C for 7 hours, then cooled to room temperature and poured into EA, washed with saturated brine. The organic phase was collected and concentrated under reduced pressure, and the residue was purified by prep-HPLC to obtain **HT-393-1** (110 mg, yield: 26.1%) as a yellow solid. LCMS: *m*/*z* = 487.1 [M+H]⁺.

### Step 2: Synthesis of 2-(2,6-dioxoopidin-3-yl)-5-((2-(pyrrolidin-3-yloxy)ethyl)amino)isoindole-1,3-dione

**HT393-1** (110 mg, 0.226 mmol), DCM (2 mL), and TFA (1 mL) were added into a dry flask and stirred at room temperature for 2 hours. The reaction was concentrated under reduced pressure, then EA and saturated aq. NaHCO₃ were added. The organic phase was collected and concentrated under reduced pressure to obtain compound **HT393-2** (90 mg, crude). LCMS: m/z = 387.3 [M+H]⁺.

### Step 3: Synthesis of 4-chloro-3-(2-chloroethoxy)-8-(2-(3-(2-(2-((2-(2,6-dioxopiperidin-3-yl)-1,3-dioxoisoindol-5-yl)amino)ethoxypyrrolidin-1-yl)pyrimidin-5-yl)-5,6,7,8-tetrahydronaphthalene-2-carbonitrile

To a dry flask was sequentially added **HT393-2** (60 mg, 0.155 mmol), **INT-6** (62 mg, 0.169 mmol) and TEA (78 mg, 0.772 mmol) in DMSO (5 mL), and the reaction was stirred at 80 °C for 2 hours. The reaction was cooled to room temperature, then poured into EA and washed with saturated brine. The organic phase was collected and concentrated under reduced pressure, and the residue was purified by flash chromatography to obtain **HANT-393** (52 mg, yield: 45.8%) as a yellow solid. LCMS: *m*/*z* =732.2 [M+H]⁺; ¹H NMR (400 MHz, DMSO-*d₆*) δ 11.05 (s, 1H), 8.06 (s, 2H), 7.52 (dd, *J =* 8.4, 2.5 Hz, 1H), 7.27 (d, *J*=5.1 Hz, 1H), 7.12 (t, *J*=5.6 Hz, 1H), 7.01 (s, 1H), 6.88 (d, *J*=8.4 Hz, 1H), 5.02 (dd, *J*=12.9, 5.4 Hz, 1H), 4.41-4.34 (m, 2H), 4.21 (s, 1H), 4.03 (t, *J=*5.6 Hz, 1H), 3.98-3.91 (m, 2H), 3.67-3.59 (m, 2H), 3.58-3.48 (m, 3H), 3.48-3.37 (m, 3H), 2.95-2.77 (m, 3H), 2.63-2.52 (m, 2H), 2.06-1.92 (m, 4H), 1.85-1.70 (m, 3H).

### Example 16

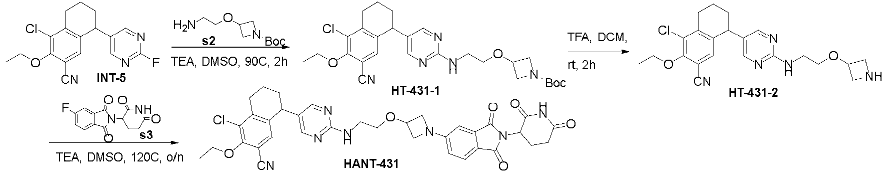

### Step 1: Synthesis of tert-butyl 3-(2-((5-(5-chloro-7-cyano-6-ethoxy-1,2,3,4-tetrahydronaphthalen-1-yl)pyrimidin-2-yl)amino)ethoxy)azetidine-1-carboxylate

**INT-5** (100 mg, 0.3 mmol), **S2** (98 mg, 0.45 mmol) and TEA (91 mg, 0.90 mmol) were dissolved in 10 mL of DMF and stirred at 90 °C for 2 hours under N₂. The reaction was cooled to room temperature, then extracted with EA. The organic phase was dried with anhydrous Na₂SO₄, filtered and concentrated under reduced pressure. The crude product was purified by flash chromatography to obtain compound **HT431-1** (110 mg, yellow oil, 70% yield). LCMS (ESI): *m*/*z* =528.2 [M+H]⁺.

### Step 2: Synthesis of 8-(2-((2-(azetidin-3-yloxy)ethyl)amino)pyrimidin-5-yl)-4-chloro-3-ethoxy-5,6,7,8-tetrahydronaphthalene-2-carbonitrile

**HT431-1** (110 mg, 0.21 mmol) and TFA (1 mL) were dissolved in 10 mL DCM and stirred at room temperature for 2 hours. The reaction was neutralized with saturated aq. NaHCO₃, and the organic phase was dried with anhydrous Na₂SO₄ and concentrated under reduced pressure. The crude product was purified by flash chromatography to obtain compound **HT431-2** (80 mg, yellow oily body, yield: 91%). LCMS (ESI): *m*/*z* =428.2 [M+H]⁺.

### Step 3: Synthesis of 4-chloro-8-(2-((2-(2-(1-(2-(2,6-dioxopiperidin-3-yl)-1,3-dioxoisoindol-5-yl)azetidin-3-yl)oxy)ethyl)amino)pyrimidin-5-yl)-3-ethoxy-5,6,7,8-tetrahydronaphthalene-2-carbonitrile

**HT431-2** (80 mg, 0.18 mmol), **S3** (78 mg, 0.28 mmol), and TEA (91 mg, 0.90 mmol) were dissolved in 10 mL DMSO and stirred overnight at 120 °C under N₂. The reaction was cooled to room temperature, then extracted with EA. The organic phase was dried with anhydrous Na₂SO₄ and concentrated under reduced pressure. The crude product was purified by flash chromatography to obtain compound **HANT-431** (13.4 mg, white solid, yield: 11%). LCMS (ESI): *m*/*z* =684.2 [M+H]⁺; ¹H NMR (400 MHz, DMSO-*d₆*) δ 11.05 (s, 1H), 8.02 (s, 2H), 7.64 (d, *J =* 8.3 Hz, 1H), 7.27 (s, 1H), 7.13 (t, *J =* 5.7 Hz, 1H), 6.80 (d, *J =* 2.1 Hz, 1H), 6.66 (dd, *J =* 8.4, 2.2 Hz, 1H), 5.05 (dd, *J =* 12.9, 5.4 Hz, 1H), 4.53-4.48 (m, 1H), 4.28-4.22 (m, 2H), 4.20-4.17 (m, 2H), 4.01 (t, *J =* 6.1 Hz, 1H), 3.86 (dd, *J =* 9.6, 3.9 Hz, 2H), 3.57-3.54 (m, 2H), 3.45 (q, *J* = 5.9 Hz, 2H), 2.88-2.83 (m, 3H), 2.61-2.54 (m, 2H), 2.05-1.95 (m, 2H), 1.85-1.77 (m, 3H), 1.38 (t, *J* = 7.0 Hz, 3H).

### Example 17

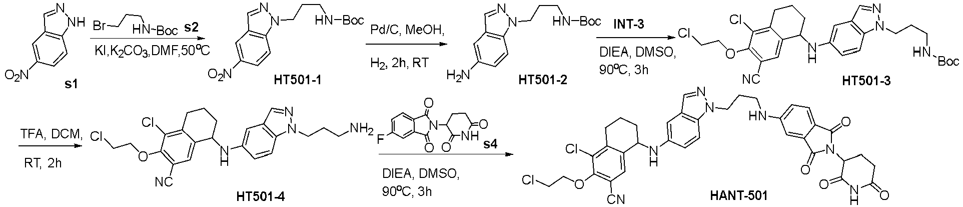

### Step 1: Synthesis of tert-butyl (3-(5-nitro-1H-indazol-1-yl)propyl)carbamate

**INT-5** (100 mg, 0.3 mmol), **S2** (98 mg, 0.45 mmol) and TEA (91 mg, 0.90 mmol) were dissolved in 10 mL DMF and stirred at 90 °C under N₂ for 2 hours. The reaction was cooled to room temperature then extracted with EA. The organic phase was dried with anhydrous Na₂SO₄, filtered, and concentrated under reduced pressure. The crude product was purified by flash chromatography to obtain compound **HT501-1** (110 mg, yellow oil, 70% yield). LCMS (ESI): *m*/*z* =528.2 [M+H]⁺.

### Step 2: Synthesis of 8-(2-((2-(azetidin-3-yloxy)ethyl)amino)pyrimidin-5-yl)-4-chloro-3-ethoxy-5,6,7,8-tetrahydronaphthalene-2-carbonitrile

**HT501-1** (110 mg, 0.21 mmol) and TFA (1 mL) were dissolved in 10 mL DCM and stirred at room temperature for 2 h. The reaction was neutralized with saturated aq. NaHCO₃, then the organic phase was dried with anhydrous Na₂SO₄ and concentrated under reduced pressure. The residue was purified by flash chromatography to obtain **HT501-2** (80 mg, yellow oily body, yield: 91%). LCMS (ESI): *m*/*z =*428.2 [M+H]⁺.

### Step 3: Synthesis of tert-butyl (3-(5-((5-chloro-6-(2-chloroethoxy)-7-cyano-1,2,3,4-tetrahydronaphthalen-1-yl)amino)-1H-indazole-1-propyl)carbamate

**HT501-2** (460 mg, 1.59 mmol) and **INT-3** (826 mg, 2.4 mmol) were dissolved in 10 mL of DMSO, and DIEA (614 mg, 4.76 mmol) was added in and the reaction was stirred at 90 °C for 3 hours. Water was added to the reaction, and the reaction mixture was extracted with EA. The organic phase was backwashed with saturated brine, dried over anhydrous Na₂SO₄, filtered and concentrated under reduced pressure. The residue was purified by flash chromatography to obtain compound **HT501-3** (300 mg, yield: 34%); LCMS (ESI): *m*/*z* =558.2[M+H]⁺.

### Step 4: Synthesis of 8-(1-(3-aminopropyl)-1H-indazole-5-amino)-4-chloro-3-(2-chloroethoxy)-5,6,7,8-tetrahydronaphthalene-2-carbonitrile

**HT501-3** (300 mg, 0.54 mmol) was dissolved in DCM (10 mL) then TFA (3 mL) was added and reacted at room temperature for 2 h. The reaction solution was adjusted with aq. NaHCO₃ to pH = 8, and then extracted with DCM. The organic phase was dried, filtered, and concentrated to obtain the crude product **HT501-4** (300 mg). LCMS (ESI): *m*/*z* =458.1[M+H]⁺.

### Step 5: Synthesis of 4-chloro-3-(2-chloroethoxy)-8-(1-(2-(2-(2,6-dioxopiperidin-3-yl)-1,3-dioxoisoindolin-5-yl)amino)propyl)-1H-indazole-5-amino)-5,6,7,8-tetrahydronaphthalene-2-carbonitrile

**HT501-4** (300 mg, 0.66 mmol) and **S4** (270 mg, 0.98 mmol) were dissolved in 10 mL DMSO, then DIEA (250 mg, 1.94 mmol) was added and the reaction was stirred at 90 °C for 3 hours. The reaction was extracted with EA and the organic phase was dried and concentrated. The residue was purified by prep-HPLC to obtain **HANT-501** (75.2 mg, yield: 16%); LCMS (ESI): *m*/*z* =714.2 [M+H]⁺.¹H NMR (400 MHz, DMSO) δ 11.05 (s, 1H), 7.79 (s, 1H), 7.73 (s, 1H), 7.55 (d, *J*=8.3 Hz, 1H), 7.45 (d, *J*=8.9 Hz, 1H), 7.18 (t, *J*=5.2 Hz, 1H), 7.02- 6.91 (m, 2H), 6.86 (s, 1H), 6.80 (dd, *J*=8.3, 0.7 Hz, 1H), 5.73 (d, *J*=8.8 Hz, 1H), 5.03 (dd, *J=*12.8*,* 5.4 Hz, 1H), 4.63 (s, 1H), 4.45-4.38 (m, 4H), 4.04-3.93 (m, 2H), 3.32-3.21 (m, 2H), 2.94-2.82 (m, 2H), 2.81-2.66 (m, 1H), 2.62-2.51 (m, 2H), 2.16-2.05 (m, 2H), 2.04-1.95 (m, 1H), 1.90-1.72 (m, 4H).

### Example 18

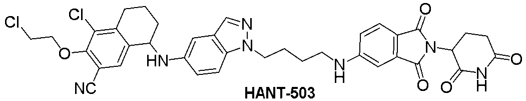

**HANT-503** was synthesized using procedures reported for **HANT-501** in Example 17, where starting material **S2** in Example 17 was replaced with tert-Butyl (4-bromobutyl)carbamate. The final product was purified by prep-HPLC to afford **HANT-503** (40.7 mg, yield: 6%); LCMS (ESI): *m*/*z* =728.2 [M+H]⁺.¹H NMR (400 MHz, DMSO) δ 11.06 (s, 1H), 7.77 (s, 1H), 7.73 (s, 1H), 7.54 (d, *J =* 8.4 Hz, 1H), 7.46 (d, *J* = 9.0 Hz, 1H), 7.10 (t, *J =* 5.3 Hz, 1H), 6.94 (td, *J =* 4.4, 2.0 Hz, 2H), 6.86 - 6.75 (m, 2H), 5.73 (d, *J =* 8.8 Hz, 1H), 5.03 (dd, *J =* 12.9, 5.3 Hz, 1H), 4.63 (d, *J* = 6.1 Hz, 1H), 4.43-4.31 (m, 4H), 4.02-3.92 (m, 2H), 3.19-3.14 (m, 2H), 2.94-2.81 (m, 2H), 2.80-2.65 (m, 1H), 2.62-2.51 (m, 2H), 2.03-1.95 (m, 1H), 1.94-1.86 (m, 3H), 1.86-1.75 (m, 3H), 1.56 - 1.47 (m, 2H).

### Example 19

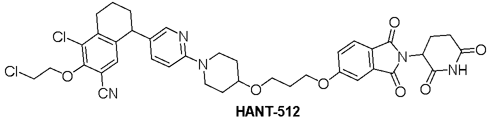

**HANT-512** was synthesized using procedures reported for **HANT-315** in Example 3, where S1 and S2 in Example 3 was replaced with 5-bromo-2-fluoropyridine and 3-(piperidin-4-yloxy)propan-1-ol. The final product was purified by prep-HPLC to afford compound **HANT-512** (18.0 mg, yellow solid), yield: 19.6 %. LCMS (ESI) *m*/*z* =760.2 [M+H]⁺; RT= 2.318 min (5.0 min). ¹H NMR (400 MHz, DMSO) δ 11.11 (s, 1H), 7.97-7.65 (m, 2H), 7.44 (d, *J*=2.2 Hz, 1H), 7.36 (dd, *J*=8.3, 2.2 Hz, 1H), 7.18 (dd, *J*=8.6, 2.6 Hz, 2H), 6.77 (d, *J*=8.8 Hz, 1H), 5.11 (dd, *J*=12.9, 5.3 Hz, 1H), 4.56-4.35 (m, 2H), 4.25 (t, *J*=6.3 Hz, 2H), 4.04 (t, *J*=5.7 Hz, 1H), 4.00-3.78 (m, 4H), 3.61 (t, *J=*6.1 Hz, 2H), 3.54-3.50 (m, 1H), 3.11 (t, *J*=10.2 Hz, 2H), 2.97-2.77 (m, 3H), 2.72-2.56 (m, 2H), 2.18-1.91 (m, 4H), 1.93-1.68 (m, 5H), 1.42 (dd, *J*=18.1, 8.8 Hz, 2H).

### Example 20

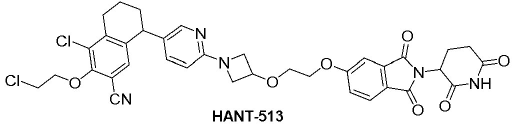

**HANT-513** was synthesized using procedures reported for **HANT-315** in Example 3, where S1 and S2 in Example 3 was replaced with 5-bromo-2-fluoropyridine and 2-(azetidin-3-yloxy)ethan-1-ol. The final product was purified by prep-HPLC to afford compound **HANT-513** (27.4 mg, yellow solid) in 23.6 % yield. LCMS (ESI): *m*/*z* =718.2 [M+H]⁺; RT= 2.172 min (5.0 min). ¹H NMR (400 MHz, DMSO-*d₆*) δ 11.10 (s, 1H), 7.84 (d, *J =* 8.2 Hz, 2H), 7.48 (d, *J =* 2.2 Hz, 1H), 7.39 (dd, J = 8.3, 2.3 Hz, 1H), 7.20 (dd, *J =* 8.6, 2.4 Hz, 1H), 7.15 (s, 1H), 6.36 (d, *J* = 8.5 Hz, 1H), 5.12 (dd, *J =* 12.9, 5.4 Hz, 1H), 4.62-4.44 (m, 1H), 4.48-4.24 (m, 4H), 4.23-4.09 (m, 2H), 4.05 (t, *J* = 5.6 Hz, 1H), 4.00-3.88 (m, 2H), 3.88-3.61 (m, 4H), 2.89-2.84 (m, 3H), 2.70-2.53 (m, 2H), 2.09-1.91 (m, 2H), 1.87-1.61 (m, 3H).

### Example 21

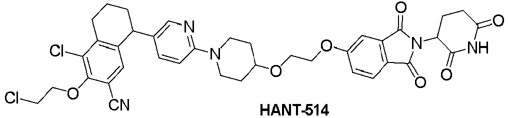

**HANT-514** was synthesized using procedures reported for **HANT-315** in Example 3, where starting materials S1 and S2 in Example 3 was replaced with 5-bromo-2-fluoropyridine and 2-(piperidin-4-yloxy)ethan-1-ol. The final product was purified by neutral prep-HPLC to afford a white solid, HANT-514 (35 mg, yield: 30.2 %). LCMS (ESI): *m*/*z* =746.2 [M+H]⁺; RT= 1.343 min (2.5 min).¹H NMR (400 MHz, DMSO-*d*₆) δ 11.10 (s, 1H), 7.91-7.77 (m, 2H), 7.47 (d, *J =* 2.2 Hz, 1H), 7.37 (dd, *J =* 8.3, 2.3 Hz, 1H), 7.25-7.13 (m, 2H), 6.79 (d, *J =* 8.8 Hz, 1H), 5.11 (dd, *J =* 12.9, 5.4 Hz, 1H), 4.45-4.37 (m, 2H), 4.35-4.29 (m, 2H), 4.09-4.01 (m, 1H), 3.99-3.88 (m, 4H), 3.86-3.78 (m, 2H), 3.68-3.56 (m, 1H), 3.17-3.04 (m, 2H), 2.96-2.80 (m, 3H), 2.64-2.52 (m, 2H), 2.12-2.01 (m, 1H), 2.00-1.86 (m, 3H), 1.85-1.69 (m, 3H), 1.51-1.35 (m, 2H).

### Example 22

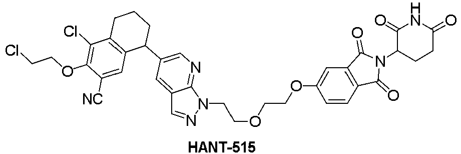

**HANT-515** was synthesized using procedures reported for **HANT-518** in Example 23, where S1 in Example 23 was replaced with HT515-1. The intermediate HT515-1 was synthesized as follows:

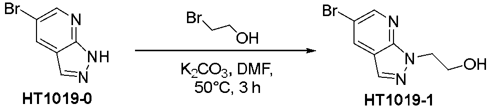

Compound HT515-0 (2.00 g, 10.1 mmol) was dissolved in DMF (50 mL), then 2-bromoethan-1-ol (1.50 g, 12.0 mmol) and K₂CO₃ (4.10 g, 29.7 mmol) were added, and the reaction was stirred at 50 °C for 3 hours. After cooled to room temperature, water (100 mL) was added into the reaction and the mixture was extracted with EA (100 mL). The organic phase was washed with saturated brine (50 mL), dried with anhydrous Na₂SO₄, filtered and concentrated under reduced pressure. The residue was purified by flash chromatography (EA:PE=1:1) to obtain the white solid crude product **HT515-1** (0.870 g, yield: 35.7%). LCMS (ESI): m/z =242.0 [M+H]⁺; RT= 1.09 min (1.80 min).

The final product was purified by prep-HPLC to afford **HANT-515** (40.6 mg, yellow solid, yield: 35.2%). LCMS (ESI): *m*/*z* =731.2 [M+H]⁺; RT= 2.52 min (5.00 min). ¹H NMR (400 MHz, DMSO-*d*₆) δ 11.11 (s, 1H), 8.40 (d, *J*=2.0 Hz, 1H), 8.05 (d, *J* =1.5 Hz, 1H), 7.78 (d, *J*=8.4 Hz, 2H), 7.35 (s, 1H), 7.25 (dd, *J*=8.3, 2.2 Hz, 1H), 7.23 (s, 1H), 5.12 (dd, *J*=12.9, 5.4 Hz, 1H), 4.62 (t, *J*=8.0 Hz, 2H), 4.41 (s, 1H), 4.40 (t, *J*=4.0 Hz, 2H), 4.21 (t, *J* =4.0 Hz, 2H), 3.98 (m, 4H), 3.77 (t, *J =* 4.0 Hz, 2H), 2.89 (m, 3H), 2.66-2.54 (m, 2H), 2.07 (s, 1H), 2.06-2.01 (m, 1H), 1.86 (m, 1H), 1.78 (m, 2H).

### Example 23

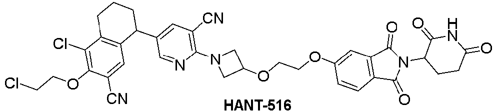

**HANT-516** was synthesized using procedures reported for **HANT-315** in Example 3, where S1 and S2 in Example 3 was replaced with 5-bromo-2-fluoronicotinonitrile and 2-(azetidin-3-yloxy)ethan-1-ol. The final product was purified by prep-HPLC to afford **HANT-516** (13 mg, yield: 11.1%). LCMS (ESI): *m*/*z =* 743.1 [M+H]⁺, RT= 2.75 min (5.00 min); ¹H NMR (400 MHz, DMSO-*d*₆) δ 11.11 (s, 1H), 8.11 (d, *J =* 2.2 Hz, 1H), 7.84 (d, *J =* 8.3 Hz, 1H), 7.68 (d, *J =* 2.1 Hz, 1H), 7.48 (d, *J =* 2.1 Hz, 1H), 7.39 (dd, *J =* 8.3, 2.2 Hz, 1H), 7.23 (s, 1H), 5.12 (dd, *J =* 13.0, 5.3 Hz, 1H), 4.53 (s, 1H), 4.49-4.32 (m, 6H), 4.12 (s, 1H), 4.03 (dd, *J =* 9.5, 3.8 Hz, 2H), 4.01-3.91 (m, 2H), 3.83 (s, 2H), 2.87 (dd, *J =* 13.4, 5.5 Hz, 3H), 2.63-2.52 (m, 2H), 2.11-2.01 (m, 1H), 1.95 (s, 1H), 1.75 (d, *J =* 7.5 Hz, 3H).

### Example 24

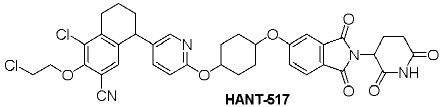

**HANT-517** was synthesized using procedures reported for **HANT-315** in Example 3, where S1 and S2 in Example 3 was replaced with 5-bromo-2-fluoropyridine and cyclohexane-1,4-diol. The final product was purified by prep-HPLC to afford **HANT-517** (4.8 mg, yellow solid), yield: 5.6 %. LCMS (ESI): *m*/*z* =717.2 [M+H]⁺; RT= 1.51 min (1.80 min). ¹H NMR (400 MHz, DMSO-*d₆*) δ 11.10 (s, 1H), 7.88 (d, *J*=2.4 Hz, 1H), 7.85-7.81 (m, 1H), 7.49 (d, *J*=2.2 Hz, 1H), 7.43-7.36 (m, 2H), 7.22 (s, 1H), 6.75 (d, *J*=8.6 Hz, 1H), 5.11 (dd, *J=*12.9, 5.3 Hz, 2H), 4.84 (s, 1H), 4.48-4.33 (m, 2H), 4.16 (d, *J*=6.7 Hz, 1H), 4.03-3.89 (m, 2H), 2.93-2.78 (m, 3H), 2.71-2.56 (m, 2H), 2.02 (dd, *J*=15.2, 7.9 Hz, 2H), 1.87 (s, 8H), 1.77 (d, *J*=6.5 Hz, 3H).

### Example 25

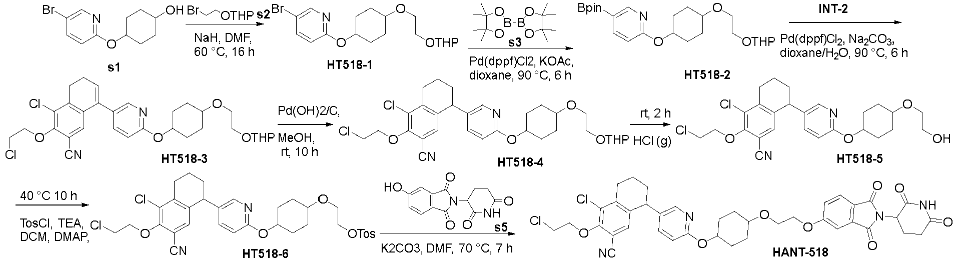

### Step 1: 5-bromo-2-((4-(2-((tetrahydro-2H-pyran-2-yl)oxy)ethoxy)cyclohexyl)oxy)

**S1** (2.00 g, 7.38 mmol) was dissolved in DMF (50 mL), then NaH (0.440 g, 18.3 mmol) was added under N₂ protection in an ice bath and stirred for 1 h. S2 (2.20 g, 10.5 mmol) was added, and the reaction was stired at 60 °C for 16 h. The reaction was cooled down, quenched by water (100 mL), and extracted with EA (100 mL). The organic phase was washed with saturated brine (50 mL), dried with anhydrous Na₂SO₄, filtered and concentrated under reduced pressure. The residue was purified by flash chromatography (EA:PE=1:3) to obtain **HT518-1** (0.500 g, yield: 17.0%) as a white solid. LCMS (ESI): *m*/*z* =422.0 [M+H]⁺; RT = 1.50 min (1.80 min).

### Step 2: 2-((4-(2-((tetrahydro-2H-pyran-2-yl)oxy)ethoxy)cyclohexyl)oxy)-5-(4,4,5,5-tetramethyl-1,3,2-dioxaborolan-2-yl)pyridine

**HT518-1** (350 mg, 0.88 mmol), **S3** (446 mg, 1.76 mmol), KOAc (258 mg, 2.63 mmol) and Pd(dppf)Cl₂ (64 mg, 0.087 mmol) were sequentially dissolved in 1,4-dioxane (20 mL) in a dry flask, and stirred for 6 hours under N₂ at 90 °C. The resulting reaction solution **HT518-2** was used directly in next step.

### Step 3: 4-Chloro-3-(2-chloroethoxy)-8-(6-((4-(2-(tetrahydro-2H-pyran-2-yl)ethoxy]cyclohexyl)oxy)pyridin-3-yl)-5,6-dihydronaphthalene-2-carbonitrile

To the reaction solution **HT518-2** was added water (4 mL), **INT-2** (300 mg, 0.721 mmol), Na₂CO₃ (229 mg, 2.16 mmol) and Pd(dppf)Cl₂ (53 mg, 0.073 mmol), and the reaction was stirred at 90 °C under N₂ for 6 hours. The reaction was cooled to room temperature and extracted with EA. The organic phase was washed, dried and concentrated. The residue was purified by flash chromatography (EA:PE=1:2) to obtain **HT518-3** (260 mg, colorless oil) as a yellow solid, yield: 71.7%. LCMS (ESI): *m*/*z* =587.3 [M+H]⁺; RT = 1.660 min (2.50 min).

### Step 4: 4-Chloro-3-(2-chloroethoxy)-8-(6-(4-(2-(tetrahydro-2H-pyran-2-yl)oxy)ethoxycyclohexyl)oxypyridin-3-yl)-5,6,7,8-tetrahydronaphthalene-2-carbonitrile

**HT518-3** (260 mg, 0.517 mmol) was dissolved in methanol (5 mL), then Pd(OH)₂/C (70 mg) was added and stirred under H₂ for 10 h at room temperature. The reaction was filtered, concentrated to obtain the crude **HT518-4** (220 mg, colorless oil), yield: 84.3%. LCMS (ESI): *m*/*z* = 589 [M+H]⁺; RT = 3.036 min (5.0 min).

### Step 5: 4-Chloro-3-(2-chloroethoxy)-8-(6-((4-(2-hydroxyethoxycyclohexyl)oxy)pyridin-3-yl)-5,6,7,8-tetrahydronaphthalene-2-carbonitrile

**HT518-4** (260 m g, 0.442 mmol) was dissolved in DCM (4 mL), then HCl in 1,4-dioxane (1 mL, 4 M) was added, and the reaction was stirred at room temperature for 2 hours. The reaction was quenched by water, then adjusted to pH~8 with aq. NaHCO₃ under ice bath. The organic phase was washed with saturated brine, dried with anhydrous Na₂SO₄, filtered and concentrated under reduced pressure. The residue was purified by flash chromatography (EA:PE=3:2) to obtain compound **HT518-6** (140 mg, white oily), yield: 62.8%. LCMS (ESI): *m*/*z* =505.2 [M+H]⁺; RT= 2.544; RT = 2.544 min (5 min).

### Step 6: 2-((4-((5-(5-chloro-6-(2-chloroethoxy)-7-cyano-1,2,3,4-tetrahydronaphthalen-1-yl)pyridin-2-yl)oxy)cyclohexyl)oxy)

**HT518-5** (140 mg, 0.278 mmol) was dissolved in DCM (5 mL), followed by p-toluenesulfonyl chloride (105 mg, 0.553 mmol), TEA (84.0 mg, 0.832 mmol), and DMAP (3.40 mg, 0.0278 mmol). The reaction was stirred at 40 °C for 10 h, then water (10 mL) was added in, and the mixture was extracted with DCM (10 mL). The organic phase was washed with saturated brine (10 mL), dried with anhydrous Na₂SO₄, filtered and concentrated under reduced pressure. The residue was purified by flash chromatography (EA:PE=1:2) to obtain **HT518-6** (160 mg, red oily), yield: 87.5 %. LCMS (ESI): *m*/*z* =659.2 [M+H]⁺; RT = 1.61 min (1.80 min).

### Step 7: 4-Chloro-3-(2-chloroethoxy)-8-(6-((4-(2-((2-(2-(2,6-dioxopiperidin-3-yl)-1,3-dioxoisoindol-5-yl)oxy)ethoxycyclohexyl)oxypyridin-3-yl)-5,6,7,8-tetrahydronaphthalene-2-carbonitrile

**HT518-6** (140 mg, 0.212 mmol) was dissolved in DMF (5 mL), then **S5** (117 mg, 0.424 mmol) and K₂CO₃ (88 mg, 0.637 mmol) were added, and the reaction was stirred at 70 °C for 7 h. The reaction was cooled to room temperature and extracted with EA. The organic phase was washed with saturated brine, dried over anhydrous Na₂SO₄, and purified by HPLC (formic acid system) to obtain **HANT-518** (47.3 mg, white solid), yield: 29.2%. LCMS (ESI): *m*/*z* =761.2 [M+H]⁺; RT = 3.081 min (5.0 min). ¹H NMR (400 MHz, DMSO-*d*₆) δ 11.12 (s, 1H), 7.93-7.75 (m, 2H), 7.52-7.44 (m, 1H), 7.41-7.33 (m, 2H), 7.24-7.18 (m, 1H), 6.73-6.66 (m, 1H), 5.12 (dd, *J*=12.9, 5.3 Hz, 1H), 5.06-4.90 (m, 1H), 4.45-4.37 (m, 2H), 4.34-4.28 (m, 2H), 4.20-4.12 (m, 1H), 4.02-3.92 (m, 2H), 3.84-3.75 (m, 2H), 3.54-3.41 (m, 1H), 2.94-2.79 (m, 3H), 2.65-2.52 (m, 2H), 2.12-1.91 (m, 5H), 1.88-1.59 (m, 5H), 1.54-1.30 (m, 3H).

### Example 26

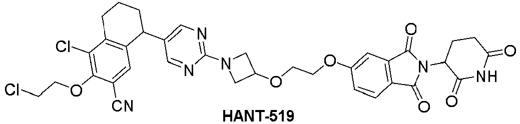

**HANT-519** was synthesized using procedures reported for **HANT-371** in Example 13, where HT371-2 in Example 13 was replaced with HT519-1. The intermediate HT519-1 was synthezied as below:

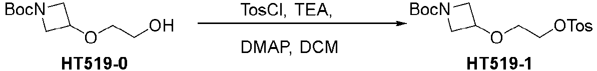

The crude **HT519-0** (200 mg, 0.920 mmol) and TosCl (351 mg, 1.84 mmol) were dissolved in 10 mL DCM, then 4-DMAP (11 mg, 0.092 mmol) and TEA (372 mg, 3.68 mmol) were added, and the reaction was stirred for 6 h at room temperature under N₂. Water was added to the reaction solution and the mixture was extracted with EA. The organic phase was backwashed with saturated brine, dried over anhydrous Na₂SO₄, filtered and concentrated under reduced pressure. The residue was purified by flash chromatography (PE:EA=4:1) to obtain **HT519-1** (300 mg, yellow oil) in 88 % yield. LCMS (ESI): *m*/*z* =316.1 [M+H-56]⁺; RT = 1.447 min (2.5 min).

The final product was purified by prep-HPLC to afford **HANT-519** (22.3 mg, white solid), yield: 14.4 %. LCMS (ESI): *m*/*z* =719.2 [M+H]⁺; RT= 2.533 min (5.0 min). ¹H NMR (400 MHz, DMSO-*d*₆) δ 11.13 (s, 1H), 8.09 (s, 2H), 7.85 (d, *J =* 9.6 Hz, 1H), 7.48 (s, 1H), 7.39 (d, *J =* 9.0 Hz, 1H), 7.27 (s, 1H), 5.13 (d, *J =* 11.9 Hz, 1H), 4.52 (s, 1H), 4.37 (d, *J =* 15.0 Hz, 4H), 4.28-4.19 (m, 2H), 4.06 (s, 1H), 3.97 (s, 2H), 3.85 (d, *J =* 17.9 Hz, 4H), 2.86 (s, 3H), 2.65 (d, *J=* 19.5 Hz, 2H), 2.03 (dd, *J* = 27.5, 18.5 Hz, 2H), 1.77 (d, *J =* 10.8 Hz, 3H).

### Biological Tests:

### Androgen receptor (AR) reporter gene assay

Experimental Reagents: 11-Ketodihydrotestosterone (11-KDHT) was purchased from MCE (cat. HY-135794); Enzalutamide (MDV3100) was purchased from Selleck (cat. S1250); Bright-Lite Luciferase Assay kit was purchased from Vazyme (cat. DD1204-01);

Experimental Methods:
1. The described compounds were dissolved in 100% DMSO at a final concentration of 10 mM. 100 nL each of 400X compound to be tested and 400X 11-KDHT (40 nM) represtively were added to a 384-well assay plate with Echo, followed by 20 µL of complete medium, and centrifuged at 1000 rpm for 1 min, and oscillated for 20-30 min at room temperature.
2. 20 µL of AR+ARE/Luc HEK293T cells (10,000 cells/well) were inoculated into the assay plate containing the compounds and 11-KDHT, and centrifuged at 1000 rpm for 1 min. The assay plate was incubated in a 37 °C CO₂ incubator for 24h.
3. 40 µL of Bright-Lite assay reagent was transferred to each well of the assay plate, centrifuged at 1000 rpm for 1 min, and shaken for 2 min at room temperature protected from light.
4. Luminescence values (RLU) were measured using Envision. Based on the results, the IC₅₀ values of the compounds described were calculated.

**Table 1. AR inhibition of Representative Compounds**

| **No.** | **ARE Luc IC₅₀ (nM)** | **No.** | **ARE Luc IC₅₀ (nM)** | **No.** | **ARE Luc IC₅₀ (nM)** |
|---|---|---|---|---|---|
| HANT-312 | 377 | HANT-336 | 1158 | HANT-371 | 292 |
| HANT-314 | 490 | HANT-338 | 657 | HANT-390 | 476 |
| HANT-315 | 30 | HANT-339 | 569 | HANT-393 | 428 |
| HANT-315-P1 | 2 | HANT-342 | 27 | HANT-431 | 744 |
| HANT-315-P2 | 3871 | HANT-346 | 29 | HANT-514 | 73 |
| HANT-331 | 822 | HANT-354 | 21 | HANT-512 | 318 |
| HANT-328 | 1831 | HANT-329 | 123 | HANT-362 | 913 |
| HANT-513 | 7.6 | HANT-518 | 209 | HANT-516 | 32 |
| HANT-517 | 575 | HANT-519 | 23 | HANT-515 | 754 |
| Enzalutamide | 58 | HANT-369 | 1247 | | |

### AR degradation experiment:

In this experiment, cells stably expressing AR-V7-HiBiT was constructed by fusing HiBiT tag (PromegaTM) into the C-terminus of the AR-V7 genome of 22RV1 cells (ATCC) using CRISPR/Cas9 technology. The concentration of test compounds started from 1000 nM, and diluted with DMSO by 3-fold gradient for 11 points. The cells were seeded into 384-well plates with 5000 cells per well using RMPI-1640 culture medium After co-incubation with the test compound for 24 hr at 37 °C, luminescent signal of the HiBiT tag in the treated cells was measured using the Nano-Glo HiBiT Lytic Detection System (Promega, N3040) according to the manufacturer's operation manual (the abundance of AR-V7 HiBiT protein is proportional to the strength of the luminescent signal), and the dose-response curve was plotted by GraphPad Prism to calculate the DC₅₀ of the subject compounds (the concentration of the compound required for degradation 50% of the target protein).

**Table 2. AR degradation of Representative Compounds**

| **No.** | **HiBiT DC₅₀ (nM)** | **No.** | **HiBiT DC₅₀ (nM)** |
|---|---|---|---|
| HANT-315 | 8.7 | HANT-346 | 24 |
| HANT-315-P1 | 4.3 | HANT-354 | 13 |
| HANT-342 | 40 | HANT-371 | 284 |
| HANT-331 | >10000 | HANT-338 | >10000 |
| Enzalutamide | >10000 | | |

## Claims

1. A compound of formula (I), or a stereoisomer, a solvate, a hydrate or a pharmaceutically acceptable salt thereof, 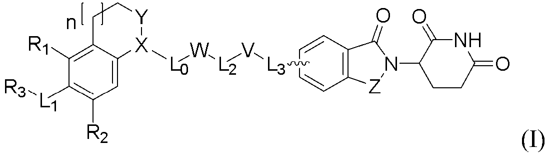 wherein, n is 0 or 1;
X is N, CCH₃ or CH;
Y is NR₄ or CH₂, wherein R₄ is H, acetyl or C₁₋₃ alkyl;
Z is C(O) or CH₂;
R₁ and R₂ are each independently selected from H, halogen, CN, C₁₋₄ alkyl, C₁₋₄ alkoxy, and halo C₁₋₄ alkyl;
L₁ is a single bond, O, S or NH;
R₃ is C₁₋₆ alkyl, C₃₋₆ cycloalkyl or C₃₋₆ cycloalkyl-C₁₋₄ alkyl; optionally being substituted with halogen or CN;
L₀ is a single bond, O, S, NH, -C(O)-NH-, -NH-C(O)-, -NH-(CH₂)- or -NH-(CH₂)₂-;
W is C₆₋₁₂ aryl, 5-12-membered heteroaryl or 4-12-membered heterocyclyl; each of which is optionally substituted with 1, 2 or 3 substituents independently selected from: halogen, CN, oxo, NH₂, NR₇R₈, OH, OR₈, R₈, -C(O)-NHR₇, -OC(O)-NHR₈, -SO₂R₈, -SO₂NHR₇, -NR₇SO₂R₈, -NR₇SO₂NHR₈ and -NHC(O)-R₈;
wherein, R₇ is each independently selected from H, C₁₋₄ alkyl and acetyl; R₈ is C₁₋₄ alkyl optionally substituted with one or more halogens, hydroxyls or NH₂;
L₂ is a single bond, -NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₈ alkyl-, -C₂₋₆ alkenyl-, -C₁₋₄ alkyl-O-C₁₋₄ alkyl-, -C₁₋₄ alkyl-NH-C₁₋₄ alkyl-, -C₃₋₆ cycloalkyl, -O-C₃₋₆ cycloalkyl, -NH-C₃₋₆ cycloalkyl, phenyl, 3-10-membered heterocycloalkyl or 5-10-membered heteroaryl;
L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₈ alkyl-, -C₂₋₆ alkenyl-, -C₁₋₄ alkyl-O-C₁₋₄ alkyl-, -C₁₋₄ alkyl-NH-C₁₋₄ alkyl-, -C₃₋₆ cycloalkyl, -C₃₋₆ cycloalkyl-O-, -C₃₋₆ cycloalkyl-NH-, phenyl, 3-10-membered heterocycloalkyl or 5-10-membered heteroaryl; and
the alkyl, the heteroaryl and the heterocycloalkyl in L₂ and L₃ are each optionally substituted with one or more substituents independently selected from halogen, OH, NH₂, CN, C(O)C₁₋₆ alkyl, C(O)NH₂, C₁₋₆ alkyl, C₃₋₆ cycloalkyl, C₁₋₆ alkoxy, -NHC₁₋₆ alkyl and -N(C₁₋₆ alkyl)₂;
V is CH₂, O, S or NH;
wherein, the heteroaryl, the heterocyclyl, the heterocycloalkyl are each independently comprising 1, 2, 3 or 4 heteroatoms independently selected from N, O and S.

2. The compound, or the stereoisomer, the solvate, the hydrate or the pharmaceutically acceptable salt thereof according to claim 1, wherein
n is 0 or 1;
X is CH;
Y is CH₂;
Z is C(O) or CH₂;
R₁ and R₂ are each independently selected from halogen, CN, C₁₋₄ alkyl, C₁₋₄ alkoxy, and halo C₁₋₄ alkyl;
L₁ is a single bond, O, S or NH;
R₃ is C₁₋₆ alkyl optionally substituted with halogen or CN;
L₀ is a single bond, O, S, or NH;
W is C₆₋₁₂ aryl or 5-10-membered heteroaryl, the aryl and the heteroaryl being each optionally substituted with 1, 2 or 3 substituents independently selected from: halogen, CN, OH, NH₂, oxo, C(O)NH₂, C₁₋₄ alkyl and C₁₋₄ alkoxy;
L₂ is a single bond, -NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₆ alkyl-, -C₁₋₄ alkyl-O-C₁₋₄ alkyl -, -C₁₋₄ alkyl-NH-C₁₋₄ alkyl-, -O-C₃₋₆ cycloalkyl, -NH-C₃₋₆ cycloalkyl, 3-10-membered heterocycloalkyl or 5-10-membered heteroaryl;
L₃ is -C₁₋₆ alkyl-O-, -C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₆ alkyl-, -C₁₋₄ alkyl-O-C₁₋₄ alkyl-, -C₁₋₄alkyl-NH-C₁₋₄ alkyl-, -C₃₋₆ cycloalkyl-O-, -C₃₋₆ cycloalkyl-NH-, 3-10-membered heterocycloalkyl or 5-10-membered heteroaryl; and
V is CH₂, O, S or NH;
wherein, the heterocycloalkyl or the heteroaryl is each independently comprising 1, 2, 3 or 4 heteroatoms independently selected from N, O and S, for example, the heterocycloalkyl or the heteroaryl each comprises 1 N heteroatom and optionally further comprises 1, 2 or 3 heteroaryl atoms independently selected from N, O and S.

3. The compound according to any one of preceding claims having the structure of formula (II):

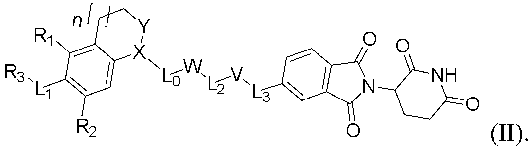

4. The compound, or the stereoisomer, the solvate, the hydrate or the pharmaceutically acceptable salt thereof according to any one of proceeding claims, wherein W is 5-10-membered heteroaryl; the heteroaryl comprises 1 N heteroatom and optionally further comprises 1, 2 or 3 heteroatoms independently selected from N, O and S, and is optionally substituted with 1, 2 or 3 substituents independently selected from halogen and CN.

5. The compound, or the stereoisomer, the solvate, the hydrate or the pharmaceutically acceptable salt thereof according to any one of proceeding claims, wherein L₂ is a single bond, - NH-C₁₋₆ alkyl-, -NH-C₂₋₆ alkenyl-, -NH-C₁₋₄ alkyl-O-C₁₋₄ alkyl-, -O-C₁₋₆ alkyl-, -O-C₂₋₆ alkenyl-, -O-C₁₋₄ alkyl-O-C₁₋₄ alkyl-, -C₁₋₆ alkyl-, -C₁₋₄ alkyl-O-C₁₋₄ alkyl-, -C₁₋₄ alkyl-NH-C₁₋₄ alkyl-, -O-C₃₋₆ cycloalkyl, -NH-C₃₋₆ cycloalkyl or 3-10-membered heterocycloalkyl; wherein the heterocycloalkyl comprises 1 N heteroatom and optionally further comprises 1, 2 or 3 heteroatoms independently selected from N, O and S.

6. The compound, or the stereoisomer, the solvate, the hydrate or the pharmaceutically acceptable salt thereof according to any one of proceeding claims, wherein L₂ is a single bond, - NH-C₁₋₄ alkyl-, -O-C₁₋₄ alkyl-, -C₁₋₄ alkyl-, -O-C₃₋₆ cycloalkyl or 4-6-membered heterocycloalkyl; the 4-6-membered heterocycloalkyl comprises 1 N heteroatom, and L₂ is connected to W via a heteroatom when L₂ is not a single bond or -C₁₋₄ alkyl-.

7. The compound, or the stereoisomer, the solvate, the hydrate or the pharmaceutically acceptable salt thereof according to any one of proceeding claims, wherein L₃ is -C₁₋₆ alkyl-O-, - C₂₋₆ alkenyl-O-, -C₁₋₄ alkyl-O-C₁₋₄ alkyl-O-, -C₁₋₆ alkyl-NH-, -C₂₋₆ alkenyl-NH-, -C₁₋₄ alkyl-O-C₁₋₄ alkyl-NH-, -C₁₋₆ alkyl-, -C₁₋₄ alkyl-O-C₁₋₄ alkyl-, -C₁₋₄ alkyl-NH-C₁₋₄ alkyl-, -C₃₋₆ cycloalkyl-O-, -C₃₋₆ cycloalkyl-NH- or 3-10-membered heterocycloalkyl; the heterocycloalkyl comprises 1 N heteroatom and optionally further comprises 1, 2 or 3 heteroatoms independently selected from N, O and S.

8. The compound, or the stereoisomer, the solvate, the hydrate or the pharmaceutically acceptable salt thereof according to any one of proceeding claims, wherein L₃ is -C₁₋₄ alkyl-O-, - C₁₋₄ alkyl-NH-, -C₃₋₆ cycloalkyl-O- or 4-6-membered heterocycloalkyl; the 4-6-membered heterocycloalkyl comprises 1 N heteroatom, and L₃ is connected to the benzene ring of the general formula via a heteroatom.

9. The compound, or the stereoisomer, the solvate, the hydrate or the pharmaceutically acceptable salt thereof according to any one of proceeding claims, wherein V is CH₂ or O.

10. The compound, or the stereoisomer, the solvate, the hydrate or the pharmaceutically acceptable salt thereof according to any one of proceeding claims, wherein n is 1.

11. The compound, or the stereoisomer, the solvate, the hydrate or the pharmaceutically acceptable salt thereof according to any one of proceeding claims, wherein L₁ is O.

12. The compound, or the stereoisomer, the solvate, the hydrate or the pharmaceutically acceptable salt thereof according to any one of any proceeding claims, wherein L₀ is a single bond or NH.

13. The compound, or the stereoisomer, the solvate, the hydrate or the pharmaceutically acceptable salt thereof according to any one of proceeding claims, wherein
n is 1;
X is CH;
Y is CH₂;
Z is C(O);
R₁ is halogen;
R₂ is CN;
L₁ is O;
R₃ is ethyl optionally substituted with halogen;
L₀ is a single bond or NH;
W is 5-10-membered heteroaryl comprising 1, 2 or 3 N heteroatoms, which is optionally substituted with 1 or 2 substituents independently selected from halogen and CN;
L₂ is a single bond, -NH-CH₂-, -NH-CH₂-CH₂-, -O-CH₂-, -O-CH₂-CH₂-, -C₁₋₃ alkyl-, -O-C_{3- 6} cycloalkyl- or 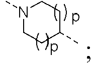 wherein p is each independently selected from 0 and 1, and L₂ is connected to W via a heteroatom when L₂ is not a single bond or -C₁₋₃ alkyl-;
L₃ is -CH₂-CH₂-O-, -CH₂-CH₂-CH₂-O-, -CH₂-NH-, -C₃₋₆ cycloalkyl-O- or 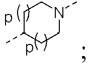 wherein p is each independently selected from 0 and 1, e.g., p is each 0, and L₃ is connected to the benzene ring of the general formula via a heteroatom; and
V is CH₂ or O.

14. The compound, or the stereoisomer, the solvate, the hydrate or the pharmaceutically acceptable salt thereof according to claim 1, wherein the compound is selected from:

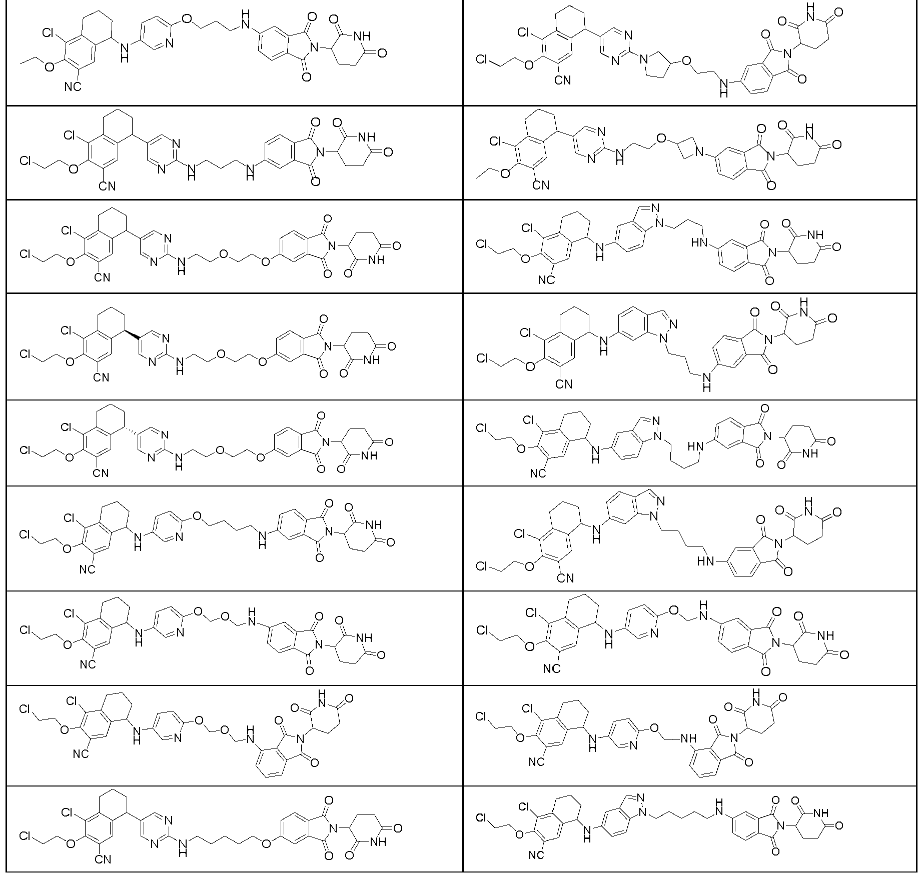

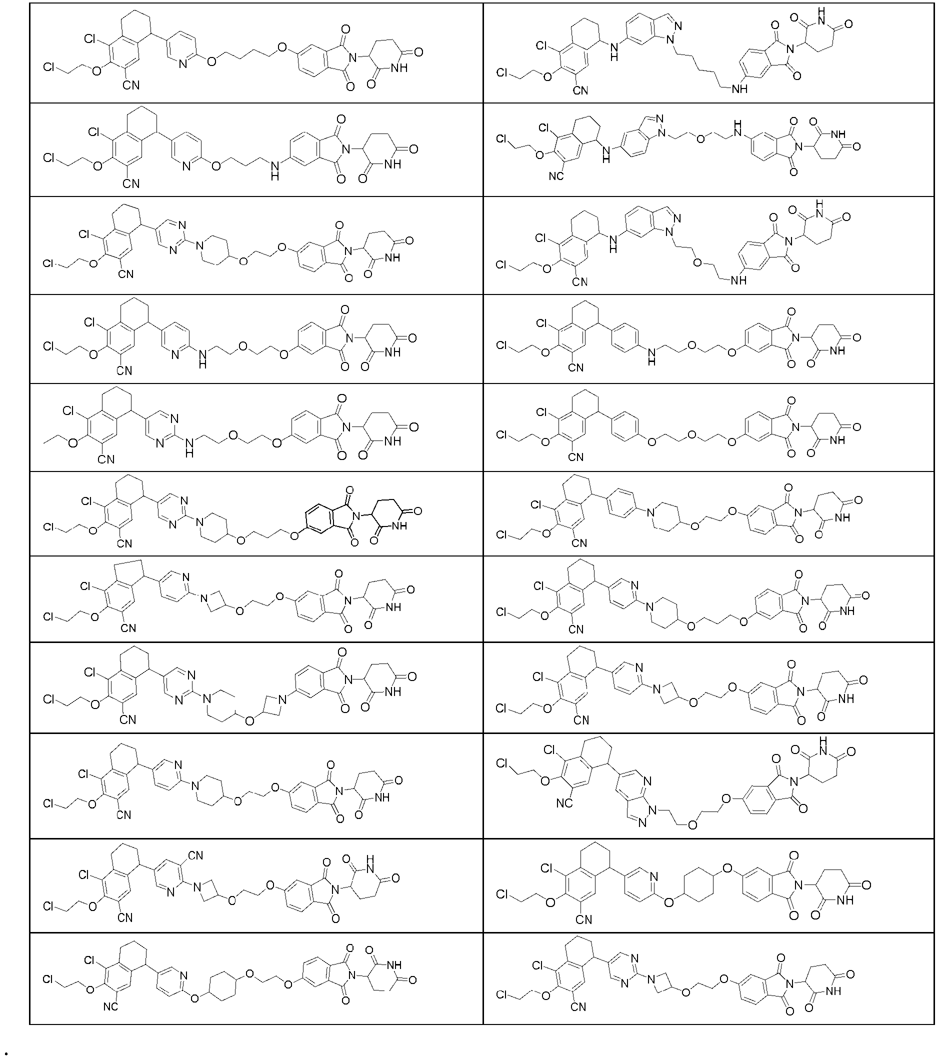

15. A pharmaceutical composition comprising the compound, or the stereoisomer, the solvate, the hydrate or the pharmaceutically acceptable salt thereof according to any one of claims 1-14, and pharmaceutically acceptable excipients.

16. The compound, or the stereoisomer, the solvate, the hydrate or the pharmaceutically acceptable salt thereof according to any one of claims 1-14, which is used as drugs.

17. Use of the compound, or the stereoisomer, the solvate, the hydrate or the pharmaceutically acceptable salt thereof according to any one of claims 1-14, in the treatment or prevention of androgen receptor-dependent diseases or disorders in individuals, in particular, AR-SV-positive diseases or disorders or AR-V7-positive diseases or disorders; wherein the disorders or diseases selected from, prostate cancer, other prostate diseases (e.g., prostatic hyperplasia, prostatitis), breast cancer, non small cell lung cancer, renal cell carcinoma, gonadal tumors, seminoma, pancreatic cancer, ovarian cancer, fallopian tube cancer, peritoneal cancer, salivary gland cancer, bladder cancer, acne, hirsutism, hidradenitis suppurativa, androgenetic alopecia, cryptorchidism, androgen insensitivity syndrome and Kennedy's disease.

18. Use of the compound, or the stereoisomer, the solvate, the hydrate or the pharmaceutically acceptable salt thereof according to any one of claims 1-14, in the preparation of drugs for use in the treatment or prevention of diseases or disorders in individuals, wherein the disorders or diseases are androgen receptor-dependent diseases or disorders, in particular AR-SV-positive diseases or disorders or AR-V7-positive diseases or disorders; wherein the disorders or diseases selected from, for example, prostate cancer, other prostate diseases (e.g., prostatic hyperplasia, prostatitis), breast cancer, non small cell lung cancer, renal cell carcinoma, gonadal tumors, seminoma, pancreatic cancer, ovarian cancer, fallopian tube cancer, peritoneal cancer, salivary gland cancer, bladder cancer, acne, hirsutism, hidradenitis suppurativa, androgenetic alopecia, cryptorchidism, androgen insensitivity syndrome and Kennedy's disease.

19. A method for treating or preventing diseases or disorders in individuals, comprising administering an effective amount of the compound, or the stereoisomer, the solvate, the hydrate or the pharmaceutically acceptable salt thereof according to any one of claims 1-14 to an individual in need thereof, wherein the disorders or diseases are androgen receptor-dependent diseases or disorders, in particular AR-SV-positive diseases or disorders or AR-V7-positive diseases or disorders; wherein the disorders or diseases selected from, for example, prostate cancer, other prostate diseases (e.g., prostatic hyperplasia, prostatitis), breast cancer, non small cell lung cancer, renal cell carcinoma, gonadal tumors, seminoma, pancreatic cancer, ovarian cancer, fallopian tube cancer, peritoneal cancer, salivary gland cancer, bladder cancer, acne, hirsutism, hidradenitis suppurativa, androgenetic alopecia, cryptorchidism, androgen insensitivity syndrome and Kennedy's disease.

20. A method for inhibiting and/or degrading an androgen receptor in vivo or in vitro, comprising administering an effective amount of the compound, or the stereoisomer, the solvate, the hydrate or the pharmaceutically acceptable salt thereof according to any one of claims 1-14, in contact with the androgen receptor.

21. A pharmaceutical composition comprising the compound, or the stereoisomer, the solvate, the hydrate or the pharmaceutically acceptable salt thereof according to any one of claims 1-14, and one or more other therapeutic agents.
